(19) Europäisches Patentamt
European Patent Office
Office européen des brevets

(11) Publication number: 0 048 159
A2

# (12) EUROPEAN PATENT APPLICATION

(21) Application number: 81304210.8

(22) Date of filing: 15.09.81

(51) Int. Cl.3: C 07 C 103/52
C 07 D 207/16, C 07 D 207/22
C 07 D 207/28, C 07 D 277/06
A 61 K 37/02

(30) Priority: 17.09.80 US 187992
21.08.81 US 295135

(43) Date of publication of application:
24.03.82 Bulletin 82/12

(84) Designated Contracting States:
BE CH DE FR GB IT LI LU NL SE

(71) Applicant: UNIVERSITY OF MIAMI
Coral Gables Florida 33124(US)

(72) Inventor: Ryan, James Walter
3420 Poinciana Ave
Miami Florida(US)

(72) Inventor: Chung, Alfred
8781 Southwest 87th
Miami Florida(US)

(74) Representative: De Minvielle-Devaux, Ian Benedict Peter et al,
CARPMAELS & RANSFORD 43, Bloomsbury Square
London WC1A 2RA(GB)

## (54) Novel carboxyalkyl peptides and thioethers and ethers of peptides as antihypertensive agents.

(57) Novel inhibitors of angiotensin converting enzyme are disclosed which have the general formula

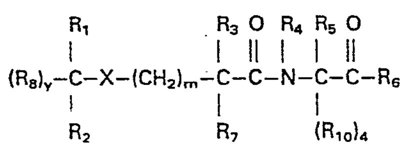

Wherein X = X, O or $NR_9$, $R_4$ and $R_5$ form with −N−C− a 4-6 membered ring structure as described and the other R substituents are selected from a variety of disclosed groups.

EP 0 048 159 A2

BACKGROUND OF THE INVENTION

Angiotensin converting enzyme (peptidyldipeptide hydrolase, hereinafter referred to as ACE) occupies a central role in the physiology of hypertension. The enzyme is capable of converting the decapeptide angiotensin I, having the sequence

AspArgValTyrIleHisProPheHisLeu

to an octapeptide, angiotensin II, by removal of the carboxy-terminal HisLeu. The symbols for the above chemical moieties and others used throughout this application are explained in the following table. In each instance, the symbol for an amino acid is also used herein at times to refer to a mono or divalent radical of such acid, and those of ordinary skill in the art will readily understand the context of each specific use:

Ala = alanine
Arg = arginine
Asp - aspartic acid
Aze = azetidine
Boc = t-butyloxycarbonyl
Cbo = carbobenzyloxy
Cys = cysteine
<Glu = pyro-L-glutamic acid
Glu = glutamic acid
Gly = glycine
Hip = Hippuric acid (Benzoyl-glycine)
His = histidine
Ile = isoleucine
Leu = leucine
Lys = lysine
Met = methionine
Orn = ornithine
Phe = phenylalaine
Pip = pipecolinic acid
Pro = proline
ΔPro = 3,4-dehydroproline
Ser = serine
Thr = threonine
Thy = thyronine
Tos = Tosyl
Trp = tryptophan
Tyr = tyrosine
Val = valine
Pht = phthaloyl
ACE = Angiotensin converting enzyme
Hepes = N-2-hydroxyethylpiperazine-n'-2-ethanesulfonic acid

Angiotensin I is formed by the action of the enzyme renin, an endopeptidase found in kidney, other tissues and plasma, on a serum $\alpha$-2 globulin.

Blood pressure is affected by certain peptides found in the blood. One of these, angiotensin II, is a powerful pressor (blood pressure elevating) agent. Another, bradykinin, a nonapeptide with the sequence ArgProProGlyPheSerProPheArg is a powerful depressor (blood pressure lowering) agent. In addition to a direct pressor effect, angiotensin II stimulates release of aldosterone which tends to elevate blood pressure by causing retention of extracellular salt and fluids. Angiotensin II is found in measurable amount in the blood of normal humans. However, it is found at elevated concentrations in the blood of patients with renal hypertension.

The level of ACE activity is ordinarily in excess, in both normal and hypertensive humans, of the amount needed to maintain observed levels of angiotensin II. However, it has been found that significant blood pressure lowering is achieved in hypertensive patients by treatment with ACE inhibitors. [Gavras, I., et al., New Engl. J. Med. 291, 817 (1974)].

ACE is a peptidyldipeptide hydrolase. It catalyzes the hydrolysis of the penultimate peptide bond at the C-terminal end of a variety of acylated tripeptides and larger polypeptides having an unblocked $\alpha$-carboxyl group. The action of ACE results in hydrolytic cleavage of the penultimate peptide bond from the carboxyl-terminal end yielding as reaction products a dipeptide and a remnant.

The reactivity of the enzyme varies markedly depending on the substrate. At least one type of peptide bond, having the nitrogen supplied by proline, is not hydrolyzed at all. The

apparent Michaelis constant (Km) varies from substrate to substrate over several orders of magnitude. For general discussion of the kinetic parameters of enzyme catalyzed reactions, see Lehninger, A., Biochemistry, 2nd Ed., Worth Publishers, Inc., New York, 1975, pp. 189-195. Many peptides which are called inhibitors of the enzymatic conversion of angiotensin I to angiotensin II are in fact substrates having a lower Km than angiotensin I. Such peptides are more properly termed competitive substrates. Examples of competitive substrates include bradykinin, and the peptide $BPP_{5a}$ (also called SQ20475) from snake venom, whose sequence is <GluLysTrpAlaPro.

Numerous synthetic peptide derivatives have been shown to be ACE inhibitors by Ondetti, et al. in U.S. patent 3,832,337 issued August 27, 1974.

The role of ACE in the pathogenesis of hypertension has prompted a search for inhibitors of the enzyme that could act as antihypertensive drugs. See for example U.S. patents 3,891,616, 3,947,575, 4,052,511 and 4,053,651. A highly effective inhititor, with high biological activity when orally administered, is D-3-methylpropanoyl-L-proline, designated SQ14225, or "captopril" disclosed in U.S. patent 4,046,889 to Ondetti et al., issued September 6, 1977, and in scientific articles by Cushman, D.W. et al., Biochemistry **16**, 5484 (1977), and by Ondetti, M. et al., Science **196**, 441 (1977). The inhibitor SQ14225 reportedly has an $I_{50}$ value of $2.3 \times 10^{-8}$M. The $I_{50}$ value reported by Cushman, et al., *supra* is the concentration of inhibitor required to produce 50% inhibition of the enzyme under a standard assay system containing substrate at a level substantially above $K_m$. It will be understood that $I_{50}$ values are directly comparable

when all potential factors affecting the reaction are kept constant. These factors include the source of enzyme, its purity, the substrate used and its concentration, and the composition of the assay buffer. All $I_{50}$ data reported herein have been performed with the same assay system and same enzyme (human urinary ACE) and the same level of substrate and are therefore internally consistent. Discrepencies with data obtained by other workers may be observed. Indeed such discrepancies do exist in the literature, for unknown reasons. See, for example, the $I_{50}$ values for $BPP_{9a}$ reported by Cushman, D.W., et al., Experientia **29**, 1032 (1973) and by Dorer, F.E., et al., Biochim.Biophys.Acta **429**, 220 (1976).

The mode of action of SQ14225 has been based upon a model of the active site of ACE developed by analogy with the better known related enzyme, carboxypeptidase A. The active site was postulated to have a cationic site for binding the carboxyl end group of the substrate and a pocket or cleft capable of binding the side chain of the C-terminal amino acid and providing expecially tight binding for the heterocyclic ring of a terminal proline residue. A similar pocket for the penultimate amino acid residue was postulated, and the published data suggested a rather stringent steric requirement, since the D-form of the inhibitor was substantially more potent than its stereoisomer or the 3-methyl and unsubstituted analogs. The sulfhydryl group on the inhibitor, postulated to be bound at the active site near the catalytic center, was believed to play a central role in inactivation of the enzyme by combining with the zinc moiety known to be essential for catalytic activity. Substituents on

the sulfhydryl, such as a methyl group, and an S-acetyl derivative, substantially reduced potency of the inhibitor. See Cushman, D.W., et al., Biochemistry, supra.

*In vitro* study of the mechanism by which SQ14225 and its analogs act to inhibit ACE has been somewhat hampered by the instability of these molecules under ambient conditions. For example, it has been observed that a fresh aqueous solution of concentration, e.g., 1 mg per ml of SQ14225 at a pH of about 8 becomes substantially less active upon standing for as little as 30 minutes, and that activity continues to decrease as the solution stands for longer periods. It is believed that this loss in activity is mainly the result of dimerization of SQ14225 occurring at the sulfhydryl end groups, whereby a disulfide is formed which is largely inactive as an inhibitor. Since the free sulfhydryl group is highly reactive and may be readily oxidized to polar acidic moieties such as sulfone and sulfoxide groups, it may also be that the observed *in vitro* loss of activity—of aqueous solutions of SQ14225 on standing is in some part a consequence of one or more such oxidation reactions, with formation of a sulfone or sulfoxide which does not function effectively as an inhibitor for ACE.

Such reports of SQ14225 clinical testing as are currently available, some of which refer to the compound under the name "Captopril" or "Capoten", suggest that the product is sufficiently stable in the normal gastric and intestinal environments of most patients to be an effective inhibitor for ACE when administered orally. It is not yet clear, however, whether there may be a group of patients for

which SQ14225 is substantially ineffective. Because of the high reactivity of the free sulfhydryl group, SQ14225 could readily form mixed disulfides with serum, cellular proteins, peptides or other free sulfhydryl group-containing substances in the gastric or intestinal environments, in addition to the possibility for dimer formation or oxidative degradation reactions. A mixed disulfide with protein may be antigenic and, indeed, occasional allergic reactions have been clinically observed. See Gavras, et al., New England J.Med. 298, 991 (1978). Disulfides and oxidative degradation products of SQ14225, if formed, may at best be expected to be largely ineffective as inhibitors. It may be postulated accordingly that dose response to SQ14225 may vary with conditions of administration and among individual patients. Moreover, in at least some patients, unwanted side effects may occur and maintenance of an effective concentration of the inhibitor in the body may be difficult to control.

Adverse effects of SQ14225 in man include fevers and rashes (Gavras et al., supra). Hoorntje et al., The Lancet i, 1212-1214 (1980) describe the performance of renal biopsies on 13 patients treated wtih SQ14225. All biopsies showed evidence of immune complex deposition along the glomerular basement membranes, although 9 of 13 patients were asymptomatic at the time of the biopsy. These authors also discussed similarities of their findings with those induced by another drug with a free mercapto group, D-penicillamine.

In an effort to devise inhibitors of angiotensin converting enzyme that are more stable than captopril and less likely to

or an analogous six-membered ring and $R_3$ must then be selected from any of groups (i) - (xxxi) above.

E. When at least one of $R_7$ and $R_8$ is not hydrogen, or when X is $NR_9$ and $R_9$ is methyl, $R_1$ may be as defined in B above, or may be from any of groups (i) to (x) above, when $R_3$ is as defined in C above.

F. When $R_4$ and $R_5$ together are any structure that is disubstituted or monosubstituted with a moiety other than -OH, or when $R_4$ and $R_5$ form any recited structure other than one of

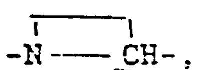, 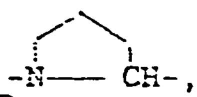, 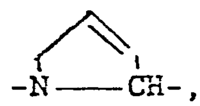, 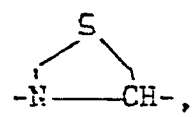, 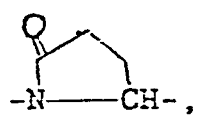, 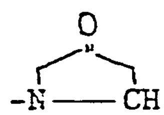

and 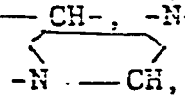, $R_1$ may be as defined in B or may be from any of groups (i) to (x) when $R_3$ is as defined in C above.

2. A compound according to claim 1 in which X is -NR9.

3. A compound according to claim 1 in which X is NH.

4. A compound according to claim 2 in which $R_1$ and $R_3$ are the same and are selected from groups (i) to (xxxi).

5. A compound according to claim 2 in which $R_1$ and $R_3$ are different and each is selected from groups (i) to (xxxi).

6. A compound according to claim 2 in which $R_1$ is selected from groups (xi) to (xxxi) and $R_3$ is as defined in part B of claim 1.

7. A compound according to claim 1 in which $R_3$ is selected from groups (i) to (xxxi) and $R_1$ is as defined in part C of claim 1.

8. A compound according to claim 1 in which $R_3$ is selected from any of groups (i) - (xxxi) and $R_1$ and $R_2$ form with -CH a lactone ring as set forth in part D of claim 1.

9. A compound according to claim 2 in which $R_4$ and $R_5$ and $\overset{O}{\|}{C}-R_6$ form a proline moiety.

10. A compound according to any of claims 1 - 9 for use in inhibiting angiotensin converting enzyme in a mammal.

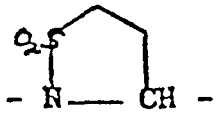, 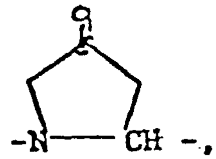, 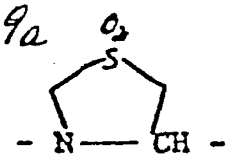, 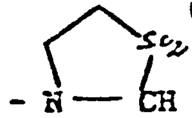,

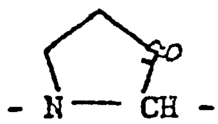, 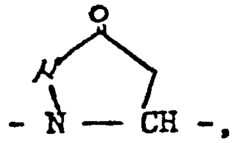, 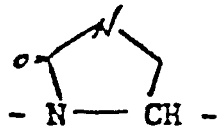, 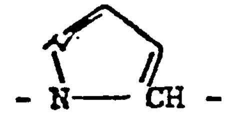,

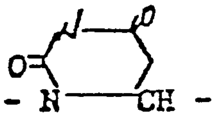, 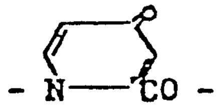, 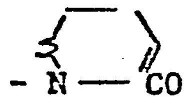 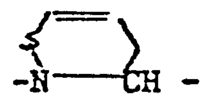, 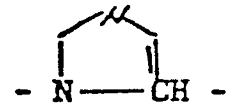,

it being understood that any of these structures may be monosubstituted with -OH, $-OCH_3$, F, 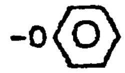, 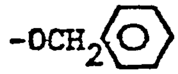, 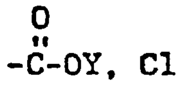, Cl, Br, I, phenyl, hydroxyphenyl, -SH, $-SCH_3$, 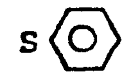, 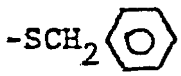, $-NHCH_3$, $-CH_2NH_2$, $-CH_3$, $-CH_2OH$, propyl, guanidino, nitroguanidino or thioguanidino and that the five or six-membered rings may be disubstituted with -OH, F, Cl, Br, I, $OCH_3$, or any combination of two of these substituents;

$R_6$ is $NH_2$, -OM or -SM, wherein M may be H, an alkyl group of 1-3 carbon atoms or any other ester moiety hydrolyzable under mammalian <u>in vitro</u> conditions to -OH, or an ionically bonded anion of a physiologically acceptable non-toxic salt;

$R_7$ is $H_1$-$CH_3$, halomethyl, hydroxymethyl, aminomethyl or mercaptomethyl;

$R_8$ is H-, $CH_3$-, amino, halomethyl, hydroxymethyl, aminomethyl, dihalomethyl, trihalomethyl, mercaptomethyl, methoxymethyl, methylthiomethyl, methoxycarbonylmethyl, cyanomethyl, benzyl, acetoxymethyl, $CH_2 = CH - CH_2$, isobutyl, mercaptoalkyl of 2-3 carbon atoms, hydroxyalkyl of 2-3 carbon atoms, ethyl, acetylthioethyl, benzamido, acetamido, phthaloylaminoalkylene wherein the alkylene group has 1-4 carbon atoms, alkoxycarbonyl isoalkylene wherein the alkyl group contains 1-6 carbons and the isoalkylene group contains 3-5 carbons, benzoylamine, alkanoylamine of 1-6 carbons, alkylamide of 1-6 carbons, phenylamine or

(A) $R_1$ and $R_3$ are the same or different and are ... of

(i) mono-N substituted alkylene of 2-4 carbons wherein the N substituent is benzoyl, Boc, Cbo, Tos, formyl or acetyl;

(ii) hydroxyphenyl or hydroxyphenyl-(1-6C)-alkylene hydroxalkylene having 1-4 carbons, or thiol analogs thereof,

(iii) mercaptoalkylene of 1-6 carbons;

(iv) phenylalkylene wherein the alkylene group has 1-6 carbons, $C_1$-$C_5$ straight or branched chain alkyl or phenyl,

(v) phenylthioalkylene, phenoxyalkylene, benzyloxyalkylene, or benzylthioalkylene wherein the alkylene group has 1-6 carbons;

(vi) alkylthioalkylene wherein the alkyl and alkylene groups have 1-6 carbons;

(vii) alkoxyphenyl or alkoxybenzyl in which the alkoxy group has 1-6 carbons, phenoxyphenyl, phenoxybenzyl, benzyloxybenzyl or benzyloxyphenyl or a thioether analog of any of them;

(viii) 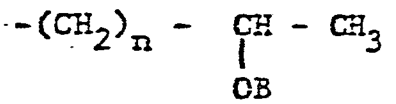 wherein n = 0 - 4 and B = H or a 1-6 carbon alkyl group; or an -SB analog thereof;

(ix) $(CH_2)_pCOOZ$ or $(CH_2)_pCOSZ$ wherein p = 0 - 3 and Z is H, phenyl, benzyl, a 1-6 carbon alkyl group, or an anion of a physiologically acceptable salt;

(x) 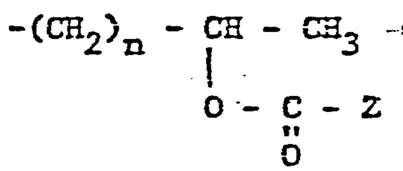 or 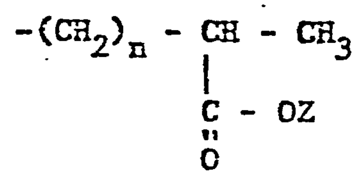

or 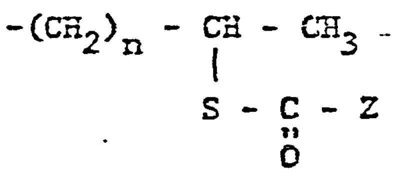 or 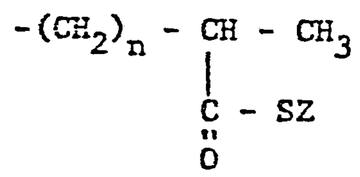

wherein n and Z each have the same significance as above;

(xi) 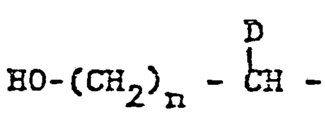 or 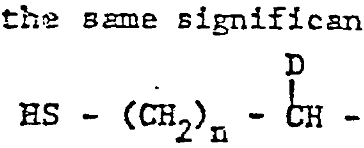 wherein n = 0 - 4, D is phenyl, thienyl or a 1-3 carbon alkyl group;

(xii) HO - $(CH_2)_n$ - C $(CH_3)_2$ -, HS - $(CH_2)_n$ - C $(CH_3)_2$ -,

$(CH_2)_n - C(CH_3)_2$ - wherein n has the same significance as above;

(xiii) p - mercaptophenyl - $(CH_2)_n - CH_2$ - or p-hydroxyphenyl - $(CH_2)_n - CH_2$ - wherein the phenyl ring has one or two nitro or amino substituents and n has the same significance as above;

(xiv) $CH_3(CH_2)_n - \overset{OH}{\overset{|}{C}}H$ - or $CH_3(CH_2)_n - \overset{SH}{\overset{|}{C}}H$ - wherein n has the same significance as above;

(xv) $NH_2$ - alkylene or $NO_2$ - alkylene containing one hydroxy or mercapto substituent and having 1 - 6 carbon atoms;

(xvi) hydroxy- or mercapto-phenoxybenzyl;

(xvii) $ZO(CH_2)_q - \overset{O}{\overset{\|}{C}} - (CH_2)_n -$, $ZS(CH_2)_q - \overset{O}{\overset{\|}{C}} - (CH_2)_n -$, $NH_2-(CH_2)_q - \overset{O}{\overset{\|}{C}} - (CH_2)_n -$, $NO_2(CH_2)_q - \overset{O}{\overset{\|}{C}} - (CH_2)_n -$; $HONH - (CH_2)_q - \overset{O}{\overset{\|}{C}} - (CH_2)_n -$; $NH_2NH - (CH_2)_q - \overset{O}{\overset{\|}{C}} - (CH_2)_n -$; $ZO - \overset{O}{\overset{\|}{C}}(CH_2)_q - \overset{O}{\overset{\|}{C}} - (CH_2)_n - ZS - \overset{O}{\overset{\|}{C}} - (CH_2)_q - \overset{O}{\overset{\|}{C}} - (CH_2)_n$ - or $NH_2\overset{O}{\overset{\|}{C}}N - (CH_2)_q - \overset{O}{\overset{\|}{C}} - (CH_2)n$ wherein q = 1 - 5 and z and n have the same significance as above;

(xviii) $ZO(CH_2)_q - \overset{OH}{\overset{|}{C}}H - (CH_2)_n -$, $ZS(CH_2)_q - \overset{OH}{\overset{|}{C}}H - (CH_2)_n -$,

$NH_2 - (CH_2)_q - \overset{OH}{\overset{|}{C}}H - (CH_2)_n -$, $NO_2 - (CH_2)_q - \overset{OH}{\overset{|}{C}}H - (CH_2)_n -$,

$ZO - (CH_2)_q - \overset{OH}{\overset{|}{C}}H - (CH_2)_n -$, $ZS - - (CH_2)_q - \overset{OH}{\overset{|}{C}}H - (CH_2)_n -$

$HONH - (CH_2)_q - \overset{OH}{\overset{|}{C}}H - (CH_2)_n -$, $NH_2NH-(CH_2)_q - \overset{OH}{\overset{|}{C}}H - (CH_2)_n -$

or $NH_2\overset{O}{\overset{\|}{C}}NH-(CH_2)_q-\overset{OH}{\overset{|}{C}}H-(CH_2)_m-$ wherein Z, q and n all have the same significance as above;

(xix) $G - NH - (CH_2)_q - \overset{O}{\overset{\|}{C}} - (CH_2)_n -$, $G - NH(CH_2)_q - \overset{OH}{\overset{|}{C}}H -$

$(CH_2)_n -$, $G - (CH_2)_q - \overset{O}{\overset{\|}{C}} - (CH_2)_n -$, $G - (CH_2)_q - \overset{OH}{\overset{|}{C}}H -$

$(CH_2)_n$ -, $NH_2 - \overset{O}{\overset{\|}{C}} - (CH_2)_q - \overset{O}{\overset{\|}{C}} - (CH_2)_n - NH_2 - \overset{O}{\overset{\|}{C}} - (CH_2)_q -$

$CH - (CH_2)_n -$

wherein G is an alkacyl or alkacyloxy group of 1 - 6 carbons, a benzoyl or benzoyloxy group, or a phenylalkacyl or phenylalkacyloxy group wherein the alkacyl or alkacyloxy group contains 2 - 6 carbons and q and n have the same significance as set forth above;

(xx) $K - (CH_2)_n - \overset{O}{\overset{\|}{C}} - (CH_2)_n -$ or $K (CH_2)_n - \overset{OH}{\overset{|}{CH}} - (CH_2)_n -$ wherein n has the significance stated above and K is selected from carboxyphenyl, aminophenyl, nitrophenyl, halophenyl, hydroxphenyl, alkylthiophenyl, alkylphenyl, mercaptophenyl, cyanophenyl, mercapto-carbonylphenyl, alkylcarbonylphenyl, alkylcarbonyloxphenyl, hydrazinophenyl, ureidophenyl, alkylcarbonylaminophenyl, alkylcarbonylthiophenyl, alkyloxyphenyl and hydroxyaminophenyl, wherein all alkyl groups contain 1 - 6 carbon atoms;

(xxi) $L - (CH_2)_n - \overset{O}{\overset{\|}{C}} - (CH_2)_n -$ or $L (CH_2)_n - \overset{OH}{\overset{|}{CH}} - (CH_2)_n -$

wherein n has the significance stated above and L is selected from cycloalkyl groups of 3 - 7 carbons which may be unsubstituted with up to two groups selected from among carboxy, amino, nitro, halo, hydroxy, mercapto, mercaptocarbonyl, hydroxyamino, alkyl, alkylcarbonyl, alkylcarbonyloxy, alkylthio, alkylcarbonylamino, alkylcarbonylthio, cyano, hydrazino, ureido and alkyloxy, wherein all alkyl groups contain 1 - 6 carbon atoms;

(xxii) guanidino alkylene, thioguanidinoalkylene, or nitroguanidino alkylene in which the alkylene groups contain 1 - 6 carbon atoms;

(xxiii) ring substituted aryl groups in which the ring substituents may be the same or different and may comprise up to

five per ring of any of the following: - $NH_2$, - OZ, -SZ, halogen, -CN, -$NO_2$, -COOZ, -COSZ, $CONH_2$, -$NHNH_2$, alkyl, alkylcarbonyl, alkylcarbonyloxy, alkylcarbonylamino, haloalkyl, dihaloalkyl, trihalomethyl, hydroxyamino, alkylcarbonylthio, phenoxy, and benzyloxy wherein the alkyl groups contain 1 - 6 carbon atoms and Z has the same significance as above;

(xxiv) amidoalkylene or alkylcarbonyl-aminoalkylene wherein the alkyl and alkylene groups contain 1 - 6 carbon atoms;

(xxv) hydroxyaminoalkylene of 1 - 6 carbons;

(xxvi) vinyl and substituted vinyl groups in which the substituents may be alkyl, aryl, cycloalkyl or heterocyclic groups;

(xxvii) unsubstituted heterocyclic groups selected from among phenothiazinyl, pyrrolidinyl, pyrrolyl, quinolinyl, imidazolyl, pyridyl, thyminyl, benzothiazinyl, indolyl, thienyl, purinyl, piperidinyl, morpholinyl, azaindolyl, pyrazinyl, pyrimidyl, piperonyl, piperazinyl, furanyl, thiazolyl and thiazolidinyl, cytosinyl,

(xxviii) alkylene or alkenyl groups of 1 - 6 carbons substituted with one of the heterocyclic rings from (xxvii) above;

(xxix) groups from (xxvii) or (xxviii) above containing up to four ring substitutents on the heterocyclic ring selected from among -OZ, -SZ, -COOZ, -$NO_2$, -$NH_2$, -COSZ, halogen, haloalkyl, dihaloalkyl, trihalomethyl, cyano, $CONH_2$, alkyl, alkylcarbonyl, alkylcarbonyloxy, alkylcarbonyl-amino, alkylcarbonylthio, phenoxy, benzyloxy, $-NH\overset{O}{\overset{\|}{C}}-NH_2$, -$NHNH_2$ and HONH - wherein Z has the same significance as above;

(xxx) groups from (xxvii), (xxviii) or (xxix) attached to one valence of an etheric -O- or -S-, and

(xxxi) mono-, di- or tri-alkyl-, alkenyl- or phenyl-silyl or selenyl wherein the alkyl or alkenyl groups contain 1 - 6 carbons.

B. When $R_1$ is a group from among (xi) - (xxxi) above; $R_3$ may also be any of H, 1 - 5 carbon straight or branched chain alkyl, phenyl, -OH, alkoxy of 1 - 5 carbons, benzyloxy, benzyloxyalkylene or phenoxyalkylene wherein the alkylene has 1 - 5 carbons, alkoxyalkylene having 1 - 5 carbons in the alkoxy and alkylene groups, aminoalkylene of 1 - 6 carbons, alkenyl of 1 - 6 carbons, benzyl, hydroxyalkyl of 1 - 6 carbons, mercaptoalkyl of 1 - 6 carbons, histidinyl, haloalkyl of 1 - 6 carbons, 4 - aminomethyl-benzyl, acetamidoalkyl of 1 - 5 carbons, benzylthiomethylene, or dimethylaminoalkyl of 1 - 5 carbons.

C. When $R_3$ is a group from among (i) - (xxxi) above, $R_1$ may also be any of H, $C_1$ - $C_8$ straight or branched chain alkyl, phenyl, benzyl, unsubstituted aminoalkylene of 2 - 6 carbons, hydroxyalkylene of 1 - 6 carbons, hydroxyphenyl, phenoxyalkylene or benzyloxyalkylene wherein the alkylene group has 1 - 6 carbons, cycloalkyl of 3 - 6 carbons, cycloalkyl methyl, 3 indolyl-, phenylethyl, methylthioethyl, 3 indolyl alkyl wherein the alkyl group contains 1 - 5 carbons, imidazolyl, imidazolylalkyl wherein the alkyl group contains 1 - 5 carbons, phenoxymethyl, phenylthiomethyl, 4-aminomethyl benzyl, 2-aminophenethyl, napthylethyl, 4-halophenethyl, 3,4-dihalophenethyl or phenoxyphenethyl, or

D. $R_1$ and $R_2$ together may form with -CH a lactone ring of the formula:

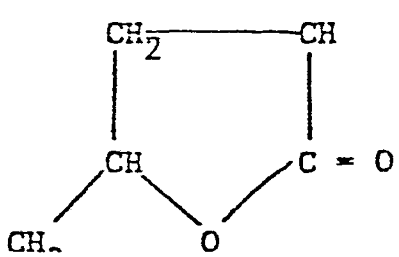

or

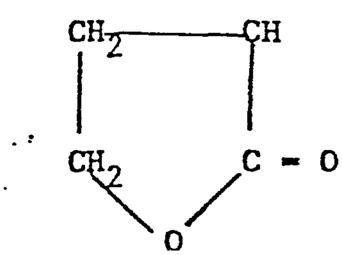

or an analogous six-membered ring and $R_3$ must then be selected from any of groups (i) - (xxxi) above.

E. When at least one of $R_7$ and $R_8$ is not hydrogen, or when X is $NR_9$ and $R_9$ is methyl, $R_1$ may be as defined in B above, or may be from any of groups (i) to (x) above, when $R_3$ is as defined in C above.

F. When $R_4$ and $R_5$ together are any structure that is disubstituted or monosubstituted with a moiety other than -OH, or when $R_4$ and $R_5$ form any recited structure other than of of

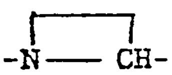, 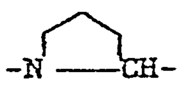, 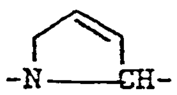, 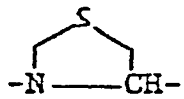, 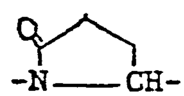, 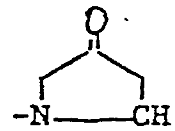

and 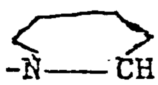, $R_1$ may be as defined in B or may be from any of groups (i) to (x) when $R_3$ is as defined in C above.

In the general formula above, asterisks indicate possible asymmetric centers. These centers may be racemized or in any optically active form. The S-form is preferred, however. It has been noted that compounds wherein X = $NR_9$, m = 0, and at least one of the carbons attached to the -N- of $NR_9$ exhibits asymmetry, are particularly effective ACE inhibitors.

The inhibitors are useful as orally effective antihypertensive agents.

DETAILED DESCRIPTION OF THE INVENTION

The invention in its broad aspects include thioether, ether, or secondary amine compounds whihc contain at least one amino acid or reltaed structure. The amino acid or related structure is selected either grom the group comprising, proline, 3,4-dehydroproline, 3-hydroxyproline, 4-hydroxyproline, 4-thioproline, thiazolidine-4-carboxylic acid, 4-keto-proline, 5-keto-proline, pyroglutamic acid, pipecolinic acid and azetidine-2-carboxylic acid wherein the carboxyl group of the amino acid or related structure can be replaced by a -COSH group or the amino acid or related structure contains the sequence $-N-\underset{(R_{10})_x}{CH}-\overset{O}{\overset{\|}{C}}-R_6$ wherein the -N- and $-\underset{(R_{10})_x}{CH}-$ are part of a ring with $R_4$ and $R_5$. The compounds of this invention are further characterized by the presence of at least one asymmetric carbon in the main chain shown by the general formula. In the most preferable form of compound according to this invention, at least one of $R_1$ and $R_3$ is a relatively large and bulky group.

The thioether compounds of formula I can be produced by several methods of synthesis. In the examples of synthesis which follow for the thioether as well as the ether and secondary amine compounds, proline will be utilized as the prototype amino acid moiety. It is to be understood that this is done for illustration purposes only and that the other structures can be substituted for proline in thesemethods

unless noted otherwise. According to one preferred method, the compound $R_1\underset{R_8}{C}(OH)COOC_2H_5$ is reacted with $P_2S_5$ to form the $R_1\underset{R_8}{C}(SH)COOC_2H_5$. The compound $CH_2 = \underset{R_7}{C}(R_3)COCl$ is reacted with Pro to form $CH_2=\underset{R_7}{C}(R_3)\overset{O}{C}$-Pro. The two products of these reactions are reacted together to yield the compounds $R_1\underset{R_8}{C}(COOC_2H_5)S-CH_2-\overset{R_7}{C}(R_3)-\overset{O}{C}$-Pro. Saponification removes the ethyl alcohol radical and forms the corresponding salt. The free acid can be formed therefrom by acidification. In this method $\Delta$Pro cannot be substituted for Pro.

In a second preferred method, the compound $R_1\overset{R_8}{C}(Br)COOH$ is

reacted with isobutylene in the presence of sulfuric acid to form the t-butyl ester of $R_1\overset{R_8}{C}(Br)COOH$. This compound is then reacted with $R_3\overset{R_7}{\underset{(CH_2)_m-SH}{C}}COOH$ to form the product

$R_1\overset{R_8}{C}(COOC(CH_3)_3)-S-(CH_2)_m-\overset{R_7}{C}(R_3)-\overset{O}{\overset{\|}{C}}OH$. Next, the t-butyl ester of Pro is coupled to this product using conventional coupling methods, such as the dicyclohexylcarbodiimide (DCC) method. Other coupling methods include the mixed anhydride, symmetrical anhydride, acid chloride, active ester, Woodward reagent K, of the like, methods. For a review of the coupling methods, see Methoden der Organischen Chemie (Houben-Weyl), Vol. XV, part II, page 1 et seq. (1974). The product formed,

$R_1\overset{R_8}{C}(COOC(CH_3)_3)-S-(CH_2)_m-\overset{R_7}{C}-(R_3)-\overset{O}{\overset{\|}{C}}-Pro$ t-butyl ester, is

deprotected, i.e. the t-butyl ester groups are removed by conventional means such as treatment with trifluoroacetic acid (TFA) and anisole to produce the desired end product. For a review of other deprotecting methods see Methoden der Organischen Chemie (Houben-Weyl), Vol. XV, part I, page 376 et seq. (1974). An alternative method is the reaction of

$R_1\underset{R_8}{C}(Br)COOH$ with $HS-(CH_2)_m-\underset{R_7}{C}(R_3)-\overset{O}{\overset{\|}{C}}-Pro$ to form the desired

product. This alternative method will not work for $\Delta$Pro although the original method will. $R_1\overset{R_8}{C}(Br)COOH$ can be prepared by reacting $R_1\overset{R_8}{C}(NH_2)COOH$ with HBr in the presence of $NaNO_2$ or with KBr in 2.5N $H_2SO_4$ in the presence of $NaNO_2$ to yield the desired product.

The ether compounds of formula I can also be prepared by several other methods. According to one preferred method, the compound is reacted with the diethyl ester of malonic acid to yield $R_1-CH(COOC_2H_5)_2$. This product is then reacted with bromine to produce $R_1-C(Br)(COOC_2H_5)_2$. The Compound $R_3-\overset{R_7}{\underset{(CH_2)_m-OH}{C}}-COOH$ is coupled to an ester of Pro using conventional coupling means. These two products are then reacted to form the compound $R_1CH(COOC_2H_5)_2-O-(CH_2)_m-\overset{R_7}{C}(R_3)-\overset{O}{\overset{\|}{C}}-Pro$ ester. The various ester groups are removed by conventional means and one carboxyl group is removed by acidification and heat to produce the desired product. By substituting $R_3-\overset{R_7}{\underset{(CH_2)_m-SH}{C}}-COOH$ for the $R_3-\overset{R_7}{\underset{(CH_2)_m-OH}{C}}-COOH$ and following this procedure, the thioether compounds can be formed but this will not work with $\Delta$Pro.

In still another method, the compound $R_1-C(R_2\text{.blocking group})(R_8)-OH$ is reacted with $Br-(CH_2)_m-C(R_7)(R_3)-COOH$ t-butyl ester to produce $R_1C(R_2\text{.blocking group})(R_8)-O-(CH_2)_m-C(R_7)(R_3)-COOH$ t-butyl ester. The ester is removed, the product reacted with Pro, and the blocking group is removed to yield a final product of the formula shown above.

The secondary amine compounds of this invention wherein $R_8$=H and X is NH may be synthesized by the following method. The compound $R_1\overset{O}{\overset{\|}{C}}-COOH$ is coupled with thiophenol using the mixed anhydride method to produce $R_1-\overset{O}{\overset{\|}{C}}-\overset{O}{\overset{\|}{C}}-S-C_6H_5$. This product is then reacted with $H_2N-(CH_2)_m-C(R_7)(R_3)-\overset{O}{\overset{\|}{C}}-Pro$ to yield $R_1CH(\overset{O}{\overset{\|}{C}}-S-C_6H_5)-NH-(CH_2)_m-C(R_7)(R_3)-\overset{O}{\overset{\|}{C}}-Pro$. This compound is reacted with NaSH to form $R_1-CH(\overset{O}{\overset{\|}{C}}-SH)-NH-(CH_2)_m-C(R_7)(R_3)-\overset{O}{\overset{\|}{C}}-Pro$.

Compounds of this invention in which $R_1$ and $R_2$ are bridged to form a lactone ring can be prepared using 2-halo-lactones, e.g., $\alpha$-Br-$\gamma$-valerolactone and $\alpha$-Br-$\gamma$-butyrolactone. The $\alpha$-bromo group is reactive with $HS-(CH_2)_m-CH(R_3)-\overset{O}{\overset{\|}{C}}-N(R_4)-CH(R_5)-COR_6$ or those analogs in which an $NH_2$-. or OH-group is substituted for the HS-group to form compounds in which X is -S-, -O- or -NH- in formula I.

$\Delta$Pro cannot be used in this procedure unless added as $-N(R_4)-CH(R_5)-COR_6$ as a final step, i.e., after the $CH(R_1)(R_2)-X$ bond has been formed. The lactone ring can be opened, e.g., with a base such as $Ba(OH)_2$ to form the corresponding $\gamma$-OH-1-carboxymethyl compounds. The hydroxy-group can be converted to a salt with sodium, potassium or an organic cation such as that from arginine

Compounds of this invention wherein $X=NR_9$ may be made in various ways. For example, an keto carboxylic acid of the general formula $R_3\overset{O}{\overset{\|}{C}}-COOH$ may be coupled to Pro to give a produce $R_3\overset{O}{\overset{\|}{C}}-\overset{O}{\overset{\|}{C}}-Pro$ using a conventional coupling agent such as dicyclohexylcarbodiimide ("DCC") or diphenylphosphorylazide ("DPPA"). This product may in turn be coupled, in the presence of a reducing agent such as sodium cyanoborohydride in aqueous solution with an organic solvent such as chloroform or dichloromethane, to a compound of the general formula $R_8\underset{R_2}{\overset{R_1}{C}}-NHR_9$ to give the desired compound. Alternatively, $R_3\underset{O}{\underset{\|}{C}}-COOH$ may first be coupled with $R_8\underset{R_2}{\overset{R_1}{C}}-NHR_9$ and the product then coupled to Pro in a second coupling step. As those of ordinary skill in the art will readily understand, conventional blocking roups such as Boc, CbO, etc., may be introduced at appropriate stages to protect reactive groups, especially carboxyl or amido groups, and may be removed as desired.

In another general method a suitable α keto carboxylic acid $R_1\overset{O}{\overset{\|}{C}}-COOH$ is coupled to $H_2N-(CH_2)_m-\underset{R_7}{\overset{R_3}{C}}-COOH$ and the product is then coupled to Pro or alternatively $H_2N(CH_2)_m-\underset{R_7}{\overset{R_3}{C}}-COOH$ is coupled to Pro and that product is coupled to $R_1\overset{O}{\overset{\|}{C}}-COOH$ to give an end product of this invention.

Suitable compounds of the general formula $R_8-\underset{R_2}{\overset{R_1}{C}}-NH_2$ for use in making the compounds of the invention include, but are not limited to glutamic acid α-benzyl ester, glutamic acid α-ethyl ester, glutamic acid α-methyl ester, glutamic acid α-tert butyl

ester, aspactic-α-benzyl ester, 2-amino-5-carboxy-indan-2-carboxylic acid, p-carboxyphenylalanine-α- methyl ester, o-carboxytyrosine-α-methyl ester, 2-aminomalonic acid methyl ester, 2 aminoadipic acid-1-ethylester, 2 amino-pimelic acid-1 ethyl ester, 2-aminosuberic acid-1 ethyl ester, 2-amino azelaic acid-1-ethyl ester, 2-amino sebacic acid-1 ethyl ester, tert. leucine, 2-methylglutamic acid, α-amino-γ-guanidino butyric acid, α-amino-β-guanidinopropionic acid, β-fluorophenylalanine, β-hydroxyvaline, γ-oxalysine, γ-hydroxy ornithine, $N^{ε}$-hydroxylysine, γ-N methyl arginine, γ-hydroxy arginine, cancanin, 5,5'-dihydroxyleucine, β-carboxyaspartic acid, β-fluoroaspartic acid, β-methylaspartic acid, β-methylene aspartic acid, p-amidino phenylalanine, p-guanidinophenylalanine, p-methylphenylalanine, (2-ethoxy-5-nitro) phenyl-alanine, (2-hydroxy-5-nitro) phenylalanine, (4-mercapto) phenylalanine, 2-amino-2-indoleacetic acid, 2-amino-3-adamantyl-propionic acid, β-methylene norvaline, α-amino-γ-(4 carboxythiazolyl) butyric acid, 3-chloroglutamic acid, α-amino-δ-nitro-valeric acid, 4-azalysine, (2,4,5-trihydroxyphenyl)alanine, (3 bromo-5-methoxypehnyl) alanine, β-(3,5 dimethyl-4 methoxyphenyl)alanine, 3,5-di (ethylthio)-4-(4' hydroxyphenoxy)-phenylalanine, 3,5-di(ethylthio)-4(3'-isopropyl-4'-methoxyphenoxy)phenylalanine, β-pyrrolyl-alanine, 2-amino-4-pyrrolyl-butyric acid, 2-amino-5 pyrrolyl-valeric acid, β-(2 pyridyl)alanine, β-(3 pyridyl) alanine, β(6-aminopurin-9yl)alanine, β-(4-amino-2-hydroxy-pyrimidin-1-yl)alanine, β-(2,4-dihydroxy-5-methyl-pyrimidin-1-yl)alanine, β-(6-hydroxy-purin-9-yl)alanine, β-(6-dimethyl-amino-purin-9-yl)alanine, β-(6-mercaptopurin-9-yl)alanine, β-(6-methylthiopurin-9-yl)alanine. 4-azatryptophan. 4-methyl-

oxo-1-pyridyl)lysine, S-(2-hydroxy-2-carboxyethanethiomethyl)-cysteine, 2-amino-3-(6-thieno [3,2-b]pyrrolyl)-propionic acid, 3,3',5,5' tetramethylthyronine, δ-hydroxy-β-lysine, 2-aminohex-4-ynoic acid, N$^{\delta}$-hydroxyornithine, 4-piperazinobut-2-ynoic acid, 4-piperidinobut-2-ynoic acid, 4-pyrrolidinobut-2-ynoic acid, α-amino-γ-nitroguanidinobutyric acid, α-amino-β(1-imidazolyl) propionic acid, 4-nitrohistidine, 2-methyl-3(2',4'-diiodo-5'-hydroxyphenyl) alanine, 4-(3' amino-2',4',6'-triiodophenyl) isovaline, 4-(3' acetamido-2',4',6'-triiodophenyl)-isovaline, 4-(3'-hydroxy-2',4',6'-triiodophenyl) isovaline, 2-amino-4-thiosulfobutyric acid, S-(3-aminopropyl) homocysteine, S-(cyclopentyl methyl) homocysteine, S-(cyclopentyl methyl) homocysteine, 5'-guanosylhomocysteine, β (cytosin-1-yl)-alanine, -S-[(diphenyl-α-naphthyl)methyl]-L-cysteine, -S-[(diphenyl-β-naphthyl)methyl]-L-cysteine, 2-amino-6-(methylthio) caproic acid, $N^{\varepsilon}$, $N^{G}$-dimethyl-L-arginine, $N^{G}$, $N'^{G}$-dimethyl-L-arginine, $N^{\varepsilon}N^{\varepsilon}N^{\varepsilon}$-trimethyl-δ-hydroxy-L-lysine, $N^{\varepsilon}$-(5-amino-5-carboxypentyl)-5-hydroxy-L-lysine, δε-dihydroxy-L-norleucine, cis-1-amino-1,3-dicarboxycyclohexane, trans-1-amino-1,3-dicarboxycyclohexane, 3,3,4,4,4,-pentafluoro-2-aminobutyric acid, 3,3,4,4,5,5,5-heptafluoro-2-aminovaleric acid, ω-fluoro-D,L- and L-allo-isoleucine, 2,6-diamino-4-hexynoic acid, O-(α-D-glucopyranosyl)-L-serine, 2-amino-5,6-dihydroxyindan-2-carboxylic acid, 3-(m-fluorophenyl)-2-methylalanine, 3-(m-bromophenyl)-2-methylalanine, 3-(m-iodophenyl)-2-methylalanine, 2-[(m-iodophenyl)methyl]glycine, -4-(m-iodophenyl)-2-methyl-2-aminobutyric acid, 3,5,3'-tri-isopropyl-DL-thyronine, 3,5-dimethyl-3'-isopropyl-thyronine, 3,5-di-isopropyl-thyronine, 3,5-di-isopropyl-4'amino-thyronine, 3,5,-di-isopropyl-3'-bromo-thyronine, 3,5-di-isopropyl-3'methyl-thyronine, 3,5-di-s-butyl-

thyronine, 3,5-di-s-butyl-4'-amino-thyronine, 3,5-di-s-butyl-3'-bromo-thyronine, 3,5-di-s-butyl-3'-iodo-thyronine, 4-fluoro-tryptophan, 5-fluoro-tryptophan, 6-fluoro-tryptophan, β-(5-hydroxy-6-iodo-2-pyridyl)-alanine, β-(benzimidazol-5-yl)-alanine, β-(2-amino-6-hydroxypurin-9-yl)-alanine, β-(2-amino-6-mercaptopurin-9-yl)-alanine, $N^{ε}$-(5-Amino-6-chloro-4-pyrimidyl) lysine, -Amino- -(6-chloro-9-purinyl)caproic acid, 4-Fluoro-DL-histidine, S-Methyl-2-methyl-cysteine, S-Ethyl-2-methyl-cysteine, S-Propyl-2-methyl-cysteine, S-Isopropyl-2-methyl-cysteine, S-Butyl-2-methyl-cysteine, S-Isobutyl-2-methyl-cysteine, S-t-Butyl-2-methyl-cysteine, S-Amyl-2-methyl-cysteine, S-Isoamyl-2-methyl-cysteine, S-Allyl-2-methyl-cysteine, S-(β-Aminoethyl) homocysteine, γ,δ,δ'-trihydroxy-leucine, $N^{ε}$-(indole-3-acetyl)-lysine, p-hydroxymethylphenylalanine O-ethylhomoserine, 5-methyl-2-aminohex-4-enoic acid, α-(3-hydroxyphenyl)glycine, α-(3,5-dihydroxyphenyl)glycine, β-(cyclohexa-1,4-dienyl)alanine, β-cyclohex-1-enyl)-alanine, β-(1-hydroxycyclohexyl)-alanine, 4-bromoacetyl-phenylalanine, 4-bromoacetamido-phenylalanine, 3-chloroacetamido-phenylalanine, 4-fluoro-3-chloroacetamido-phenylalanine, 3,4,5-tri-iodo-phenylalanine, 3,5-di-isopropyl-3'-iodo-thyronine, β-(4-methoxy-1-naphthyl)-α-methylalanine, β-(4-hydroxy-1-naphthyl)-α-methylalanine, α-(2-indanyl)glycine, β-trimethylsilyl-alanine, α-amino-β-(methylamino)propionic acid, $N^{ε}N^{ε}$-bis(2-cyanoethyl)-lysine, α,δ-dimethylnorleucine, α-methyl-$N^{δ}N^{δ}$-diethylornithine, α-ethyl-3,4-dimethoxy-phenylalanine, α-methyl-4-morpholino-phenylalanine, β-(2-amino-4-pyrimidinyl) alanine, β-(2-amino-4-pyrimidinyl)alanine,3-(2-Methyl-4,5-dihydroxyphenyl)-alanine, 3-(2-Ethyl-4,5-dihydroxyphenyl)-alanine, 3-(2-Isopropyl-4,5-dihydroxyphenyl)-alanine, 3-(2-t-Butyl-4,5-dihydroxyphenyl)-alanine, 3-(2,5-Dimethoxy-4-methylphenyl)-alanine, 3-Ethyl-α-methyl-tyrosine, 2-amino-3,3-dimethylhex-5-enoic acid, 2-aminohexa-4,5-dienoic acid, 2-amino-3,3-dimethylhexa-4,5-dienoic acid, 2-aminohepta-4,5-dienoic acid, 2-amino-3,3-dimethylnona-4,5-dienoic acid, 2-aminohepta-5,6-dienoic acid, 2-amino-3-methylhepta-5,6-dienoic acid, 2-amino-5-t-butyl-6,6-dimethylhepta-3,4-dienoic acid, 2-amino-5-methylhepta-3,4-dienoic acid, 2-aminohept-4-en-6-ynoic acid, ε-hydroxy-β-carboxy-norleucine, β-carboxy-lysine, B-(3,4-dihydroxyphenyl)-α-methyl-serine, S-benzyl-β,γ-dimethyl-homocysteine, S-benzyl-α,γ,γ-trimethyl-homocysteine, β-methyl-methionone, α-methyl-selenomethionine, β-methyl-selenomethionine, α-methyl-selenomethionine, γ,γ'-difluoro-valine, δ,δ,'-difluoro-leucine, γ-fluoro-allothreonine, β-hydroxy-asparagine, β-hydroxy-isoleucine, β-methoxy-isoleucine, α-amino-γ-(methylamino)butyric acid, α-amino-β-(ethylamino)propionic acid,3-Isopropyl-α-methyl-tyrosine, 3-t-Butyl-α-methyl-tyrosine, 2-Amino-5-hydroxy-indan-2-carboxylic acid, 2-Amino-5-methoxy-indan-2-carboxylic acid, 2-Amino-5-carboxy-indan-2-carboxylic acid, 2-Amino-5-chloro-indan-2-carboxylic acid, 2-Amino-5-bromo-indan-2-carboxylic acid, 2-Amino-5-iodo-indan-2-carboxylic acid, 3-(2,4-Difluorophenyl)-alanine, 3-(3,4-Difluorophenyl-alanine, 3-(3,5-Difluorophenyl)-alanine, 3-(2,5-Difluorophenyl)-alanine, 3-(2,6-(Difluorophenyl)alanine, 3-(2,3,5,6-Tetrafluorophenyl)-alanine, 3-(3,5-Dichloro-2,4,6-trifluorophenyl)alanine, 3-(2,3,4,5,6-Pentafluorophenyl)-alanine, β-(1,2-Dihydro-2-oxo-3-pyridyl)-alanine, β-(1,2-Dihydro-2-oxo-4-pyridyl)-alanine, β-(1,2-Dihydro-2-oxo-5-]yridyl)-alanine, β-1,2-Dihydro-2-oxo-6 pyrydil)-alanine, β-(2-Fluoro-3-pyridyl)-alanine, β-(2-Fluoro-5-pyridyl)-alanine, β-(2-Fluoro-6-pyridyl)-Alanine, β-(2-Bromo--3-]yridyl)-alanine, β-(2-Bromo-4-pyridyl)-alanine, β-(2-Bromo-5-]yridyl)-alanine, β-(2-Bromo-6-]yridyl)-alanine, β-(2-Chloro-3-pyridyl)-alanine, β-(2-Chloro-4-pyridyl)-alanine, β-(2-Chloro-5-pyridyl)-alanine, β-(2-Chloro-6-pyridyl)-alanine, β-(Thymin-

1-yl)-alanine.

It is further contemplated that

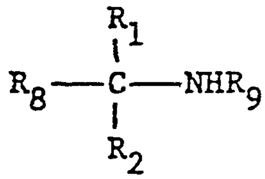

may be

selected from among any of the known amino acids or esters or primary amides thereof in which, when $R_1$ is any of $CH_3$, $NH_2-(CH_2)_3$, $NH_2(CH_2)_4-$, $CH_3S(CH_2)_2-$, benzyl, p-hydroxybenzyl, 3,4-dimethoxybenzyl, $CH_3O\overset{O}{\overset{\|}{C}}-(CH_2)_2-$ or 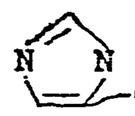$-CH_2$, $R_8$ is selected from among $CH_3$, $(CH_3)_2$ $CHCH_2-$, $PhtN(CH_2)_3$, $CH_2=CH-CH_2-$, benzyl-, nitrilomethylene-, $CH_3-O-\overset{O}{\overset{\|}{C}}-CH_2-$, $CH_3OCH_2-$, $CH_3SCH_2-$, $CH_2F-$, $CHF_2-$, $CF_3-$, $CH_2Cl-$, $CF_2Br-$, $PhtN(CH_2)_2)$, $CH_3\overset{O}{\overset{\|}{C}}-S(CH_2)_3$, $HS(CH_2)_3-$, or

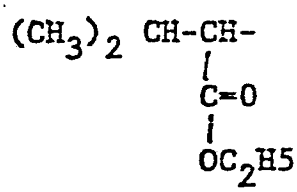

There are also known aminoacids, and esters or primary amides thereof in which when $R_8$ is hydroxymethyl, $R_1$ may be methyl, ethyl, isopropyl, isobutyl, phenyl, benzyl or methylthioethyl.

It is contemplated that reactants of the general formula

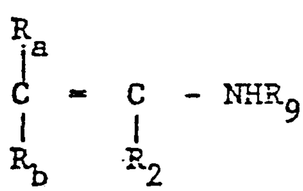

wherein

$R_2$ is COOH may be utilized in lieu of

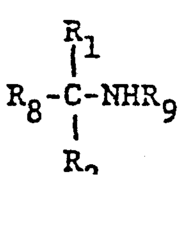

in the coupling reaction with

$R_3C(=O)-C(=O)-OH$ or its coupling product already described.

In such cases, $R_a(R_b)C=C(R_2)-NHR_9$

may be, e.g. dehydroalanine, α,β -dehydrophenylalanine, vinylglycine or a known compound in which $R_a$ and $R_b$ are both methyl or ethyl or $R_a$ is phenyl or a substituted phenyl group such as 3,4 dimethoxyphenyl and $R_b$ is methyl. In this instance various functional groups such as halo, hydroxy or mercapto groups and their methylene analogs, may later be added to one or both carbons of the unsaturated bond via well-known and conventional organic chemical procedures.

Many suitable variations in $R_8-C(R_1)(R_2)-NH_2$ will readily occur to those of ordinary skill in the art.

It is to be understood that $R_8-C(R_1)(R_2)-NHR_9$, including any of those specifically named, may be used as $NH_2(CH_2)_m-C(R_3)(R_7)-COOH$ in processes herein disclosed when $R_9$ is H and $R_2$ is COOH. In such cases, $R_1$ becomes $R_3$, and $R_8$ becomes $R_7$ and m=0.

Compounds $R_1C(=O)-COOH$ or $R_3C(=O)-COOH$ used in any of the procedures disclosed herein may be selected from known keto carboxylic acids, including but not limited to

pyruvic acid,

phenylpyruvic acid,

3-cyclohexyl-2-oxopropionic acid,

6-methyl-2-oxoheptanoic acid,

4-methyl-1-oxopentanoic acid,

2-oxobutyric acid,

3-methyl-2-oxobutyric acid,

2-oxoglutaric acid,

2-oxoadipic acid,

2-oxo-4-phenylbutyric acid,

4-(3-indolyl)-2-oxobutyric acid,

N-acetylaminoethyl-2-oxo-4-phenylbutyrate,

dimethylaminoethyl-2-oxo-4-phenylbutyrate,

2-oxo-5-methylhexanoate,

phenoxypyruvic acid,

phenylthiopyruvic acid,

4-p-chlorophenyl-2-oxobutyrate,

indole-3-pyruvic acid,

2-oxo-3-p-cyanophenylpropionate,
4-α-naphthyl-2-oxobutyrate
4-(3,4,-dichlorophenyl)-2-oxo-butyrate, or

2-oxo-4-p-phenoxyphenylbutyric acid and others readily apparent to those of ordinary skill in the art.

Another versatile method for synthesizing the compounds of this invention having a carbonyl or hydroxy substituent on $R_1$ or $R_3$ side chains involves the use of a diazomethyl intermediate. See for example, Aldrichimica Acta 3(4), 9 (1970), an article available on request from Aldrich Chemical Co., Milwaukee, Wisconsin 53233, and Boyer, J.H. et al Chem. Rev. 54, 1-57(1954). Typically a carboxylic acid is reacted with diazomethane via a mixed anhydride reaction:

$$R\text{-}\overset{\overset{O}{\parallel}}{C}\text{-}OH \quad + \quad CH_2N_2 \longrightarrow R\text{-}\overset{\overset{O}{\parallel}}{C}\text{-}CH_2\text{-}N_2$$

The product is then reacted with an acid such as HBr or HCl, in a solvent such as ethyl acetate, to form an α-halomethyl-ketone,

$$R-\overset{O}{\overset{\|}{C}}-CH_2-Br$$

This product can then be reacted with an equivalent of diethylformamidomalonate, then decarboxylated in aqueous HCl to form derivatives of 2-amino-4-keto carboxylic acid, that is compounds of the formula $R-\overset{O}{\overset{\|}{C}}-CH_2-\overset{H}{\underset{NH_2}{C}}-COOH$.

Such compounds of the general formula can then be coupled with compounds of the general formula $O=\overset{R_3}{C}-\overset{O}{\overset{\|}{C}}-\overset{R_4}{N}-\overset{R_5}{CH}-\overset{O}{\overset{\|}{C}}-R_6$ in the presence of a reducing agent such as sodium cyanoborohydride to form compounds of this invention. Alternatively, $R\underset{O}{\underset{\|}{C}}-CH_2-\overset{H}{\underset{NH_2}{C}}-COOH$ can be coupled with $R_3-\underset{O}{\underset{\|}{C}}-COOH$ to form

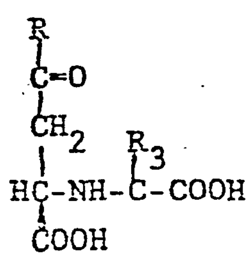

which in turn may be coupled with Pro in the presence of DCC or DPPA to form compounds of this invention.

The diazomethyl intermediate can be formed with virtually any carboxylated organic compound, e.g., $R-\overset{O}{\overset{\|}{C}}-OH$ can be a difunctional amino acid, a trifunctional amino acid, any

dicarboxylic acid or any carboxylic acid. Appropriate protecting groups may be used as needed. Hydroxy derivatives are synthesized by conventional reduction of corresponding keto analogs.

This type of reaction is especially useful when R signifies a $HOOC-CH_2-$, $HOCH_2-$ or $HSCH_2-$, it is contemplated in such instances that the named functional groups may be further reacted to form esters, thioesters, ethers, "reverse" esters or thioesters, amides or "reverse" amides and the like.

The compounds of this invention have one or more asymmetric carbons as indicated by the asterisks in the general formula. The compounds accordingly exist in stereoisomeric forms or in racemic mixtures thereof. All of these are within the scope of the invention. The above described synthesis can utilize a racemate or one of the enantiomers as starting material. When the racemic starting material is used in the synthetic procedure or a racemic mixture results from the synthesis, the stereoisomers obtained in the product can be separated by conventional chromatographic or fractional crystallization methods. In general, the S- isomer with respect to the carbon bearing $R_1$ constitutes the preferred isomeric form. Also the S- isomer with respect to the carbon bearing $R_3$ is preferred, as is the S- isomer of the carbon bearing $R_5$.

The compounds of this invention form basic salts with various inorganic and organic bases which are also within the scope of the invention. Such salts include ammonium salts, alkali metal salts like sodium and potassium salts (which are preferred), alkaline earth metal salts like the calcium and magnesium salts, salts with organic bases, e.g., dicyclohexylamine, benzathine, N-methyl-D-glucamine, procaine salts,

salts with amino acids like arginine, lysine and the like. The non-toxic, physiologically acceptable salts are preferred.

The salts are formed in conventional manner by reacting the free acid form of the product with one or more equivalents of the appropriate base providing the desired cation in a solvent or medium in which the salt is insoluble, or in water and removing the water by freeze drying. By neutralizing the salt with an insoluble acid like a cation exchange resin in the hydrogen form (e.g., polystyrene sulfonic acid resin like Dowex 50) or with aqueous acid and extraction with an organic solvent, e.g., ethyl acetate, dichloromethane or the like, the free acid form can be obtained, and, if desired, another salt formed.

Additional experimental details are found in the examples which are preferred embodiments and also serve as models for the preparation of other members of the group.

The compounds of this invention inhibit the conversion of the decapeptide angiotensin I to angiotensin II and therefore are useful in reducing or relieving angiotensin related hypertension. The action of the enzyme renin on angiotensinogen, a pseudoglobulin in blood plasma, produces angiotensin I. Angiotensin I is converted by angiotensin converting enzyme (ACE) to angiotensin II. The latter is an active pressor substance which has been implicated as the causative agent in various forms of hypertension in various mammalian species, e.g., rats and dogs. The compounds of this invention intervene in the angiotensin $\xrightarrow{\text{(renin)}}$ angiotensin I $\xrightarrow{\text{(ACE)}}$ angiotensin II sequence by inhibiting angiotensin converting enzyme and reducing or eliminating the formation of the pressor substance angiotensin II. Thus by the administration of a composition

including their physiologically acceptable salts, angiotensin-dependent hypertension in the species of mammal suffering therefrom is alleviated. A single dose, or in some cases up to two to four divided daily doses, provided on a basis of about 0.03 to 20 mg. per kilogram per day, is appropriate to reduce blood pressure. The substance is preferably administere orally, but parenteral routes such as subcutaneous, intramuscul intravenous or intraperitoneal can also be employed.

The compounds of this invention can be utilized to achieve the reduction of blood pressure by formulating them in compositions such as tablets, capsults or elixirs for oral administration or in sterile solutions or suspensions for parenteral administration. About 10 to 500 mg. of a compound or mixture of compounds of formula I, including the physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to

instance, tablets may be coated wtih shellac, sugar or both. A syrup or elixir may contain the active compound, sucrose as a sweetening agent, methyl and propyl parabens as preservatives, a dye and a flavoring such as cherry or orange flavor. Antioxidants may also be added. Suitable antioxidants are α-tocopherol nicotinate, vitamin A, C, E and analogs of vitamin E known in the art, retinal palmitate and other antioxidants known in the art as food additives such as the gallates.

Sterile compositions for injection can be formulated according to conventional pharmaceutical practice by dissolving or suspending the active substance in a vehicle such as water, a naturally occurring vegetable oil like sesame oil, coconut oil, peanut oil, cottonseed oil, etc., or a synthetic fatty vehicle like ethyl oleate or the like. Buffers preservatives, and the like can be incorporated as required.

The present invention will be further described by the following examples. All temperatures are in degrees Celsius unless otherwise indicated. Molar equivalents of the reactants are usually utilized.

Example 1

Synthesis of N-(1-carboxy-3-keto-5-phenylpentyl)-Ala-L-thiazolidine-2-carboxylic acid

A. Hydrocinnamic acid, 40 μmoles, in ethyl acetate is reacted at -30°C with diazomethane via the mixed anhydride reaction to yield 1-diazo-2-keto-4-phenylbutane. The latter compound is converted to the desired product on reaction with HBr in ethyl acetate, and the product 1-bromo-2-keto-4 phenylbutane is isolated by fractional crystallization.

B. The product of Step A, 10 μ moles, in ethanol is reacted with diethylformamidomalonate, 10 μ moles, in the presence of KI, 10 moles, and sodium ethoxide, 10 μ moles, at reflux temp for 10 hours. The resultant compound is decarboxylated in 6N HCl. The product, the HCl salt of 2 amino-4-keto-6 phenylhexanoic acid, is isolated by crystallization from $H_2O$/ethanol.

C. The product of Step B, 20 moles, in ethanol/$H_2O$ containing molecular sieves and 2 equivalents of $NaHCO_3$, is reacted with 200 moles of pyruvoyl-L-thiazolidine-4-carboxylic acid in the presence of 22 moles of sodium cyanoborohydride for 24 hrs. at room temperature. The desired product, N-(1-carboxy-3-keto-phenyl-5pentyl)-Ala-L-thiazolidine-4-carboxylic acid, is obtained by conventional chromatography.

Example 2

Synthesis of N-(1-carboxy-1-methyl-3-keto-5-phenyl-pentyl)-Ala-L-thiazolidine-4-carboxylic acid

A. Hydrochloride solution of the methylester of 2-amino-4-keto-phenyl hexanoic acid from Step B, Example 1, 30 m/mols and 6.36 g. (60 m. mols) of freshly distilled benzaldehyde in 20 m. of methylene chloride is cooled to 0°C. and there is slowly added a solution of 3.03 g. (30 m. mols) of triethyl amine in 7 ml. of methylene chloride. The reaction mixture is

allowed to stand overnight at room temperature with stirring. After removal of the solvent, the residue is taken up in ethyl acetate. The oil is washed with water and saturated sodium chloride and then dried over magnesium sulfate and concentrated to give the methylester of 2-(benzylidene amino)-4-keto-6-phenyl-hexanoic acid.

B. To a solution of lithium diisopropyl amide (20 m. mols) in tetrahydrofuran, cooled to -73°C. and magnetically stirred under a blanket of nitrogen, is added a solution of 18 m. mols of the methylester of the product of Step A hereof in 30 ml. of tetrahydrofuran. At the end of the addition, the cooling bath is replaced by a warm water bath. When the temperature of the reaction mixture reaches 40°C. the nitrogen inlay is replaced by an expandable ballon. The reaction mixture is then saturated with iodomethane by passing a rapid stream of iodomethane through the solution while the temperature is maintained between 40° and 50°C. After the misture is slurried for one hour under a freon atmosphere, the mixture is quenched with brine and extracted with ether to give the methylester of 2 methyl-2-(benzylidene amino)-4-keto-6 phenyl acid.

C. A mixture of the product of Step B in 10 ml. of ether and 55 ml. of 2N aqueous HCl is vigorously stirred at room temperature for three hours. The aqueous phase is decanted, washed with ether and concentrated in vacuo. The crystalline residue is washed with methanol and ether to give the methylster of 2-amino-4-keto-2-methyl-6-phenyl hexanoic acid.

Examples 3 - 5

Example 1 is repeated several times, substituting for pyruvoyl-L-thiazolidine -4-carboxylic acid in step C an acid as shown in column A. The product is analogous to that of Example 1 but has the $R_3$ group shown in column B:

| Example | A | B ($R_3$) |
|---|---|---|
| 3 | 3-phenylpyruvoyl-L-thiazolidine-4-Carboxylic acid | 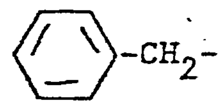 |
| 4 | -keto-4-phenylbutyryl-L-thiazolidine-4-carboxylic acid | 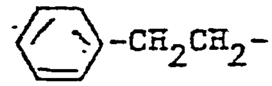 |
| 5 | -keto-glutaryl-L-thiazolidine-4-carboxylic acid | $HOOC-CH_2CH_2$ |

Examples 6 - 8

Example 2 is repeated several times, substituting for pyruvoyl-L-thiazolidine-4-carboxylic acid in Step B the acids of Examples 3 - 5. Each of the products differs from the analogous product of Examples 3 -5 respectively in that $R_8$= methyl in each of them.

Examples 9 - 102

Example 1 is repeated a number of times, substituting for
Example 1 is repeated a number of times, substituting for
pryuvoyl-L-thiazolidine-4-carboxylic acid in Step C an acid as shown in Column A. The product in each instance contains the

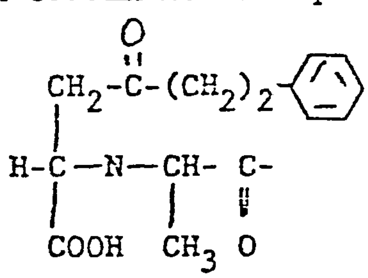

group of Example 1, but t is $-\overset{R_4}{N}-\overset{R_5}{C}-\overset{O}{\overset{\|}{C}}-R_6$ moiety is as shown in column B:

| Ex. No. | A | B |
|---|---|---|
| 9 | pyruvoyl-L-Pro | 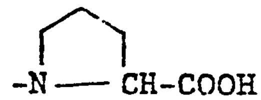 |
| 10 | pyruvoyl-L- Glu | 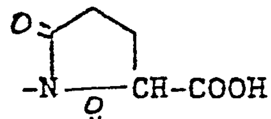 |
| 11 | pyruvoyl-4-keto-L-Pro | 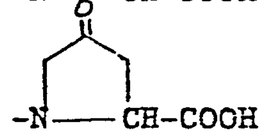 |
| 12 | pyruvoyl-3-keto-L-Pro | 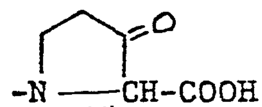 |
| 13 | pyruvoyl-4 hydroxy-L-Pro | 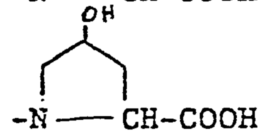 |
| 14 | pyruvoyl 3 hydroxy-L-Pro | 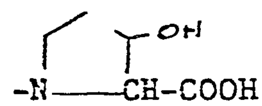 |
| 15 | pyruvoyl-L-azetidine-2-carboxylic acid | 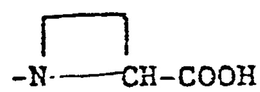 |
| 16 | pyruvoyl-L-pipecolinic acid | 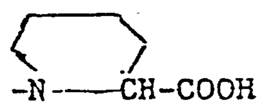 |
| 17 | pyruvoyl-4-methoxy-L-Pro | 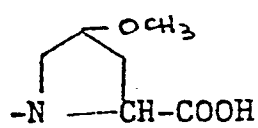 |
| 18 | pyruvoyl-4-bromo-L-Pro | 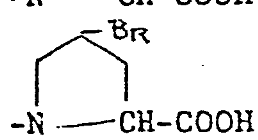 |
| 19 | pyruvoyl-3-methoxy-L-Pro | 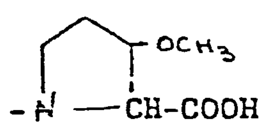 |

| Ex. No. | A | B |
| --- | --- | --- |
| 20 | - pyruvoyl-4-bromo-L-Pro | 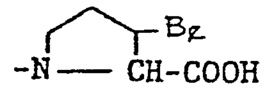 |
| 21 | - pyruvoyl-3-fluoro-L-Pro | 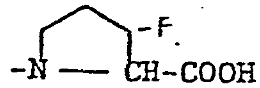 |
| 22 | - pyruvoyl-5-fluoro-L-Pro | 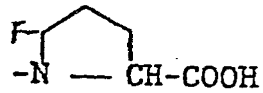 |
| 23 | - pyruvoyl-5-methoxy-L-Pro | 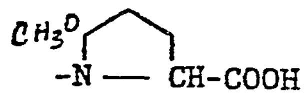 |
| 24 | - pyruvoyl-5-hydroxy-L-Pro | 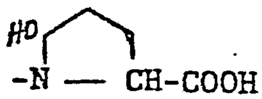 |
| 25 | - pyruvoyl-5-iodo-L-Pro | 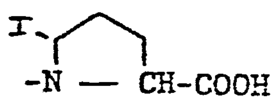 |
| 26 | - pyruvoyl-5-bromo-L-Pro | 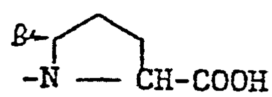 |
| 27 | - pyruvoyl-5-chloro-L-Pro | 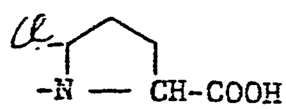 |
| 28 | - pyruvoyl-4-chloro-L-Pro | 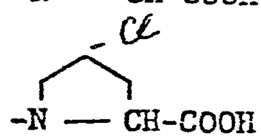 |
| 29 | - pyruvoyl-4-fluoro-L-Pro | 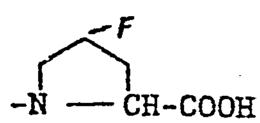 |
| 30 | - pyruvoyl-4-iodo-L-Pro | 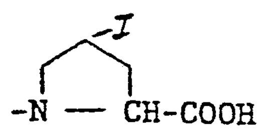 |
| 31 | - pyruvoyl-3-chloro-L-Pro | 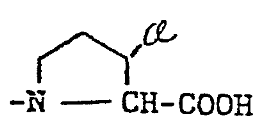 |
| 32 | - pyruvoyl-3-fluoro-L-Pro | 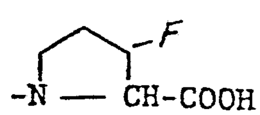 |
| 33 | - pyruvoyl-3-iodo-L-Pro | 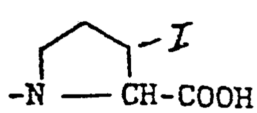 |

0048159

| Ex. No. | A | B |
|---|---|---|
| 34 | - pyruvoyl-3-hydroxy∠Glu | 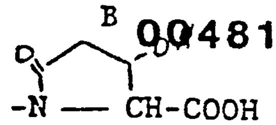 |
| 35 | - pyruvoyl-3,4-dihydroxy-L-Pro | 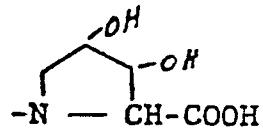 |
| 36 | - pyruvoyl-3,5-difluoro-L-Pro | 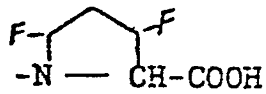 |
| 37 | - pyruvoyl-3,4-diodo-L-Pro | 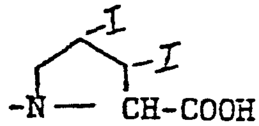 |
| 38 | - pyruvoyl-4,5-dichloro-L-Pro | 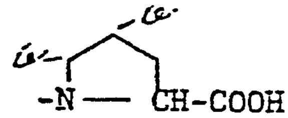 |
| 39 | - pyruvoyl-3-chloro-4-bromo-L-Pro | 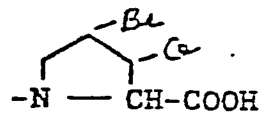 |
| 40 | - pyruvoyl-3,5-dibromo-L-Pro | 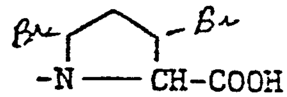 |
| 41 | - pyruvoyl-3-fluoro-5-hydroxy-L-Pro | 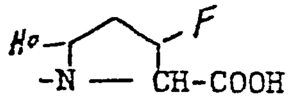 |
| 42 | - pyruvoyl-3-chloro-4-hydroxy-L-Pro | 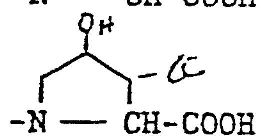 |
| 43 | - pyruvoyl-4-mercapto-L-Pro | 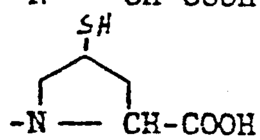 |
| 44 | - pyruvoyl-3-thio-L-Pro | 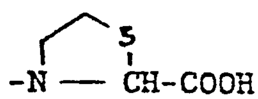 |
| 45 | - pyruvoyl-3-thioxo-L-Pro | 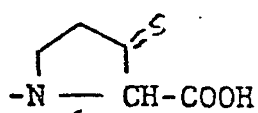 |
| 46 | - pyruvoyl-4-thioxo-L-Pro | 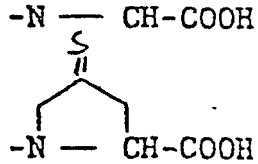 |
| 47 | - pyruvoyl-5-thio-L-Pro | 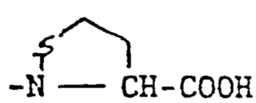 |
| 48 | - pyruvoyl-5-thioxo-L-Pro | 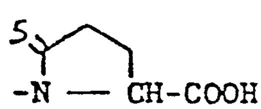 |
| | | 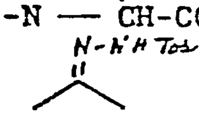 |

| Ex. No. | A | B |
|---|---|---|
| 50 | pyruvoyl-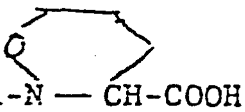 | 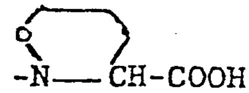 |
| 51 | pyruvoyl-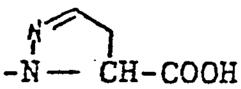 | 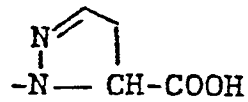 |
| 52 | pyruvoyl-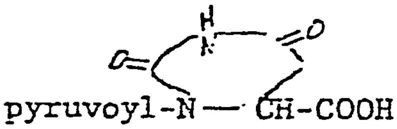 | 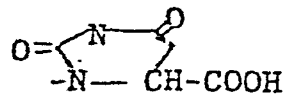 |
| 53 | pyruvoyl-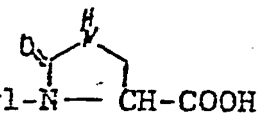 | 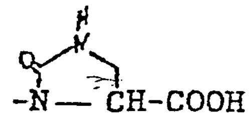 |
| 54 | pyruvoyl-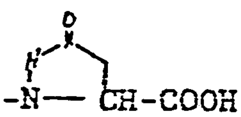 | 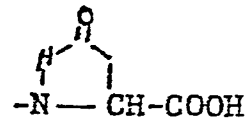 |
| 55 | pyruvoyl-3-carboxy-L-Pro | 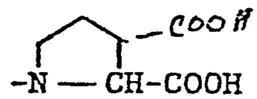 |
| 56 | pyruvoyl-4-phenylthio -L-Pro | 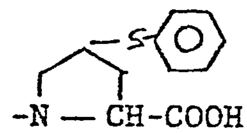 |
| 57 | pyruvoyl-5-hydroxyphenyl-L-Pro | 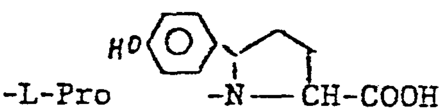 |
| 58 | pyruvoyl-3-phenoxy-L-Pro | 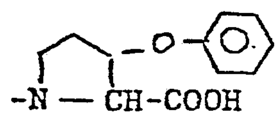 |
| 59 | pyruvoyl-5-benzyloxy-L-Pro | 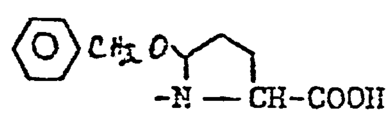 |
| 60 | pyruvoyl-3-methylthio-5-keto-L-Pro | 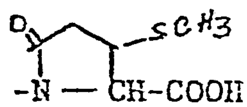 |
| 61 | pyruvoyl-4-guanidino-L-Pro | 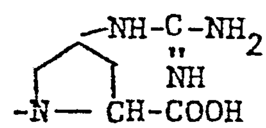 |
| 62 | pyruvoyl-3-aminomethyl-L-pipecolinic acid | 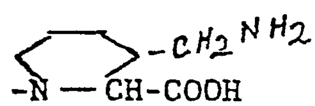 |
| 63 | pyruvoyl-4-methylthio-L-pipecolinic acid | 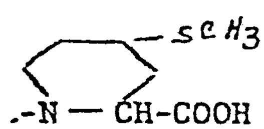 |

| Ex. No. | A | B |
|---|---|---|
| 64 | pyruvoyl-3-methylamino-L-Pro | 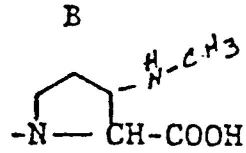 |
| 65 | pyruvoyl-5-phenyl-L-Pro | 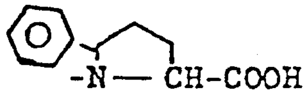 |
| 66 | pyruvoyl-4-propyl-L-Pro | 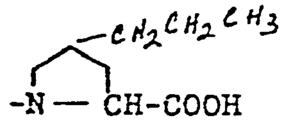 |
| 67 | pyruvoyl-3-methyl-L-Pro | 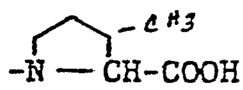 |
| 68 | pyruvoyl-4-methylene-L-Pro | 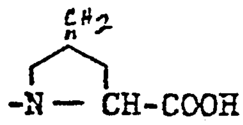 |
| 69 | pyruvoyl-3-benzyloxy-4-keto-L-Pro | 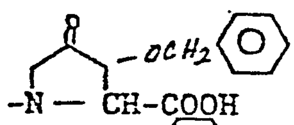 |
| 70 | pyruvoyl-4-benzylthio-3-keto-L-Pro | 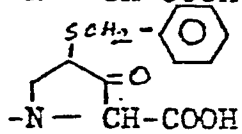 |
| 71 | pyruvoyl-5-acetoxy L-Pro | 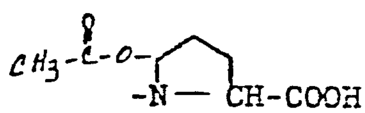 |
| 72 | pyruvoyl-3,4-dihydroxy-L-azetidine-2-carboxylic acid | 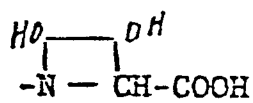 |
| 73 | pyruvoyl-3-hydroxy-L-azetidine-2-carboxylic acid | 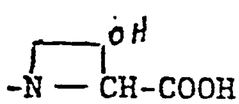 |
| 74 | pyruvoyl-4-hydroxy-L-azetidine-2-carboxylic acid | 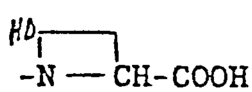 |
| 75 | pyruvoyl-3-methoxy-L-azetidine-2-carboxylic acid | 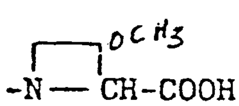 |
| 76 | pyruvoyl-4-methoxy-L-azetidine-2-carboxylic acid | 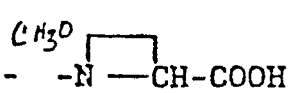 |

| Ex. No. | A | B |
|---|---|---|
| 77 - | pyruvoyl-3,4-dimethoxy-L-azetidine-2-carboxylic acid | 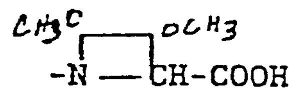 |
| 78 - | pyruvoyl-3-chloro-L-azetidine-2-carboxylic acid | 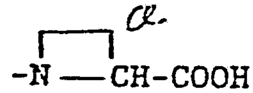 |
| 79 - | pyruvoyl-3-chloro-4-fluoro-L-azetidine-2- carboxylic acid | 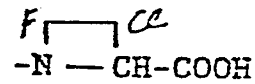 |
| 80 - | pyruvoyl-3,4-difluoro-L-azetidine carboxylic acid | 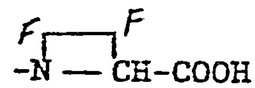 |
| 81 - | pyruvoyl-3-bromo-4-chloro-L-azetidine-2- carboxylic acid | 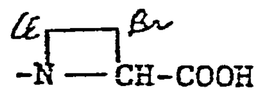 |
| 82 - | pyruvoyl-3-hydroxy-4-iodo-L-azetidine carboxylic acid | 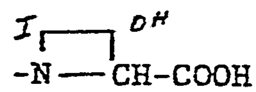 |
| 83 - | pyruvoyl-3-iodo-4-bromo-L-azetidine-2- carboxylic acid | 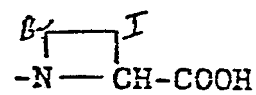 |
| 84 - | pyruvoyl-3-chloro-4-iodo-L-azetidine-2- carboxylic acid | 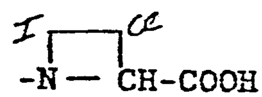 |
| 85 - | pyruvoyl-3,4 difluoro-Pip | 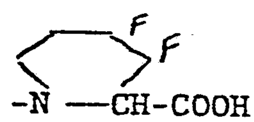 |
| 86 - | pyruvoyl-4,5 dichloro-Pip | 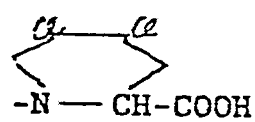 |
| 87 - | pyruvoyl-5,6 diiodo-Pip | 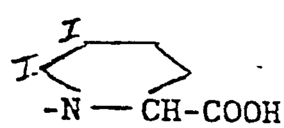 |
| 88 - | pyruvoyl-3,5 dibromo-Pip | 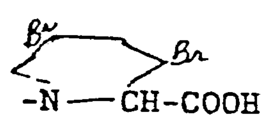 |
| 89 - | pyruvoyl-4-chloro-6-fluoro-Pip | 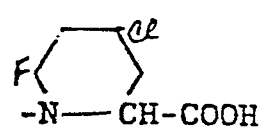 |

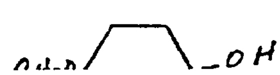

| Ex. No. | A | B |
|---|---|---|
| 91 - | Pyruvoyl-5-fluoro-6-methoxy-Pip | 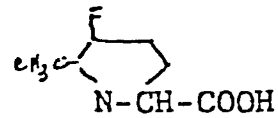 |
| 92 - | pyruvoyl-4-phenoxy-Pip | 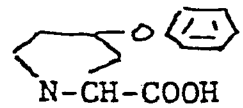 |
| 93 - | pyruvoyl-4,5-dihydroxy-Pro | 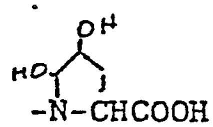 |
| 94 - | pyruvoyl-3,5-dihydroxy-Pro | 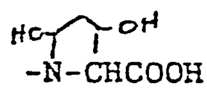 |
| 95 - | pyruvoyl-3,4-dimethoxy-Pro | 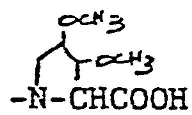 |
| 96 - | pyruvoyl-3-iodo-5-methoxy-Pro | 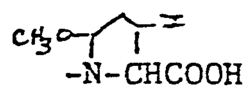 |
| 97 - | pyruvoyl-3-methoxy-5-bromo-Pro | 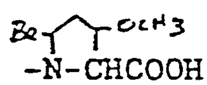 |
| 98 - | pyruvoyl-4-methoxy-5 chloro-Pro | 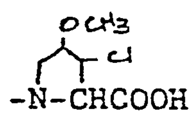 |
| 99 - | pyruvoyl-5-mercapto-Pip | 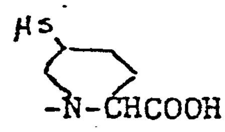 |
| 100 - | pyruvoyl-6-mercapto-Pip | 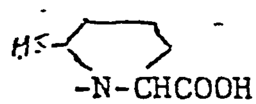 |
| 101 - | pyruvoyl-4-mercapto-Pip | 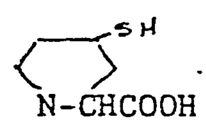 |
| 102 - | pyruvoyl-3-mercapto-Pip | 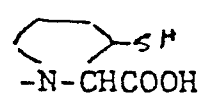 |

## Examples 103-196

Examples 9-102 are each repeated using each of the phenylpyruvoyl analogs of each of the pyruvoyl compounds used in Examples 9-102. In each instance a compound is obtained wherein 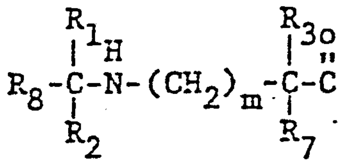 corresponds to

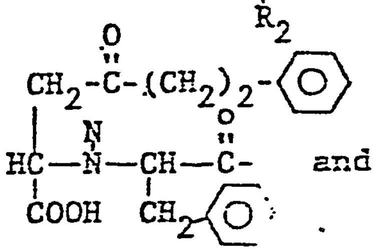 and the 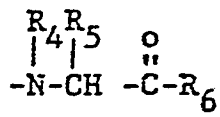

moiety is the B moiety of the example being repeated.

## Examples 193-290

Examples 9-102 are again each repeated using each of the 2-keto-4 phenylbutyryl analogs of the pyruvoyl compounds of Examples 9-102. In each instance a compound is obtained

wherein 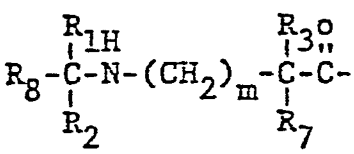 corresponds to

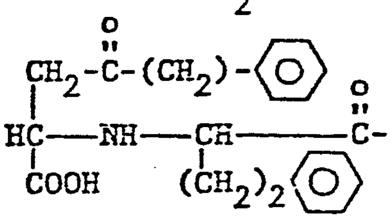 and the 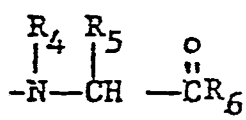

moiety is the B moiety of the particular example repeated.

## Examples 291-384

Examples 9-102 are each again repeated, replacing the pyruvoyl compounds of Examples 9-102 with their respective α-keto glutaryl analogs. In each instance the compound obtained

contains the structure 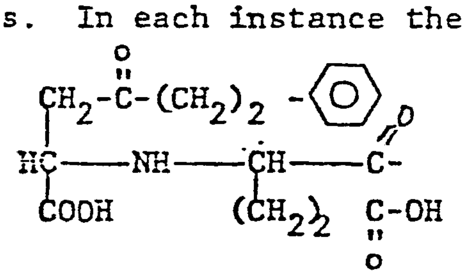

and the $-N-CH_x-\overset{O}{\overset{\|}{C}}-R_6$ moiety corresponding to B of the repeated example.

Example 385

Treatment of any of the intermediate products from Step B of Examples 9-384 according to Example 2 produces the 1- methyl derivatives of each of the final products of Examples 9-384 respectively.

Examples 386-410

Example 1 is repeated a number of times, substituting for hydrocinnamic acid each of the acids from Column A below, to yield a product of the general formula

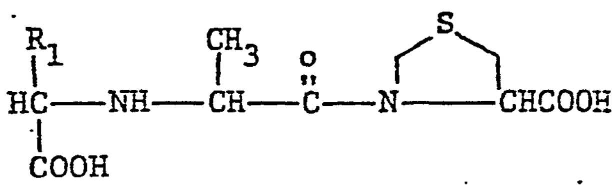

wherein $R_1$ is in each case as shown in Column B.

| Ex. | Column A | Column B($R_1$) |
|---|---|---|
| 386 | benzoic acid | 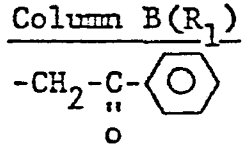 |
| 387 | p-hydroxybenzoic acid | 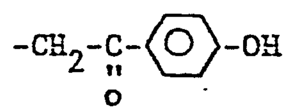 |
| 388 | p-methoxybenzoic acid | 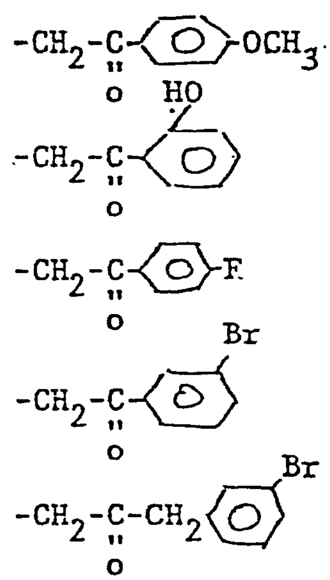 |
| 389 | o-hydroxybenzoic acid | |
| 390 | p-fluorobenzoic acid | |
| 391 | m-bromobenzoic acid | |
| 392 | m-bromophenylacetic acid | |
| 393 | 2-phenylpropanoic acid | 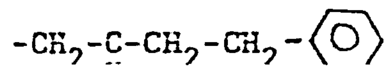 |

| Ex. | Column A | Column B |
|---|---|---|
| 394 | acetic acid | $-CH_2-\underset{\underset{O}{\|}}{C}-CH_3$ |
| 395 | propanoic acid | $-CH_2-\underset{\underset{O}{\|}}{C}\ CH_2\ CH_3$ |
| 396 | butyric acid | $-CH_2-\underset{\underset{O}{\|}}{C}-(CH_2)_2\ CH_3$ |
| 397 | isobutyric acid | $-CH_2-\underset{\underset{O}{\|}}{C}-CH-(CH_3)_2$ |
| 398 | pentanoic acid | $-CH_2-\underset{\underset{O}{\|}}{C}-(CH_2)_3\ CH_3$ |
| 399 | isopentanoic acid | $-CH_2-\underset{\underset{O}{\|}}{C}-CH_2\ CH-(CH_3)_2$ |
| 400 | 2 aminoacetic acid | $-CH_2-\underset{\underset{O}{\|}}{C}-CH_2NH_2$ |
| 401 | 3 aminopropanoic acid | $-CH_2-\underset{\underset{O}{\|}}{C}-CH_2\ CH_2\ NH_2$ |
| 402 | 3-hydroxypropanoic acid | $-CH_2-\underset{\underset{O}{\|}}{C}-CH_2CH_2OH$ |
| 403 | cyclopentanecarboxylic acid | $-CH_2-\underset{\underset{O}{\|}}{C}-$ 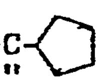 |
| 404 | cyclohexanecarboxylic acid | $-CH_2-\underset{\underset{O}{\|}}{C}-$ 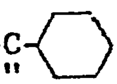 |
| 405 | 3,3-dimethylaminopropanoic acid | $-CH_2-\underset{\underset{O}{\|}}{C}-CH_2CH-N\genfrac{}{}{0pt}{}{CH_3}{CH_3}$ |
| 406 | 3-methylthiopropanoic acid | $-CH_2-\underset{\underset{O}{\|}}{C}-CH_2-CH_2SCH_3$ |
| 407 | 3-ethylthiopropanoic acid | $-CH_2-\underset{\underset{O}{\|}}{C}-(CH_2)_2-S-CH_2CH_3$ |

| Ex. | Column A | Column B |
|---|---|---|
| 408 | phenoxyacetic acid | 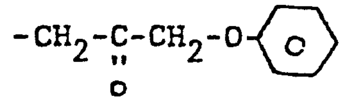 |
| 409 | benzyloxyacetic acid | 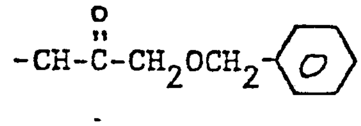 |
| 410 | phenylaminoacetic acid | 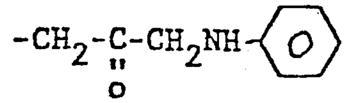 |

Any α-amino group-containing diamino acid may be used in Step B if an appropriate protecting group-e.g., Boc or Cbo is first reacted therewith. In such a case, the amino group formed in Step B of the procedure must be reprotected prior to reaction with

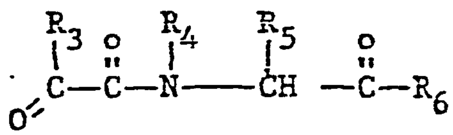

as in Step C.

Procedures for deprotection of $NH_2$ groups, well known in the art, can be used after Step C.

In addition, any of the pyruvoyl, phenypyruvoyl, α-keto-4-phenylbutyryl or glutaryl compounds of Examples 3-5 and 9-385 may be substituted for pyruvoyl-thiazolidine-2-carboxylic acid in each of Examples 386-410.

## Example 411

Synthesis of N-(1-carboxy-2-benzylthio-2,2-dimethyl-ethyl)-α-methyl-Ala-L-Pro-amide

3.2 g. of powdered molecular sieves are added to a solution of 5 mmoles of 3-benzylthio-3,3-dimethylpyruvic acid and 1 mmole α-methyl-alanyl-L-proline amide in 10 ml of ethanol and 5 mmoles of $NaHCO_3$ in water. The misture is stirred for 30 minutes, and then 130 mg. of sodium cyanoborohydride in 2 ml of ethanol is added dropwise with stirring over 4 hours.

The mixture is stirred overnight. The molecular

sieves are removed by filtration. Solvent is removed under vacuum and the product is dried over NaOH and $P_2O_5$ pellets in a vacuum desiccator. The named compound is obtained in pure form by conventional chromatography.

Example 412

Synthesis of N-(L-1-methoxycarbonyl-2-p-benzyloxphenethvl)-Ala-L-Pro-methyl ester

The HCl salt of O-benzyl-L-tyrosine methyl ester (2 mmols) in 7 ml of absolute ethanol plus 0.25 ml of water was mixed with 2 mmols of $NaHCO_3$. To this solution was added 10 mmols of N-pyruvoyl-L-proline methyl ester plus 3.2 g. of powdered molecular sieves. The mixture was stirred for 30 minutes and then sodium cyanoborohydride, 130 mg. in 2.5 ml of absolute ethanol, was added dropwise, with stirring, over a period of 4 hours at room temperature. The mixture was stirred overnight. The molecular sieves were removed by filtration and the precipitate was washed with a small amount of ethanol. The solvent from the combined filtrates was removed under reduced pressure to yield an oil. Part (0.38 g.) of the crude product was purified by partition chromatography (1.2 x 98 cm. column) with Sephadex G-25 equilibrated butanol/acetic acid/$H_2O$(4:1:5 by volume). The product (277 mg.) was eluted with upper phase. The recovered material was further purified on Sephadex LH-20 (2.2 x 100 cm. column), equilibrated and developed with tetrahydrofuran/isopropanol (3:7 by vol.).

Example 413

Synthesis of N-(L-1-carboxy-2-p-benzyloxyphenethyl)-Ala-L-Pro

The product of Example 412, 330 mg., was saponified in 1.5 ml. of 1 M KOH in methanol at room temperature for 1 hour.

The crude product was purified by partition chromatography as described in Example 412 (Sephadex G-25 step). The partially purified product was obtained in apparently pure form by chromatography on Dowex 50W-X8 (1.2 x 48 cm. column) eluted with 2% pyridine.

## Example 414

Synthesis of N-(L-1-carboxy-2-p-hydroxyphenylethyl)-Ala-L-Pro

The product of Example 413, 10 mg. in 1.0 ml. methanol, was hydrogenated for 5 hours at room temperature with hydrogen gas at 10 psi and with 10 mg. of 10% palladium on carbon as catalyst. The catalyst was removed by filtration, and solvent was removed with a rotary evaporator. The material was further purified by chromatography on Sephadex G-10 (1.2 x 99 cm.), equilibrated and developed with 2% pyridine. The desired product was obtained in a yield of 5.8 mg.

## Example 415

Synthesis of N-[L-1-carboxy-2-(p-hydroxy-3-iodo-phenyl)-ethyl]-Ala-L-Pro

To a 12 x 75 mm. polypropylene tube was added 2.2 mmols of Iodogen (Pierce Chemical Co.) in a 1.0 ml of dichloromethane. As the tube was rotated, the solvent was removed with a stream of dry nitrogen. The product of Example 414, 1.02 mg., and sodium iodide, 37 micrograms, were dissolved in 1 ml. of 0.05 M sodium phosphate buffer, pH 7.4, and the solution was transferred to the polypropylene tube. The tube and its contents were left in an ice bath for 10 minutes with occasional mixing. The solution containing the desired product was then transferred to a 12 x 75 mm. glass culture tube.

Alternatively, the named compound can be obtained by substituting 3-iodo-L-tyrosine methyl ester for O-benzyl-L-tyrosine methyl ester in the procedure of Example 412. The dimethyl esters of the resulting product are removed by saponification in 1 M KOH in methanol at room temperature for 1 hour. The named compound is obtained by chromatography over a column of Sephadex G-10 equilibrated and developed with 2% pyridine. The dry powder is obtained by lyophilization.

Example 416

Synthesis of N-(1-carboxy-2-hydroxy-2-phenylethyl)-Tyr-4-hydroxy-L-Pro-t-butyl ester

3.2 g. of powdered molecular sieves are added to a solution of 2 mmols of -phenylxevina and 10 mmols of N-(2-oxo-3-p-hydroxyphenylpyruvoyl)-L-4-hydroxy-Pro-t-butyl ester in 7 ml. of absolute ethanol with 2 mmols $NaHCO_3$ water. The mixture is stirred for 0.5 hours. Sodium cyanoborohydride, 130 mg. in 2 ml. of absolute ethanol, is added dropwise with stirring over 8 hours. The mixture is stirred overnight. The molecular sieves are removed by filtration and are washed with ethanol. The solvent is removed under reduced pressure. The residue is triturated with diethyl ether at 0°C., and the product is collected by filtration. The product is ~~dired~~ dried over NaOH and $P_2O_5$ in a vacuum desiccator. The named compound is obtained by conventional chromatography.

Example 417

Synthesis of N-(1-carboxy-2-hydroxy-2-phenethyl)-Tyr-L-Pro

The product of Example 416 is dissolved in 3 ml. of anhydrous trifluoroacetic acid. After 30 minutes at room temperature, the solvent is removed to yield the named compound

Example 418

Synthesis of N-(1-ethoxycarbonyl-2-hydroxy-2-phenylethyl)-Tyr-L-Pro-t-butyl ester

By substituting the ethyl ester of β phenylserine for β phenylserine in Example 416. the named compound is obtained.

Example 419

Synthesis of N-(1-ethoxycarbonyl-2-hydroxy-2-phenethyl)-Tyr-L-Pro

The product of Example 418 is dissolved in anhydrous trifluoroacetic acid at room temperature. After 30 minutes the trifluoroacetic acid is removed with a rotary evaporator under high vacuum to yield the named compound.

Example 420

Synthesis of N-(1-carboxy-2-hydroxy-2-phenylethyl)-Tyr-L-Pro-t-butyl ester

The product of Example 418 is dissolved in 3 ml. of 0.5 M NaOH in ethanol. After 1 hour at room temperature, the solution is neutralized, and the named compound is recovered by conventional chromatography.

Example 421

Synthesis of N-(L-1-ethoxycarbonyl-2-hydroxybutyl)-Trp-α-methyl-L-Pro-t-butyl ester

β-hydroxy-L-norvaline-α-ethyl ester, 2 mmols, is dissolved in a solution of 10 mmols of N-(3-indole-pyruvoyl)-α-methyl-L-proline-t-butyl ester in 10 ml. of absolute ethanol. Powdered molecular sieves 3.2 g., are added with stirring. After 30 minutes, 130 mg. of sodium cyanoborohydride in 2.5 ml. of absolute ethanol is added dropwise over a period of 4 hours. The mixture is stirred at room temperature overnight. The

filtrate is removed by evaporation under high vacuum. The desired compound is obtained by conventional chromatography.

Example 422

Synthesis of N-(L-1-ethoxycarbonyl-2-hydroxybutyl)-Trp-α-methyl-L-Pro

The product of Example 421 is treated with anhydrous HF in the presence of anisole to yield the named compound.

Example 423

Synthesis of N-(L-1-carboxy-2-hydroxybutyl)-Trp-α-methyl-L-Pro

The product of Example 422 is saponified in 1 M KOH in methanol under argon for 1 hour to yield the named compound.

Example 424

Synthesis of N-(1-carboxy-2-mercapto-2,2-dimethylethyl)-α-methyl-Ala-L-Pro-amide

The product N(1-carboxy-2-benzylthio-2,2-dimethylethyl)-α-methyl Ala-L-Pro-amide obtained in Example 411 is treated with anhydrous HF in the presence of anisole to yield the named compound.

Example 425

Synthesis of N-(1-methyl-1-phenylthiocarbonyl-m-nitro-p-hydroxy-phenylethyl)-N -Boc-Lys-L-2,3-Δ-Pro-t-butyl ester

By substituting N-(N -Boc-amino-α-oxohexanoyl)-2,3-dehydro-L-proline-t-butyl ester and 1-methyl-3-nitro-L-tyrosine-α-thiopenyl ester as reactants in the procedure of Example 416, the named compound is obtained.

Example 426

Synthesis of N-(1-methyl-1-1-thiocarbonyl-m-nitro-p-hydroxyphenyl-ethyl)-N -Boc-Lys-2,3-Δ-L-Pro-t-butyl ester

The product of Example 425 is treated with NaSH to yield

Example 427

Synthesis of N-(1-methyl-1-thiocarboxy-m-nitro-p-hydroxy-phenylethyl)-Lys-L-Pro

The product of Example 426 is issolved in anhydrous trifluoroacetic acid in the presence of anisole and allowed to stand for 30 minutes, to yield the named compound.

Example 428

Synthesis of N-(1-methyl-1-phenylthiocarbonyl-m-nitro-p-hydroxyphenylethyl)-Lys-2,3-dehydro-L-proline

The product of Example 425 is treated with trifluoroacetic acid as in Example 427 to yield the desired compound.

Example 429

Synthesis of N-(1-carboxy-1-methyl-m-nitro-p-hydroxyphenylehtyl)-Glu-(γ-t-butyl ester)-3-hydroxy-L-proline-t-butyl-ester

Through the procedure of Example 416, α-methyl-m-nitro-L-tyrosine is reacted with N-(5-Boc-amino-2-oxoglutaryl)-3-hydroxy-L-proline diphenyl methyl ester to yield the named compound.

Example 430

Synthesis of N-(1-carboxy-1-methyl-m-amino-p-hydroxyphenylethyl-Glu(γ-t-butyl ester)-3-hydroxy-L-proline-t-butyl ester

The product of Example 429 in ethanol is hydrogenated, using 10% palladium on charcoal as catalyst, in hydrogen at 30 psi for 3 hours. The catalyst is removed by filtration, and solvent is removed under reduced pressure to yield the named compound.

Example 431

Synthesis of N-(1-carboxy-1-methyl-m-amino-p-hydroxy-phenylethyl)-Glu-3-hydroxy-L-Pro.

The product of Example 430 is dissolved in anhydrous trifluoroacetic acid. After 30 minutes at room temperature, solvent is stripped to yield the named compound.

Example 432

Synthesis of N-(1-carboxy-1-methyl-m-nitro-p-hydroxyphenylethyl)-Glu-3-hydroxy-L-Pro

The product of Example 429 is dissolved in anhydrous trifluoroacetic acid. After 30 minutes at room temperature, trifluoroacetic acid is removed under high vacuum to yield the desired product.

Example 433

Synthesis of N-(1-carboxy-2,2,2-trimethylethyl )-(O-benzyl)-Tyr-2-fluoro-L-Pro ethyl ester

By reacting L-tri-leucine with benzyloxyphenylpyruvoyl-2-fluoro-L-Pro ethyl ester according to the procedure of Example 416, the desired compound is obtained.

Example 434

Synthesis of N-(1-carboxy-2,2,2-trimethylethyl)-Tyr-2-fluoro-L-Pro ethyl ester

The product of Example 433 is hydrogenated with 10% palladium on charcoal as catalyst and with hydrogen at 15 psi for 3 hours. The catalyst is removed by filtration to yield the named compound.

Example 435

Synthesis of N-(1-carboxy-2,2,2-trimethylethyl)-O-benzyl-Tyr-2-fluoro-L-Pro

The product of Example 433 is saponified to yield the desired compound.

Example 436

Synthesis of N-(1-carboxy-2,2,2-trimethylethyl)-Tyr-2-fluoro-L-Pro

The product of Example 434 is saponified to yield the named compound.

Example 437

Synthesis of N-(1-ethoxycarbonyl-3-phenylpropyl)-DL-Ala-pyroglutamic acid

A. L-pyroglutamic acid (35 mmols) is suspended in a mixture of 35 ml. of propylene oxide and 210 ml. of dry acetonitrile at room temperature for 15 minutes. Pyruvic acid chloride (36.8 mmols) is added and the reaction mixture is stirred for about 12 hours. The mixture is then chilled in an ice bath and slowly treated with 35 ml. of 1 N HCl and stirred for 5 minutes. Acetonitrile is then removed in vacuo and the resulting residue is dissolved in 500 ml. of ethyl acetate. The organic phase is washed several times with 50 ml. portions of water and then with saturated NaCl solution. The product is dried over anhydrous $MgSO_4$ and then any residual solvent is removed in a rotary evaporator. The resulting product is N-pyruvoyl-L-<Glu.

B. A solution of L-homophenylalanine ethyl ester (10 mmols), N-pyruvoyl-L-pyroglutamic acid (50 mmols) in a mixture of 20 ml. of water and 50 ml. of p-dioxane is prepared. The pH is adjusted to 6.5 with NaOH and 1.9 g. of sodium cyanoborohydride is added. The mixture is stirred at room temperature for several days.

This solution is then charged to a Dowex 50W - 8ft. column prepared with 50% p-dioxane and water. The material is washed with 50% p-dioxane-water and then with water, and eluted with 2% pyridine in water. The product fractions are combined and concentrated to dryness in vacuo to yield the named product.

Example 438

Synthesis of N-(2-keto-4-benzylmercaptobutanoyl)-5-thioxo-L-proline diphenyl-methyl ester

To a solution of 1.6 mols mmols of 5-thio-L-proline diphenylmethyl ester in 10 ml. of methylene chloride cooled in an ice bath under a blanket of nitrogen is added 1.56 mmols of triethylamine acid followed by a solution of 1.62 mmols of 4-benzylmercaptobutanoyl chloride in 12 ml. of methylene chloride over a ten minute period. The reaction mixture is stirred at 4°C overnight. The reaction mixture is then mixed with 100 ml. of ethyl acetate and washed with 20 ml. of 10% potassium sulfate twice and then followed by water, saturated sodium bicarbonate and saturated NaCl. The organic phase is then dried over anhydrous $MgSO_4$ and filtered. The solvent is removed with a rotary evaporator to yield the named product.

Example 439

Synthesis of N-(1-butoxycarbonyl-3-phenylpropyl)-D,L-S-benzyl-homocysteinyl-5-thio-L-proline diphenyl methyl ester

L-homophenylalanine-t-butyl ester (5 mmols) and N-( -keto-4 -benzyl-mercaptobutanoyl)-5-thio-L-proline diphenyl methyl ester are dissolved at room temperature under nitrogen in 20 ml. of ethanol with vigorous stirring. Thereupon 8.0 g. of molecular sieves are added and the mixture is stirred at room temperature for 30 minutes, followed by the addition of 0.325 g. of sodium cyanoborohydride in 7.5 ml. of ethanol over a period of 4 hours. The reaction mixture is further stirred at room temperature overnight. The molecular sieves are then removed by filtration and the solvent is removed in a rotary evaporator. The product is then purified with Sephadex LH-20 on a column

and eluted with tetrahydrofuran/isopropanol (3:7) to yield the named product.

Example 440

Synthesis of N-(L-1-carboxy-3-phenylpropyl)-D,L-benzyl-homocysteinyl-5-thio-L-proline

A solution of 1.0 mmols of the product of Example 439 and 1.5 ml. of anisole in 10 ml. of methylene chloride is cooled in an ice bath under nitrogen and treated with 5 ml. of trifluoroacetic acid. The reaction mixture is kept at ice bath temperature for 1 1/2 hours. It is then concentrated in vacuo and the crude product is dried in a vacuum desiccator over $P_2O_5$ and KOH overnight, whereupon the desired product is obtained.

Example 441

Synthesis of N-(L-1-carboxy-3-phenylpropyl)-D,L-homocysteinyl-5-thio-L-Pro

The product of Example 440 (0.5 mmols) in 1 ml. of anisole and 5 ml. of anhydrous HF is stirred at 0°C for one hour. The HF and anisole are removed under vacuum, and the product is dried in a vacuum desiccator in the presence of KOH and $P_2O_5$. The named product is recovered.

Example 442

Synthesis of N-(1-carboxy-3-phenylpropyl)-D,L-Ala-5-oxo-L-Pro

The synthesis of Example 437 is repeated using L-homophenylalanine t-butyl ester instead of L-homophenylalanine ethyl ester. The product is N-(L-1-t-butyloxycarbonyl-3-phenylpropyl)-D,L-Ala-pyroglutamic acid. This product is then subjected to the action of trifluoroacetic acid at room temperature to convert it to the named compound.

Example 443

Synthesis of N-(3-(3-indolyl)-2-oxobutyroyl)-L-4-benzyloxy-L-Pro-t-butyl ester

A solution of 4-(3-indolyl)-2-oxobutyric acid (10 mmols) in 20 ml. of tetrahydrofuran is cooled to -25°C. 10 mmols of N-ethyl morpholine in 2 ml. of methylene chloride is then added and this is followed by the addition of 10 mmols of isobutyl chloroformate in 3 ml. of methylene chloride. The mixture is stirred at 20°C for 15 minutes. A saturated solution of 10 mmols of L-4-benzyloxy-L-proline t-butyl ester in 5 ml. of methylene chloride is added and the reaction mixture is stirred at -15°C for 2 hours and then at room temperature for another 2 hours. The reaction mixture is then taken up in 50 ml. of ethyl acetate and washed until neutral. The organic phase is dried over anhydrous $MgSO_4$ and filtered. The solvent is removed with a rotary evaporator to yield the named compound.

Example 444

Synthesis of N-(L-1-ethoxycarbonyl-3-phenylpropyl)-D,L-homo-tryptophanyl-L-4-benzyloxyproline-t-butyl ester

The procedure of Example 439 is repeated, substituting for L-homophenylalanine-t-butyl ester the compound L-homophenyl-alanyl ethyl ester and substituting for N-(2-keto-4-benzyl-mercaptobutyl)-5-thioproline diphenyl methyl ester the compound N-4-(3-indolyl)-2-oxobutyroyl-L-4-benzyloxy-L-proline-t-butyl ester, to obtain the named compound.

Example 445

Synthesis of N-(L-1-ethoxycarbonyl-3-phenylpropyl)-D,L-homotryptophanyl-L-4-benzyloxyproline

N-(L-1-ethoxycarbonyl-3-phenylpropyl)-D,L-homo-trypto-phanyl-L-4-benzyloxyproline-t-butylester (100 mg.) and 1 ml.

mercaptoethanol are treated with 1 ml. of trifluoroacetic acid for 30 minutes. The reaction mixture is then triturated with anhydrous ether, filtered and dried in a vacuum desiccator over $P_2O_5$ and KOH to yield the desired compound.

Example 446

Synthesis of N-(L-1-ethoxycarbonyl-3-phenylpropyl)-D,L-homotryptophanyl-L-4-hydroxyproline

The product from Example 445 (50 mg.) is subjected to hydrogenolysis with 10 mg. of 10% Pd on charcoal in 2 ml. of methanol at atmospheric pressure for 2 hours. The named compound is recovered.

Example 447

Synthesis of N-(1-ethoxycarbonyl-3-phenyl-propyl-α-fluoromethyl)-alanyl-L-proline-phenylthio-ester

A. A solution of Boc-α-fluoromethyl-alanine (50 mmols) in 50 ml. of methylene chloride is cooled to -5°C. A solution of DCC (50 mmols) in 5 ml. of methylene chloride is added and the mixture is stirred at -5°C for 5 minutes. There is then added 50 mmols of HCl proline phenylthioester which is neutralized with 50 mmols of N-ethyl morpholine in 20 ml. of methylene chloride. The reaction mixture is stirred at -5°C for 1 hour and then at 4°C overnight. DCC is removed by filtration and the solvent is removed with a rotary evaporator. Ethyl acetate is added to the residue, which is washed until neutral. Residual solvent is again removed with a rotary evaporator and the named product is recovered.

B. The Boc protecting group of the compound of Step A is removed with HCl in ethyl acetate at room temperature for 15 minutes. The resulting hydrochloride salt is recovered by filtration and dried over $P_2O_5$ and KOH in a vacuum desiccator.

A solution of this salt (10 mmols) and sodium bicarbonate (10 mmols) in 30 ml. of ethanol and 5 ml. of water is added to a solution of 10 mmols of 4-phenyl 2-oxo-butyric acid ehtyl ester in 5 ml. of ethanol. Molecular sieves (12.06 g.) are added and the mixture is stirred at room temperature for 30 minutes. A solution of 0.6 g. of sodium cyanoborohydride in 15 ml. of ethanol is added dropwise over a period of 4 hours and then the reaction mixture is stirred at room temperature overnight. The mixture is worked up as described in Example 439 to yield the named compound.

Example 448

Synthesis of N-(1-ethoxycarbonyl-3-phenyl-propyl)-2-fluoromethyl alanyl-L-proline thio acid

A solution of 56.1 mg. (1 mmole) of NaSH in 0.5 ml. of ethanol is added dropwise under a nitrogen blanket to a solution of N-(1-ethoxy-carbonyl-3-phenylpropyl)-2-fluoromethyl-alanyl-2-proline phenyl thioester (1 mmole) from Example 447, dissolved in 5 ml. of ethanol. The reaction mixture is stirred at room temperature for 1 hour. The solvent is then removed with a rotary evaporator and ethyl acetate (50 ml.) plus water (5 ml.) are added to the residue. The mixture is then cooled in an ice bath, and acidified with 2N-sulfuric acid to about pH2. The organic phase is then separated, washed twice with saturated NaCl, dried over anhydrous Mg $SO_4$ and filtered. Residual solvent is removed with a rotary evaporator to yield the named compound.

Example 449

Synthesis of N-[(1-ethoxycarbonyl-1-phenylmethyl)-2-phenyethyl]-serinyl-L-proline-t-butyl ester

In the manner described in Example 439, 2-phenylmethyl-phenylalanine ethyl ester and 2-hydroxypyruvoyl-L-proline-t-

butyl ester are reacted and the reaction mixture is worked up to yield the named compound.

Example 450

Synthesis of N-[(1-ethoxycarbonyl-1-phenylmethyl)-2-phenylethyl]-serinyl-L-proline

The product from Example 449 is treated with trifluoroacetic acid in the manner described in Example 441 to give the desired product.

Example 451

Synthesis of N-[(1-carboxy-1-phenylmethyl)-2-phenylethyl]-serinyl-L-proline

The product from Example 450 is subjected to alkaline hydrolysis with 2 equivalents of KOH in 2 ml of water: dioxane (1:1) for 1 hour to produce the named compound.

Example 452

Synthesis of N-[1-phenylthio-carbonyl-1-(methoxycarbonyl-methyl)-S-methyl-3-mercaptopropyl]-phenylalanyl-t-butyl ester

Using S-methyl-2-(methoxy-carbonyl-methyl)-homocysteine thiophenyl ester and N-(3-phenyl-pyruvoyl)-L-proline-t-butyl ester and following the procedure of Example 439, the named compound is prepared.

Example 453

Synthesis of N-[1-phenylthiocarbonyl-1-(methoxycarbonyl-methyl)-S-methyl-3-mercaptopropyl]-phenylalanyl-L-Pro

The product of Example 452 (1 g.) in 2 ml. of anisole is treated with 5 ml. of trifluoroacetic acid under nitrogen for 20 minutes at room temperature. The product is precipitated with diethylether while cooling in an ice bath. The named compound is recovered after drying in a vacuum desiccator over $P_2O_5$ and KOH.

Example 454

Synthesis of N[1-thiocarbonyl-1-(methoxycarbonylmethyl)-S-methyl-3 mercaptopropyl]-phenylalanyl-L-Pro

The product of Example 453 is subjected to hydrolysis with two equivalents of sodium hydrosulfide as described in Example 448 to yield the named compound.

Example 455

Synthesis of N-(1-ethoxycarbonyl-1-fluoro-methyl- -p-hydroxyphenyl)ethyl]-N-Cbo-ornithinyl-L-Pro-t-butyl ester

A. A solution of L-Pro-t-butyl ester (0.1 mols) in 30 ml. of methylene chloride is cooled in a dry ice-acetone bath to -50°C. A small portion thereof is added with vigorous stirring to a solution of DCC (0.1 mol) in 15 ml. of methylene chloride while the temperature is maintained at -55°C. The solution is further stirred at -55°C for 3 minutes after which there is added, dropwise, a solution of freshly vacuum distilled 5_Cbo-amino-2-oxopentanoic acid. (0.1 mol) in 20 mls. methylene chloride. The reaction mixture is stirred at -55°C for 4 hours, at -15°C for 2 hours and finally at 4°C overnight. Solid Dicylcohexylurea is removed by filtration, and isopropyl ether (50 ml.) is added. The organic phase separated thereby is washed until neutral, dried, filtered and concentrated in a rotary evaporator. Upon storage in a freezer the resultant compound crystallizes out.

B. This compound is prepared according to the method described in Example 439, using β-(fluoromethyl)-tyrosine ethyl ester and N-(5-Cbo-amino)-2-oxo-pentanoyl-1-proline-t-butyl ester as primary reactants.

Example 456

Synthesis of N-[1-ethoxycarbonyl-1-fluoromethyl-2-(-p-hydroxyphenyl)ethyl]-ornithinyl-L-proline

The product of Example 455 is subjected to HF to deprotect the carboxyl group of proline and yield the named product.

Example 457

Synthesis of N-(1-ethoxycarbonyl-1-(methoxy-methyl)-2-(3,4-dimethoxyphenylethyl]-S-benzyl-cysteinyl-L-Pro-t-butyl ester

Using the method described in Example 439, α-(methoxy-methyl)-3,4-dimethoxyphenylalanine ethyl ester and N-(3-benzyl thiopyruvoyl-2-Pro-t-butyl ester, the named compound is prepared.

Example 458

Synthesis of N-[1-ethoxycarbonyl-1-(methoxymethyl)-2-(3,4-dimethoxyphenyl)-ethyl]-cysteinyl-L-Pro

The product of Example 457 is subjected to HF treatment to deprotect the carboxyl group of proline and yield the named compound.

Example 459

Synthesis of N-(1-ethoxycarbonyl-1(2-propenyl)-5-phthalylpentanoyl]-valyl-L-Pro

A. Proline (35 mmols) is suspended in a mixture of 35 ml. of propylene oxide and 210 ml. of dry acetonitrile at room temperature. Bis-trimethyl-silyl-trifluoroacetamide (77 mmols) is added and the reaction is stirred at room temperature for 15 minutes. 3-methyl-2-oxo-butanoic acid chloride (36.8 mmols) is added and the mixture is stirred for about twelve hours. The mixture is chilled in an ice bath, slowly

treated with 35 ml. of 1 N HCl and stirred for 5 minutes. Acetonitrile is then removed in vacuo and the solid residue is dissolved in 500 ml. of ethyl acetate. The organic layer separated is washed several times with 50 ml. portions of water and then with saturated NaCl, dried over anhydrous $MgSO_4$. Residual solvent is removed with a rotary evaporator and N-(3-methyl-2-oxo-butanoyl)-L-Pro is recovered.

B. Following the method of Example 447, the product of Step A is reacted with 2-amino-1-(2-propenyl)-5-phthalylpentanoic acid ethyl ester to give the named compound.

Example 460

Synthesis of N-[(1-carboxy-1-(2 propenyl)-5-phthalylpentanoyl]-valyl-L-Pro

The product of Example 459 is saponified to prepare the named compound.

Example 461

Synthesis of N-[(carboxy-1-(2-propenyl)-5-aminopropanoyl]-valyl-L-Pro.

The named compound is obtained by hydrazinolysis with hydrazine hydrate of the product of Example 460.

Example 462

Synthesis of N-(1-isobutyl-1-carboxy-3-methoxycarbonylpropyl)-leucinyl-L-Pro

A. N-(4-methyl-2-oxo-pentanoyl)-L-Pro is prepared as described in Step A of Example 459, substituting 4-methyl-2-oxo-pentanoic acid chloride for 3-methyl-2-oxo-butanoic acid chloride.

B. The product of Step A (0.00272 mols) is reacted with -isobutyl glutamic acid -methyl ester (0.000545 mols) and

sodium cyanoborohydride (103 mg.) in water (10 ml.) at pH7 (adjusted with caustic) at room temperature over several days. The product is adsorbed on an acid ion exchange resin and eluted with 2% pyridine in water, fractionally distilled and freeze dried to produce the named compound.

Example 463

Synthesis of N-(1-carboxy-1-methyl-4-phthalyl butyl)-Tyr-L-Pro-t-butyl ester

A. N-(4-hydroxyphenyl)-pyruvoyl-L-proline-t-butyl ester is prepared using the method of Step A of Example 455 and substituting 4-hydroxyphenyl pyruvic acid for the carboxylic acid therein used.

B. Following the method of Example 437, Step B, using 2-methyl-5-phthalyl-ornithine and N-(4-hydroxyphenyl)-pyruvoyl-L-proline-t-butyl ester from Step A as reactants, the named product is obtained.

Example 464

Synthesis of N-(1-carboxy-1-methyl-4-amino-butyl)-Tyr-L-Pro-t-butyl ester

The named product is obtained by hydrazinolysis of the product of Example 463.

Example 465

Synthesis of N-(1-carboxy-1-methyl-4-aminobutyl)-Tyr-L Pro

The named compound is obtained by trifluoroacetic acid treatment of the product of Example 464.

Example 466

Synthesis of N-(1-carboxy-1-methyl-4-phthalyl-butyl)-Tyr-L-Pro

The named compound is obtained by trifluoroacetic acid treatment of the product of Example 463.

Example 467

Synthesis of N-[1(-'-mercaptoethyl)-1-mercaptoethyl-4-hydroxy-2-keto-butyl]-Ala-Pro

The '-hydroxyethylketone of S-benzyl-D-homocysteine, 2 mmols, is reacted with 10 mmols of pyruvoyl-L-Pro, all in 10 ml. of ethanol plus 12 mmol $NaHCO_3$ on 2 ml. $H_2O$, in the presence of 3.2 g of powdered molecular sieves at room temperature. After 30 minutes of stirring, 130 mg. of sodium cyanoborohydride in 2 ml. of ethanol is added dropwise over a period of 4 hours. The molecular sieves are removed by filtration. The solvent from the filtrate is removed by rotary evaporation under reduced pressure. The product is isolated by conventional chromatography techniques. The S-benzyl protecting group is removed with anhydrous HF in the presence of anisole to yield the named compound.

Example 468

Synthesis of N-(1-mercaptomethyl-3-amino-2-keto propyl)-Phe-4-fluoro-L-Pro

The '-Boc-aminomethylketone of S-benzyl-D-cysteine, 2 mmols, is reacted with 3-phenylpyruvoyl-4-fluoro-L-Pro essentially as described in Example 467 to yield N-(1-benzoylthiomethyl-4-t-butyloxycarbonylamino butyl)-Phe-L-Pro. If desired, the t-Boc group can be removed with anhydrous trifluoroacetic acid, or the t-Boc and S-benzyl groups may be removed with anhydrous HF in the presence of anisole to yield the named compound.

Example 469

Synthesis of N-(L-carboxymethyl-2-keto-butyl)-homoPhe-3-chloro-L-Pro

The α-ethylketone of D-aspartic acid -β-t-butyl ester, 2 mmols, is reacted with 10 mmols of 2-keto-4-phenylbutanoyl-3-

chloro-L-Pro in the presence of sodium cyanoborohydride according to the procedure of Example 467 to yield N-(1-t-butyloxycarbonylmethyl-2-keto-butyl)-homoPhe-3-chloro-L-Pro. The latter is isolated by conventional techniques of chromatography. The t-butyl ester group is removed with anhydrous HF to yield the named compound.

## Example 470

### Synthesis of N-(1-thiocarboxymethyl-2-keto-butyl)-Val- Glu

The α-ethyketone of D-aspartic acid-β-thiophenyl ester, 2 mmols, is reacted with 3,3-dimethylpyruvoyl-5-keto-L-Pro in the presence of sodium cyanoborohydride, essentially as described in Example 467, to yield, after isolation, N-(1-phenylthiocarboxyl-methyl-2-keto-butyl)-Val-5-keto-L-Pro. By removing the thiophenyl ester with NaSH, the desired compound is obtained.

## Examples 471

Step A. Synthesis of N-(pyruvoyl)-L-Pro-t-butyl ester and related α-keto intermediate compounds.

A solution of 99.5 mmols of L-Pro-t-butyl ester in 39 ml. of chloroform was cooled to -50°C. A solution of dicyclohexylcarbodiimide (DCC), 99.5 mmols in 15 ml. of methylene chloride, at -55°C., was added dropwise with vigorous stirring. Three minutes later, pyruvic acid, 99.5 mmols in 20 ml. of chloroform at -55°C., was added dropwise. The reaction mixture was stirred at -55°C. for 4 hours at -15°C. for 2 hours at 4°C. overnight and at 22°C. for 2 hours. The dicyclohexylurea precipitate was removed by filtration and the precipitate was washed with isopropyl ether. The organic phase was washed until neutral,

under reduced pressure. The crude product was vacuum-distilled over anhydrous potassium carbonate to yield 21.1g. of oily residue. Vacuum distillation was repeated to collect the fraction distilled between 114 and 124°C. ( 1mm.$H_g$). This step yielded 11g of material that crystallized to yield white needles having a measured optical rotation, in the concentration of 0.06029g/ml of ethyl acetate of $[\alpha]_D^{25}$ = -71.95°. Elemental analysis for $C_{11}H_{19}NO_4$ (Formula weight = 241.291):

Calculated: C 59.73, H 7.94, N 5.81,O 26.52;

Found: C 59.64, H.8.10, N 5.76.

By substituting for pyruvic acid an α-keto-carboxylic acid of column A below, for pyruvic acid and/or substituting for L-proline-t-butyl ester an analog listed in column B in the synthesis given above, intermediate compounds shown in column C are obtained.

D.

| Column A | Column B | Column C |
|---|---|---|
| pyruvic acid | L-proline-ethyl ester | N-pyruvoyl-L-Pro ethyl ester |
| " " | L-Pro-benzyl ester | N-pyruvoyl-L-Pro benzyl ester |
| " " | L-Pro-amide | N-pyruvoyl-L-Pro amide |
| " " | L-Pro-thiophenyl ester | N-pyruvoyl-L-Pro-thiophenyl ester |
| " " | 3-methoxy-L-Pro | N-pyruvoyl-3- methoxy-L-Pro |
| " " | 4-Boc-amino-L-Pro-t butyl ester | N-pyruvoyl-Boc-amino-L-Pro-t butyl ester |
| " " | L-azetidine-2-carboxylic acid | N-pyruvoyl-L-Aze-2-carboxylic acid |
| " " | 4-hydroxy-L-Pro-t-butyl ester | N-pyruvoyl-4-hydroxy-L-Pro-t-Butyl ester |
| " " | 2-fluoro-L-Pro-amide | N-pyruvoyl-2-fluoro-L-Pro amide |
| " " | 3-hydroxy-L-Pro-ethyl ester | N-pyruvoyl-3-hydroxy-L-Pro ethyl ester |
| " " | 3,4-difluoro-L-Pro-ethyl ester | N-pyruvoyl-3,4-difluoro-L-Pro-ethyl ester |
| " " | 3-hydroxy-5-methyl-L-Pro-methyl ester | N-pyruvoyl-3-hydroxy-5-methyl-L-Pro methyl ester |
| " " | 4-propyl-L-Pro-t-butyl ester | N-pyruvoyl-4-propyl-L-Pro-t-Butyl ester |
| " " | 5-phenyl-thio-L-Pro-methyl ester | N-pyruvoyl-5-phenyl-thio-L-Pro-methyl ester |
| " " | 5(2-hydroxyphenyl)-thio-L-Pro ethyl ester | N-pyruvoyl-5(2-hydroxyphenyl)-thio-L-Pro-ethyl ester |

| No. | Column A | Column B | Column C |
|---|---|---|---|
| | pyruvic acid | 4-Boc-aminomethyl-L-Pro-t-butyl ester | N-pyruvoyl-4-Boc-aminomethyl-L-Pro-t-butyl ester |
| | " " | 3-methyl-thio-L-Pro-t-butyl ester | N-pyruvoyl-3-methyl thio-L-Pro-t-Butyl ester |
| | " " | B-benzylthio-L-Pro-t-butyl ester | N-pyruvoyl-B-benzylthio-L-Pro-t-Butyl ester |
| | " " | α-methyl-L-Pro-t-butyl ester | N-pyruvoyl-α-methyl-L-Pro-t-Butyl ester |
| | " " | 4-methylene-L-Pro-ethyl ester | N-pyruvoyl-4-methylene-L-Pro ethyl ester |
| | " " | 4-fluoromethyl-L-Pro ethyl ester | N-pyruvoyl-4-fluoromethyl-L-Pro ethyl ester |
| | " " | 4-acetylthio-L-Pro-t-butyl ester | N-pyruvoyl-4-acetylthio-L-Pro-t-Butyl ester |
| | " " | 3-iodo-L-Pro-t-butyl ester | N-pyruvoyl-3-iodo-L-Pro-t-Butyl ester |
| | " " | 4-iodo-L-Pro amide | N-pyruvoyl-4-iodo-L-Pro amide |
| | " " | 3,4-dichloro-L-Pro amide | N-pyruvoyl-3,4 dichloro-L-Pro amide |
| | " " | 4-hydroxymethyl-L-Pro ethyl ester | N-pyruvoyl-4-hydroxymethyl-L-Pro ethyl ester |
| | " " | L-Pip-t-butyl ester | N-pyruvoyl-L-Pip-t-Butyl ester |
| | phenyl pyruvic acid | L-Pro-t-butyl ester | N-phenylpyruvoyl-L-Pro-t-Butyl ester |
| - 56 | " " | In order each acid from Runs 1-27 | N-phenylpyruvoyl analogs of products of Runs order 1-27, respectively |
| - 84 | 3-cyclohexyl-2-oxo propionic acid | " " " " " | 3-cyclohexyl-2-oxo-N-propionyl |
| -112 | 6-methyl-2-oxoheptanoic acid | " " " " " | 6-methyl-2-oxo-N-heptanoyl analogs of products of Runs 1 -28 respectively |
| -140 | 4-methyl-2-oxopentanoic acid | " " " " " | 4-methyl-2-oxo-N-pentanoyl analogs of products of Runs 1-28, respectively |

| No. | Column A | Column B | Column C |
|---|---|---|---|
| 168 | 2-oxobutyric acid | In order, each acid from Runs 1-28 | 2-oxo-N-butyryl analogs of products of Runs 1-28, respectively |
| 196 | 3-methyl-2-oxobutyric acid | " " " " " | 3-methyl-2-oxo-N-butyryl analogs of products of Runs 1-28, respectively |
| 224 | 2-keto-glutaric acid-5-ethyl ester | " " " " " | 5-ethoxy carbonyl-2-keto-N-Butyryl analogs of products of Runs 1-28, respectively |
| 252 | 2-keto-adipic acid-6-methyl ester | " " " " " | 6-methoxy carbonyl-2-keto-N-pentanoyl analogs of products of Runs 1-28, respectively |
| 280 | 2-keto-1-phenylbutyric acid | " " " " " | 1-phenyl-2-keto-N-butyryl analogs of products of Runs 1-28, respectively |
| 308 | 2-keto-1-(p-chloro-phenyl) butyric acid | " " " " " | 1-p-chlorophenyl-2-keto-N-butyryl analogs of products of Runs 1-28, respectively |
| 336 | 4-(3-indolyl)-2-keto butyric acid | " " " " " | 3-indolyl-2-keto-N-butyryl analogs of products of Runs 1-28, respectively |
| 364 | 4-(N-acetylaminoethyl)-2-ketobutyric acid | " " " " " | 4-(N-acetylaminoethyl)-2-keto-N-butyryl analogs of products of Runs 1-28, respectively |
| 392 | dimethylaminoethyl-2-keto-4-phenylbutyric acid | " " " " " | 4-phenyl-4-dimethylaminoethyl-2-keto-N-butyryl analogs of products of Runs 1-28, respectively |
| 420 | 2-keto-5-methylhexanoic | " " " " " | 5-methyl-2-keto-N-hexanoyl analogs of products of Runs 1-28, respectively |
| 446 | N-5-Boc-aminoethyl-2-keto-4-phenyl-butyric acid | " " " " " | 4-(N-1-Boc-aminoethyl)-4-phenyl-2-keto-N-butyryl analogs of products of Runs 1-28, respectively |
| 474 | phenoxypyruvic acid | " " " " " | phenoxy-N-pyruvoyl analogs of products of Runs 1-28, respectively |

| No. | Column A | Column B | Column C |
|---|---|---|---|
| -502 | p-hydroxyphenoxypyruvic acid | In order, each acid from 1-28 | p-hydroxy phenoxy-N-pyruvoyl analogs of products of Runs 1-28, respectively |
| -530 | phenylthiopyruvic acid | " " " " " | phenylthio-N-pyruvoyl analogs of products of Runs 1-28, respectively |
| -558 | benzyl thiopyruvic acid | " " " " " | benzylthio-N-pyruvoyl analogs of products of Runs 1-28, respectively |
| -586 | indole-3-pyruvic acid | " " " " " | indolyl-N-pyruvoyl analogs of products of Runs 1-28, respectively |
| -614 | 2-keto-3-p-cyanophenyl-propionic acid | " " " " " | 3(p-cyano)phenyl-2-keto-N-propionyl analogs of products of Runs 1-28, respectively |
| -642 | 4-α-naphthyl-2-keto butyric acid | " " " " " | 4-α-naphthyl-2-keto-N-butyryl analogs of products of Runs 1-28, respectively |
| 3-670 | 4-(3,4-dichlorophenyl)-2-oxo-butyric acid | " " " " " | 4-(3,4 dichlorophenyl)-2-keto-N-butyryl analogs of products of Runs 1-28, respectively |
| 1-698 | 2-keto-4-p-phenoxy-phenyl butyric acid | " " " " " | 4-(p-phenoxy)phenyl-2-keto-N-butyryl analogs of products of Runs 1-28, respectively |
| -726 | 3,3-dimethyl pyruvic acid | " " " " " | 3,3-dimethyl-N-pyruvoyl analogs of products of Runs 1-28, respectively |
| -754 | 3-isopropyl pyruvic acid | " " " " " | 3-isopropyl-N-pyruvoyl analogs of products of Runs 1-28, respectively |
| -782 | 3,3-dimethyl-2-keto butyric acid | " " " " " | 3,3-dimethyl-2-oxo-N-pyruvoyl analogs of products of Runs 1-28, respectively |
| 3-800 | 3-methyl-2-ketopentanoic acid | " " " " " | 3-methyl-2-oxo-N-pentanoyl analogs of products of Runs 1-28, respectively |

| No. | Column A | Column B | Column C |
|---|---|---|---|
| -828 | 2-ketoheptanoic acid | In order, each acid from 1-28 | 2-oxo-N-heptanol analogs of products of Runs 1-28, respectively |
| )-856 | 3-isobutylpyruvic acid | " " " " " | 3-isobutyl-N-pyruvoyl analogs of products of Runs 1-28, respectively |
| '-884 | 3,3 dimethyl-2-keto-pentanoic acid | " " " " " | 3,3-dimethyl-2-keto pentanoyl analogs of products of Runs 1-28, respectively |
| ;-912 | 2 keto-octanoic acid | " " " " " | 2-keto-N octanoyl analogs of products of Runs 1-28, respectively |
| )-940 | 2-keto decanoic acid | " " " " " | 2-keto N-decanoyl analogs of products of Runs 1-28, respectively |
| l-968 | 3,3-diisobutyl pyruvic acid | " " " " " | 3,3-diisobutyl-N-pyruvoyl analogs of products of Runs 1-28, respectively |
| )-996 | 3-hydroxy pyruvic acid | " " " " " | 3-hydroxy-N-pyruvoyl analogs of products of Runs 1-28, respectively |
| '-1024 | 4 methylthio-2-keto-butyric acid | " " " " " | 4-methylthio-2-keto-N butyryl analogs of products of Runs 1-28, respectively |
| ?5-1052 | 3-t-butyloxycarbonyl-pyruvic acid | " " " " " | 3-t-butyloxycarbonyl-N-pyruvoyl analogs of products of Runs 1-28, respectively |
| ;3-1080 | 4-ethoxycarbonyl-2-ketobutyric acid | " " " " " | 4-ethoxycarbonyl-2-keto-N-butyryl analogs of products of Runs 1-28, respectively |
| 31-1108 | 2-hydroxyphenyl pyruvic acid | " " " " " | 2-hydroxyphenyl-N-pyruvoyl analogs of products of Runs 1-28, respectively |
| 09-1136 | 3-hydroxyphenyl pyruvic acid | " " " " " | 3-hydroxyphenyl-N-pyruvoyl analogs of products of Runs 1-28, respectively |

| No. | Column A | Column B | Column C |
|---|---|---|---|
| 7-1164 | 4-hydroxyphenyl pyruvic acid | In order, each acid from 1-28 | 4-hydroxyphenyl-N-pyruvoyl analogs of products of Runs 1-28, respectively |
| 5-1192 | 6-Boc-amino-2-keto-hexanoic acid | " " " " " | 6-Boc-amino-2-keto-N-hexanoyl analogs of products of Runs 1-28, respectively |
| 3-1220 | 6-Tos-amino-2-keto-hexanoic acid | " " " " " | 6-Tos-amino-2-keto-N-hexanoyl analogs of products of Runs 1-28, respectively |
| 1-1248 | 6-Cbo-amino-2-keto-hexanoic acid | " " " " " | 6-Cbo-amino-2-keto-N-hexanoyl analogs of products of Runs 1-28, respectively |
| 9-1276 | 6 acetylamino-2-keto-hexanoic acid | " " " " " | 6-acetylamino-2-keto-N-hexanoyl analogs of products of Runs 1-28, respectively |
| 7-1304 | 6 benzoylamino-2 keto-hexanoic acid | " " " " " | 6-benzoylamino-2-keto-N-hexanoyl analogs of products of Runs 1-28, respectively |
| 5-1332 | 5-benzylamino-2-keto-pentanoic acid | " " " " " | 5-benzoylamino-2-keto-N-pentanoyl analogs of products of Runs 1-28, respectively |
| 3-1360 | 5-Boc-amino-2-keto-pentanoic acid | " " " " " | 5-Boc-amino-2-keto-N-pentanoyl analogs of products of Runs 1-28, respectively |
| 1-1388 | 5-Tos-amino-2-keto-pentanoic acid | " " " " " | 5-Tos-amino-2-keto-pentanoyl analogs of products of Runs 1-28, respectively |
| 9-1416 | 5-Cbo-amino-2-keto-pentanoic acid | " " " " " | 5-CbO-amino-2-keto-N-pentanoyl analogs of products of Runs 1-28, respectively |
| 7-1444 | 5-acetylamino-2-keto-pentanoic acid | " " " " " | 5-acetylamino-2-keto-N-pentanoyl analogs of products of Runs 1-28, respectively |
| 5-1472 | 3-imidazolyl-pyruvic acid | " " " " " | 3-imidazolyl-N-pyruvoyl analogs of products of Runs 1-28, respectively |

| n No. | Column A | Column B | Column C |
|---|---|---|---|
| 73-1500 | $N^{im}$-benzyl-3-imidazolyl-pyruvic acid | In order, each acid from 1-28 | $N^{im}$-benzyl-3-imidazolyl-N-pyruvoyl analogs products of Runs 1-28, respectively |

Step B.

Each of the intermediate products of Runs 1-1500 is reacted with each α-aminocarboxylic acid of the following table, or an ethyl, methyl, t-butyl, benzyl, diphenylmethyl or thiophenyl ester thereof, to give a product of this invention in which 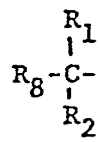 is of the general formula 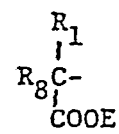

wherein E is H, ethyl, methyl, t-butyl, benzyl, diphenylmethyl or thiophenyl and

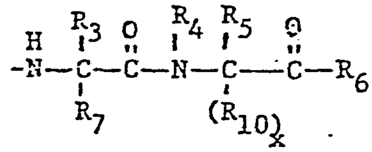 is the radical of

intermediate of Column C above that is used in the synthesis.

| Example No. | $\alpha$-Amino Acid | 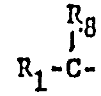 | Intermediate Used | Q |
|---|---|---|---|---|
| 471 | $\beta$-fluoro-L-phenylalanine | 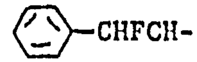 | Each of those from Runs 1-1500 | |
| 472 | $\beta$-thienyl-L-serine | 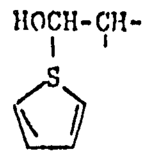 | " " " " " " | |
| 473 | 3',5'-dimethyl-L-tyrosine | 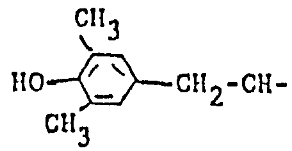 | " " " " " " | |
| 474 | $N^{\varepsilon}$-Boc-3-hydroxy-L-lysine | BOCNH-$(CH_2)_2$CH-$CH_2$-$CH_2$-CH- | " " " " " " | |
| 475 | Boc-3-amino-L-tyrosine | 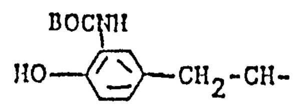 | " " " " " " | |

| Example No. | α-Amino Acid | 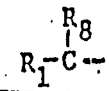 | Intermediate Used |
|---|---|---|---|
| 476 | α-methyl-4-iodo-L-Phe | 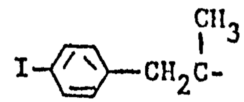 | Each of those from Runs 1-1500 |
| 477 | α-methyl-2-nitro-L-Phe | 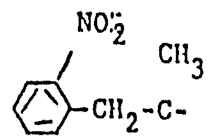 | " " " " " " |
| 478 | α-methyl-4-chloro-L-homo Phe | 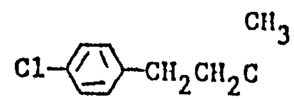 | " " " " " " |
| 479 | α-methyl-3,4-dichloro-L-homo Phe | 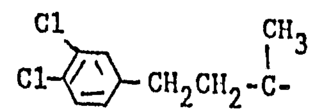 | " " " " " " |
| 480 | α-methyl-β-fluoro-L-Phe | 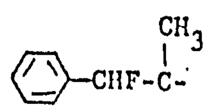 | " " " " " " |
| 481 | p-hydroxyphenoxy-L-Tyr | 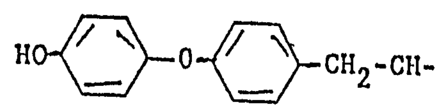 | " " " " " " |

| Example No. | α-Amino Acid | 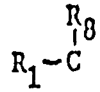 | Intermediate Used |
|---|---|---|---|
| 482 | 5-(p-methylphenyl)-3-oxo-2 aminopentanoic acid | 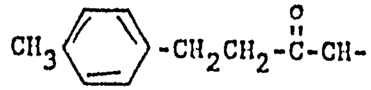 | Each of those from Runs 1-1500 |
| 483 | 6-(p-nitrophenyl)-3-oxo-2-aminohexanoic acid | 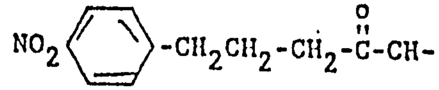 | " " " " " " |
| 484 | 5(p-methylphenyl)-3-hydroxy-2-amino-2-methyl-pentanoic acid | 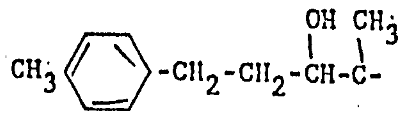 | " " " " " " |
| 485 | 3,5-dibromo-4 hydroxy-phenyl glycine | 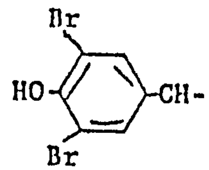 | " " " " " " |
| 486 | 2-amino-2 methyl-3-oxo-7-acetamido heptanoic acid | 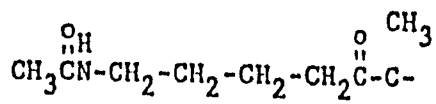 | " " " " " " |
| 487 | 4-cyclopentyl-4-oxo-2-aminobutyric acid | 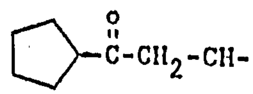 | " " " " " " |

-79-

| ample No. | $\alpha$-Amino Acid | $R_1-\overset{R_8}{C}$ | Intermediate Used |
|---|---|---|---|
| 88 | 5-nitroguanidino-2-amino-2-methyl-pentanoic acid | $NO_2NH\overset{NH}{C}-NHCH_2CH_2CH_2-\overset{CH_3}{C}-$ | Each of those from Runs 1-1500 |
| 89 | 6-benzyloxy-3-oxo-2-amino-hexanoic acid | $CH_2O-CH_2CH_2CH_2-\overset{O}{C}-CH-$ | " " " " " " |
| 90 | 2-amino-3-ureidopropionic acid (albizziin) | $NH_2\overset{O}{C}-NHCH-CH-$ | " " " " " " |

| Example No. | α-Amino Acid | $R_1-\overset{R_{18}}{C}-$ | Intermediate Used |
|---|---|---|---|
| 491 | β-phenylserine | 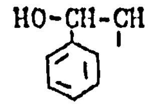 | Each of those from Runs 1-1500 |
| 492 | p-(Tos)amidonophenyl-alanine | 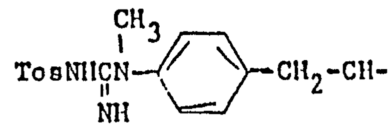 | " " " " " " |
| 493 | p-(Tos)guanidinophenyl Gly | 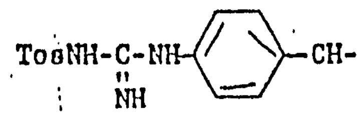 | " " " " " " |
| 494 | γ-N-methylarginine | $CH_3NH-C(=NH)-NH(CH_2)_3-CH-$ | " " " " " " |
| 495 | γ-hydroxyarginine | $NH_2-C(=NH)-NH-CH(OH)-CH_2CH_2-CH-$ | " " " " " " |
| 496 | 5,5'-dihydroxyleucine | 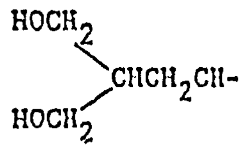 | " " " " " " |
| 497 | β-fluoroaspartic acid | HOOC-CHF-CH- | " " " " " " |
| 498 | β-methylene aspartic acid | $HOOC-C(=CH_2)-CH-$ | " " " " " " |

| Example No. | α-Amino Acid | $R_1-C(R_8)-$ | Intermediate Used |
|---|---|---|---|
| 499 | p-methyl Phe | 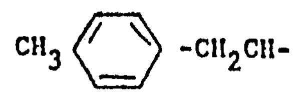 | Each of those from Runs 1-1500 |
| 500 | 2-ethoxy-5-nitro-Phe | 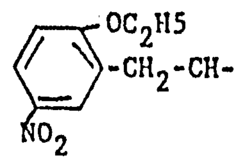 | " " " " " " |
| 501 | p-benzylthio-Phe | 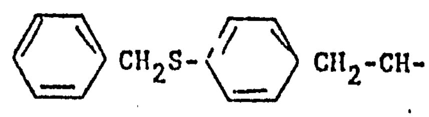 | " " " " " " |
| 502 | 2-amino-2-indole-acetic acid | 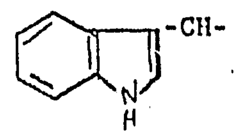 | " " " " " " |
| 503 | 2-methyl-Glu | $HOOC-CH_2-CH_2C(CH_3)-$ | " " " " " " |
| 504 | 2-amino-γ-guanidino butyric acid | $NH_2C(=NH)-NH-CH_2-CH_2-CH-$ | " " " " " " |
| 505 | γ-oxalysine | $NH_2-(CH_2)_2-O-CH_2-CH-$ | " " " " " " |

| Example No. | α-Amino Acid | $R_8$ $R_1$-C- | Intermediate Used |
|---|---|---|---|
| 506 | α,β-dehydro-Phe | 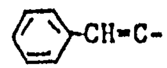 | Each of those from Runs 1-1500 |
| 507 | β,β-dimethyl-dehydro Ala | 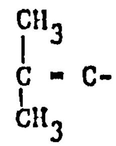 | " " " " " " |
| 508 | β,β-diethyl-dehydro Ala | 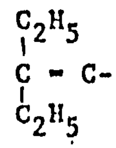 | " " " " " " |
| 509 | β-ethyl, β-phenyl-dehydro Ala | 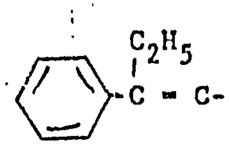 | " " " " " " |
| 510 | β-(3,5 dimethoxy)phenyl, B-methyl dehydro Ala | 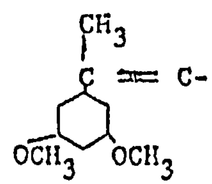 | " " " " " " |
| 511 | $N^{im}$benzyl-histidine | 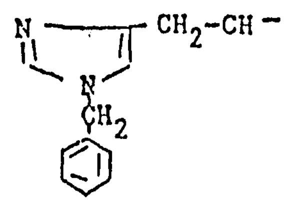 | " " " " " " |

| Example No. | α-Amino Acid | $R_1-\overset{R_8}{\overset{\vert}{C}}-$ | Intermediate Used |
|---|---|---|---|
| 512 | Thyronine | 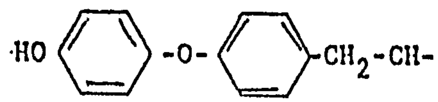 | Each of those in Runs 1-1500 |
| 513 | 2-amino-2-methyl-4-oxo-5-ethoxycarbonyl-pentanoic acid | $C_2H_5O\overset{O}{\overset{\Vert}{C}}-CH_2-\overset{O}{\overset{\Vert}{C}}-CH_2-\overset{CH_3}{\overset{\vert}{C}}-$ | " " " " " " |
| 514 | 2-amino-4-oxo-6t-Butyoxy-carbonyl hexanoic acid | $t\text{-butyl}-O-\underset{O}{\underset{\Vert}{C}}-CH_2-CH_2-\overset{O}{\overset{\Vert}{C}}-CH_2-CH-$ | " " " " " " |
| 515 | 5-Boc-amino-2-amino-3 oxopentanoic acid | $t\text{-butyl}-O\underset{O}{\underset{\Vert}{C}}-\overset{H}{N}-CH_2CH_2\overset{O}{\overset{\Vert}{C}}-CH-$ | " " " " " " |
| 516 | 4-Boc-amino-2-amino-3-oxobutyric acid | $t\ \text{butyl}\ O-\underset{O}{\underset{\Vert}{C}}NHCH-\overset{O}{\overset{\Vert}{C}}-CH-$ | " " " " " " |
| 517 | 2-amino-4-oxo-pentanoic acid | $CH_3CH_2-\overset{O}{\overset{\Vert}{C}}-CH_2-CH-$ | " " " " " " |
| 518 | 2-amino-5-hydroxy-4-oxo-pentanoic acid | $HOCH_2-\overset{O}{\overset{\Vert}{C}}-CH_2-CH-$ | " " " " " " |

| Example No. | α-Amino Acid | 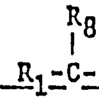 | Intermediate Used |
|---|---|---|---|
| 519 | 2-amino-5-benzylthio-4 oxopentanoic acid | 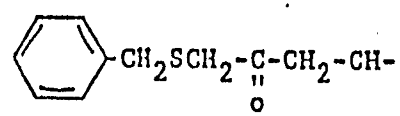 | Each of those in Runs 1-1500 |
| 520 | 2-amino-2-methyl-4-oxo pentanoic acid | 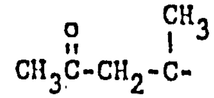 | " " " " " " |
| 521 | 2-amino-6-benzylthio-2 methyl-5 oxo-hexanoic acid | 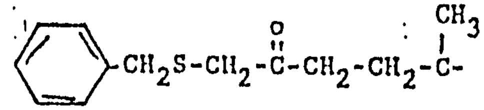 | " " " " " " |
| 522 | 2-amino-2-methyl-4 hydroxy pentanoic acid | 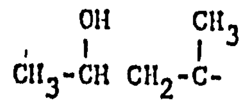 | " " " " " " |
| 523 | 2-amino-2-methyl-5-ethoxy-carbonyl-4-hydroxy pentanoic acid | 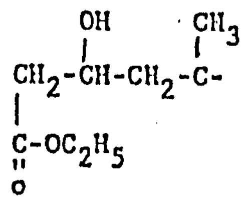 | " " " " " " |
| 524 | 2-amino-3-methoxy-2-methyl pentanoic acid | 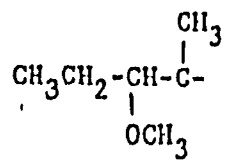 | " " " " " " |

| Example No. | α-Amino Acid | $R_8$<br>$R_1$-C- | Intermediate Used |
|---|---|---|---|
| 525 | 2-amino-4-benzyloxy-6-t-Boc-hexanoic acid | 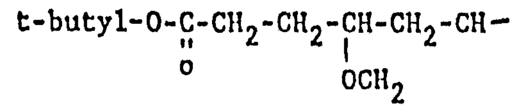 | Each of those in Runs 1-1500 |
| 526 | 3-(2-methyl-4,5 dihydroxyphenyl)-Ala | 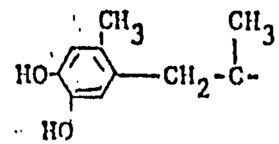 | " " " " " " |
| 527 | 3-(2-ethyl-4,5 dihydroxyphenyl)-Ala | 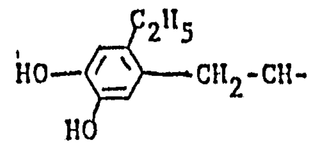 | " " " " " " |
| 528 | 3-(2-isopropyl-4,5 dihydroxyphenyl)-Ala | 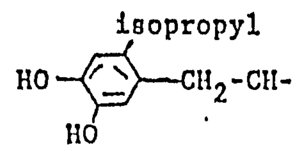 | " " " " " " |
| 529 | 3-(2,5-dimethoxy-4-methylphenyl)-Ala | 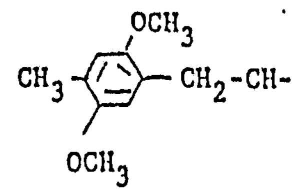 | " " " " " " |

| Example No. | α-Amino Acid | $R_1-\overset{R_8}{\overset{\mid}{C}}-$ | Intermediate Used |
|---|---|---|---|
| 530 | 3-ethyl-α-methyl Tyr | 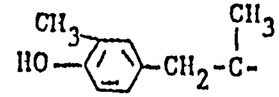 | Each of those in Runs 1-1500 |
| 531 | 3-isopropyl-α-methyl-Tyr | 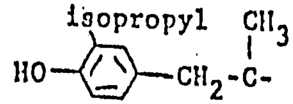 | " " " " " " |
| 532 | 3-t-butyl-α-methyl-Tyr | 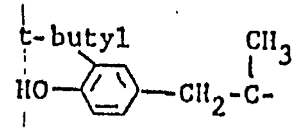 | " " " " " " |
| 533 | 3-(3,5-difluorophenyl)-Ala | 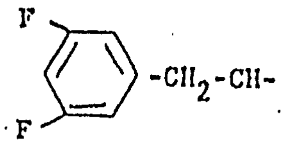 | " " " " " " |
| 534 | 3-(3,4-difluorophenyl)-Ala | 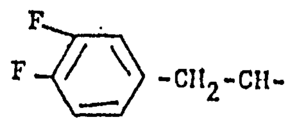 | " " " " " " |
| 535 | 3-(2,6-difluorophenyl)-Ala | 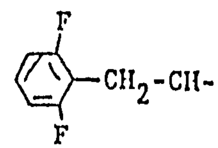 | " " " " " " |

| Example No. | α-Amino Acid | $R_1$-C- | Intermediate Used |
|---|---|---|---|
| 536 | 3-(2,3,5,6-tetrafluoro-phenyl)-Ala | 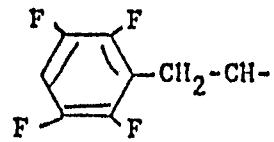 | Each of those in Runs 1-1500 |
| 537 | 3-(3,5-dichloro-2,4,6-trifluorophenyl)-Ala | 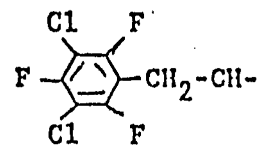 | " " " " " " |
| 538 | 3-(2,3,4,5,6-penta-fluorophenyl)-Ala | 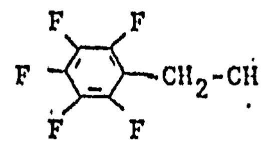 | " " " " " " |
| 539 | β-(1,2-d hydro-2-oxo-4 pyridyl)-Ala | 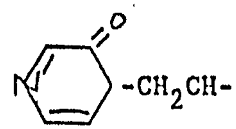 | " " " " " " |
| 540 | β-(2 fluoro-6-pyridyl)-Ala | 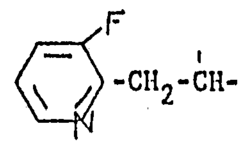 | " " " " " " |
| 541 | β-(2-chloro-3-pyridyl)-Ala | 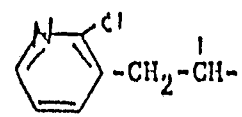 | " " " " " " |

| Example No. | α-Amino Acid | $R_1-\overset{R_\beta}{\overset{\vert}{C}}-$ | Intermediate Used |
|---|---|---|---|
| 542 | β-(thymin-1-yl)-Ala | 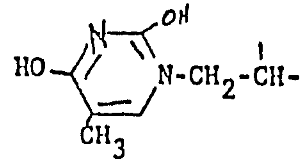 | Each of those in Runs 1-1500 |
| 543 | γ,δ,δ,-trihydroxy-Leu | $(HOCH_2)-C(OH)-CH_2CH-$ | " " " " " " |
| 544 | O-ethylhomo-Ser | $C_2H_5OCH_2CH_2CH-$ | " " " " " " |
| 545 | 3-hydroxyphenylGly | 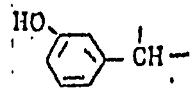 | " " " " " " |
| 546 | 3,5 dehydroxyphenylGly | 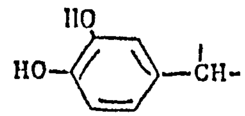 | " " " " " " |
| 547 | 3,4,5-triiodo-Phe | 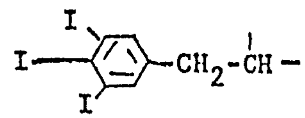 | " " " " " " |
| 548 | β-(4 methoxy-1-naphthyl) | 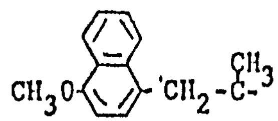 | " " " " " " |

Where no other indication is given, the α-amino acid is used in its L, D or D, L form.

Examples 549-625

A. Synthesis of N-(D-[+]-2-bromo-propionyl)-L-proline-t-butyl ester

A solution of 40 mmols of D-[+]-2-bromo-propionic acid in 15 ml. of redistilled dichloromethane was cooled to -40°C. A cold solution of dicyclohexylcarbodiimide (DCC), 40 mmols, in 10 ml. of dichloromethane, was added dropwise while maintaining the temperature between -35 and-40°C. After 10 minutes of stirring, L-proline-t-butyl ester, 40 mmols, in 15 ml. of dichloromethane at -45°C, was added dropwise with stirring. After 1.5 hours at -45°C, the reaction mixture was stirred at 4°C overnight. The precipitate of dicyclohexylurea was removed by filtration. The organic filtrate was washed until neutral, dried over anhydrous $MgSO_4$ and filtered again. The solvent was removed by rotary evaporation under reduced pressure to yield 10.6 g. of oily residue. The product exhibited an optical rotation, when dissolved in ethyl acetate in a concentration of 0.036835 g./ml. of $[\alpha]_D^{24} = -56.82°$. Upon elemental analysis for $C_{12}H_{20}NBrO_3$ (Formula weight = 306.209), the results were

Calculated: C 47.04, H 6.58, N 4.57

Found: C 47.00, H 6.52, N 4.56

Instead of using D-[+]-2-bromopropionic acid in the above synthesis of the intermediate

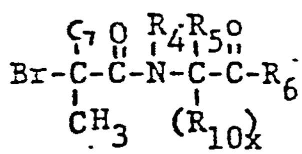

, the foregoing synthesis is repeated using each acid from the following list to make either

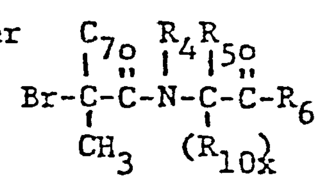

or a compound in which H- replaces Br

List A



2-bromoarginic acid

2-bromo-3-methylbutyric acid

2-bromo-3,3-dimethylpentanoic acid

2-bromo-3-imidazolyl-propionic acid

2-bromo-3-methyl-5-guanidino pentanoic acid

L-[-]-2-bromopropionic acid

2-bromoisobutyric acid

N-t-Boc-L-alanine

N-t-Boc-α-methyl-Ala

N-Boc-α- methyl-L-valine

N -Boc-α-methyl-D-valine

N -Boc-arginine

N -Boc-nitroarginine

N -Boc-γ-methyl nitroarginine

N -Boc-leucine

N -Boc-Ile

N -Bocα-methyl Leu

N -Boc asparagine

2-bromo-4-oxopentanoic acid

2-bromo-3-benzylthio-3-methyl-butyric acid

2-bromo-5-Boc-amino-3-oxo pentanoic acid

2-bromo-5-ethoxycarbonyl-4-oxo-pentanoic acid

2-bromo-3-methoxy-pentanoic acid

2-bromo-3-(p-hydroxy-m-t-butylphenyl)-propionic acid

2-bromo-3,3,3-trimethyl propionic acid

2-bromo-2-(3,4-dihydroxyphenyl)-acetic acid

2-bromo-4-t-butyloxycarbonyl-pentanoic acid

2-bromo-3,4-dimethylpentanoic acid

2-bromo-5-COb-aminopentanoic acid

2-bromo-3-(4-p-hydroxyphenoxyphenyl)-propionic acid

2-bromo-3-phenyl propionic acid

N -Boc-β-fluoro Phe

N -Boc-homo-Arg

N -Boc-homo-nitro Arg

N -Boc-glutamine

N -Boc-ρ-methylphenyl Gly

N -Boc-3,4,5-triiodo-Phe

N -Boc-3,5-dihydroxyphenyl Gly

N -Boc-3-hydroxyphenyl Gly

N -Boc-O-ethyl-α-methyl Ser

N -Boc-trileucine

N -Boc-(3 chloro-3-pyridyl)-Ala

N -Boc-(2 fluoro-6-pyridyl)-Ala

N -Boc-3-(2,3,4,5,6-pentafluorophenyl)-Ala

N -Boc-3-(3,5-dichloro-2,4,6-trifluorophenyl)-Ala

N -Boc-3-t-butyl- -methyl-Tyr

N -Boc-β,β-difluoro-Ala

N -Boc-3-isopropyl-α-methyl-Tyr

N -Boc-3(2,5-dimethoxy-4-methylphenyl)-Ala

N -Boc-3-(2-methyl-4,5 dehydroxyphenyl)Ala

N -Boc-2-amino-4-benzyloxy-6-t-Boc-hexanoic acid

N -Boc-2-amino-3-methoxy-2-methylpentanoic acid

N -Boc-β-(4 methoxy-1 naphthyl)-α-methyl-Ala

N -Boc-2-amino-2-methyl-5-ethoxycarbonyl-4-hydroxy-pentanoic acid

N -Boc-2-amino-5-Boc-amino-3-hydroxy-pentanoic acid

N -Boc-β,β-dimethyl-Cys

N -Boc-2-amino-2-methyl-4 hydroxypentanoic acid

N -Boc-2-amino-6-benzylthio-2-methyl-5-oxo hexanoic acid

N -Boc-2-amino-6-mercapto-2-methyl-5-oxohexanoic acid

N -Boc-2-amino-2-methyl-4-oxo-pentanoic acid

N -Boc-2-amino-3-methyl-4-oxo-pentanoic acid

N -Boc-2-amino-5-hydroxy-4-oxo-pentanoic acid

N -Boc-2-amino-4-oxo-pentanoic acid

N -Boc-2-amino-2-methyl-4-oxo-5-ethoxycarbonyl pentanoic acid

N -Boc-5-Cbo-amino-2-amino-3-oxo-pentanoic acid

N -Boc-thyronine

Nα-Boc-$N^{im}$-benzyl histidine

N -Boc-β-(3,4-dimethoxyphenyl)-β-methyl-dehydro-Ala

N -Boc-β,β-diethyl-dehydro-Ala

N -Boc-albizziin

N -Boc-γ-oxalysine

N -Boc-2-amino-2-indole-acetic acid

N -Boc,$N^{\gamma}$-Boc-γ-hydroxy-Lys

N -Boc,$N^{\epsilon}$ hydroxy Lys

N -Boc-δ-N-methyl-Arg

N -Boc-δ-hydroxy-Arg

N -Boc-5,5'-dihydroxy-Leu

N -Boc-β-fluoroaspartic acid

N -Boc-β-methyl-aspartic acid

N -Boc-β-methylene aspartic acid

N -Boc-p(Tos)guanidino-phenyl Gly

N -Boc-p-guanidino-phenyl-Gly

N -Boc-p-methyl Phe

N -Boc-2-ethoxy-5-nitro-Phe

N -Boc-p-benzylthio-Phe

N -Boc-p-mercapto-Phe

N -Boc-α-methyl Phe

N -Boc-α-methyl-Leu

N -Boc-α-methyl-Ile

N -Boc-α-methyl-α-aminobutyric acid

N -Boc-α-fluoromethyl-Ala

N -Boc-αBoc-aminomethyl-valine

N -Boc-α-methyl glutamic acid

N -Boc-α-methyl glutamine

N -Boc-α-fluoromethyl-norvaline

N -Boc-α-methyl-histidine

N -Boc-α-methyl Asp

N -Boc-α-methyl-homo-Lys

N -Boc-α-methyl-nitro-Arg

N -Boc-α-methyl-O-benzyl-Ser

N -Boc-α-methyl-O-phenyl-Ser

N -Boc-α-methyl-O-t-butyl-Ser

N -Boc-α-methyl-O-methyl-Ser

N -Boc-α-methyl-O(p-hydroxyphenyl)-Ser

N -Boc-α-methyl-O-benzyl-threonine

N -Boc-α-methyl-O-ethyl-threonine

N -Boc-α-methyl-S-t-butyl-cysteine

N -Boc-α-methyl-S-methyl-cysteine

N -Boc-α-methyl-S-benzyl-homocystine

N -Boc-α-methyl-O-methyl-homoserine

In each instance, the acid is used in its L-, D- or D,L- form to make the corresponding intermediate.

B. Each of the foregoing syntheses of intermediates is repeated using each of the acids named above with each of the below-listed 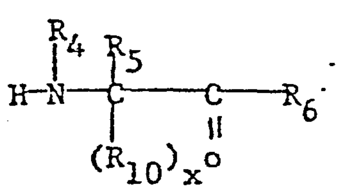 compounds in lieu of

L-Pro-t-butyl ester to make further intermediates:

List B

Pro-amide

Pro-ethyl ester

Pro methyl ester

Pro diphenylmethyl ester

Pro thiophenyl ester

thiazolidine-4-carboxylic acid

4-hydroxy-Pro

3-hydroxy-Pro

4-methoxy-Pro

4-propyl-Pro

Pip

Aze-2-carboxylic acid

5-keto-Pro

3-methoxy-Pro

2-fluoro-Pro

4-Boc-amino-Pro

4-Boc-aminomethyl-Pro

4-acetylthio-Pro

4-fluoromethyl-Pro

α-methyl-Pro

4-iodo-Pro

4-hydroxymethyl-Pro

2,3-dehydro-Pro

3,4-dehydro-Pro

4,5-dehydro-Pro

4-hydroxy-Pro

3-hydroxy-Pro

3-chloro-Pro

4-bromo-Pro

3,4-difluoro-Pro

3-hydroxy-5-methyl-Pro

5-phenylthio-Pro

2-(2 hydroxyphenyl)-thiazolidine -4 carboxylic acid

5-(2-hydroxyphenyl)thio Pro

3-methylthio-Pro

3-benzylthio-Pro

4-methyl-Pro

4-methylene-Pro

4-fluoromethyl-Pro

Each of the List B compounds is used in its L-form.

C. Each of the Nα-Boc intermediates prepared in A. or B. above is reacted with each of the α-amino acids listed in Examples 471-548 above, to yield a compound of this invention.

Example 626

Synthesis of N-(L-1-benzyloxycarbonyl-3-phenyl-propyl)-D-Val-L-Pro-t-butyl ester

Combine 2 mmols of L-homoPhe benzyl ester, 2 mmols of 2-bromo-3,3-dimethyl-propionyl-L-Pro-t-butyl ester and 5 mmols of silver oxide in 50 ml. of benzene. Reflux for 24 hours and then add 2 mmols of 2-bromo-3,3-dimethyl-propionyl-L-proline-t-butyl ester and 5 mmols of silver oxide. Reflux for another 24 hours. Cool, filter, strip off the solvent, and isolate the named compound by conventional chromatrographic techniques. The two ester groups are each removed as desired from portions of the product to yield each

a. N-(L-1-benzyloxycarbonyl-3-phenylpropyl)-D-Val-L-Pro

b. N-L-1-carboxy-3-phenylpropyl)-D-Val-L-Pro-t-butyl ester

c. N-(L-1-carboxy-3-phenylpropyl)-D-Val-L-Pro.

Examples 627-704

Using the method of Example 626, each of the bromo-intermediates from Examples 549-625 is reacted with each α-amino acid and derivative thereof from Examples 471-548 to yield a compound of this invention. The following Table illustrates representative compounds of the invention so prepared. Since, in all of these compounds, $R_2$ is COOE, wherein $E_1$ 1 is OH, $OCH_3$, $OC_2H_5$, O-t-butyl, S-phenyl or SH; $R_1$ and $R_{10}$ are each H and X = -NH-, only the variables $R_1$, $R_3$, $R_7$, amd 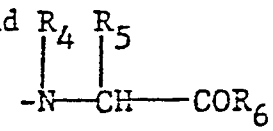 are covered in the Table:

Example 705

An intermediate of the general formula

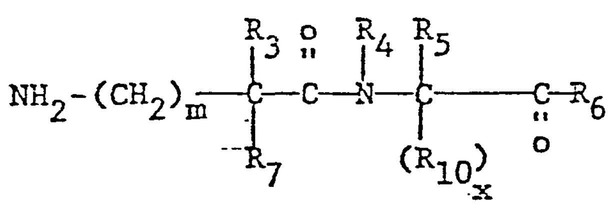

is reacted with an α-keto-carboxylic acid or a suitably protected α-keto-carboxylic acid selected from the following list:

List C

3-methyl-2-oxo-pentanoic acid

2-oxo-heptanoic acid

3-isobutyl-pyruvic acid

3,3-dimethyl-2-oxo-pentanoic acid

$N^{im}$-benzyl-3-imidazolyl-pyruvic acid

5-benzoylamido-2-oxo-pentanoic acid

5-Boc-amino-2-oxo-pentanoic acid

6-CbO-amino-2-oxo-hexanoic acid

6-Tos-amino-2-oxo-hexanoic acid

4-HO-phenyl-pyruvic acid

4-ethoxycarbonyl-2-oxo-butyric acid

4-methylthio-2-oxo-butyric acid

3-OH-3-methyl-pyruvic acid

3-phenyloxypyruvic acid

3,3-isobutyl-pyruvic acid

2-oxo-decanoic acid

2-oxo-octanoic acid

3,3-dimethyl-2-oxo-butyric acid

3-isopropyl-pyruvic acid

3,3-dimethyl-pyruvic acid

p-OH-phenoxypyruvic acid

$N^{\varepsilon}$-Boc-aminoethyl-2-oxo-4-phenylbutyric acid

5-methyl-2-oxo-heptanoic acid

dimethylaminoethyl-2-oxo-4-phenylbutyric acid

4-(3-indolyl)-2-oxo-butyric acid

2-oxo-4-(p-Cl-phenyl)-butyric acid

2-oxo-phenylbutyric acid

2-oxo-adipic acid-6-methyl ester

2-oxo-glutamic acid-5-ethyl ester

2-oxo-butyric acid

4-methyl-2-oxopentanoic acid

6-methyl-2-oxoheptanoic acid

3-cyclohexyl-2-oxopropionic acid

phenylpyruvic acid

phenylthio-pyruvic acid

benzylthio-pyruvic acid

benzyloxy-pyruvic acid

indole-3-pyruvic acid

2-oxo-3-p-cyanophenylpropionic acid

4-α-naphthyl-2-oxo-butyric acid

4-(3,4-dichlorophenyl)-2-oxo-butyric acid

2-oxo-(4-p-phenoxyphenyl)-butyric acid

Compounds of the invention which are prepared by this method include:

| Compound Identification | Compound Name |
|---|---|
| A. | N-(1-carboxy-2-trimethylethyl)-Arg-Pro |
| B. | N-(1-carboxy-4-carboxyamido-butyl)-2-amino-3,3,3-trimethyl-propionyl-L-4-fluoro-Pro. |
| C. | N-(1-carboxy-2-fluoro-2-phenylethyl)-Val-L-4-methoxy-Pro |
| D. | N-(1-carboxy-3-phenylpropyl)-2-amino-(5-Boc-amino-2-oxo)-pentanoyl-L-3,4-difluoro-Pro. |
| E. | N -(1-carboxy-4-guanidino-butyl)-His-L-5-keto-4-Pro |
| F. | N-(1-carboxy-2-phenoxyethyl)-2-amino-4-oxo-pentanoyl-L-thiazolidine-2-carboxylic acid |
| G. | N-(1-carboxy-2-benzyloxyethyl)-(m-t-butyl)Tyr-L-4-methyl-Pro |
| H. | N-(1-carboxy-4-Boc-aminobutyl)-2-amino-3,3,3-trimethyl-propionyl-L-α-methyl-Pro. |

| Compound Identification | Compound Name |
|---|---|
| I. | N-(1-carboxy-2-dimethyl-2-benzylthio-ethyl) Gln-L-Aze-2-carboxylic acid |
| J. | N-[1-carboxy-4-($N^G$-methylguanidino)butyl]-2 amino-3-methoxy-pentanoyl-L-Pip |
| K. | N-[1-ethoxycarbonyl-2-(p-guanidino-phenyl) ethyl]-Glu($\gamma$-t-butyl ester)-5-phenyl-L-Pro |

Example 706

Specific compounds of this invention are prepared by reacting an $\alpha$-amino acid or ester of the general formula

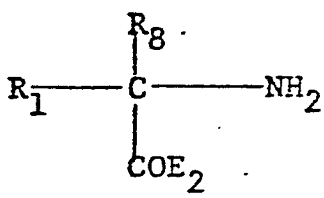

wherein $E_2$ is OH, SH, $OC_2H_5$, $OCH_2$ or O-t-butyl, with an intermediate

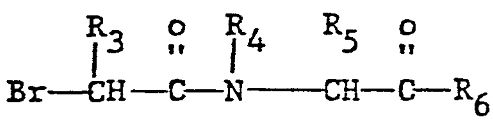

or

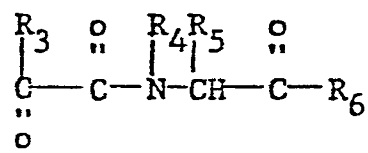

They include those of the following table, wherein X = -NH-, $R_7$ and $R_{10}$ = H, m = 0, $R_2$ is $COE_2$ and the variable groups are as depicted:

Table I

| Compound | $R_1$ | $R_3$ | $R_7$ | $R_4$ $R_5$ $-N-CH-COR_6$ |
|---|---|---|---|---|
| | 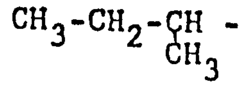 | 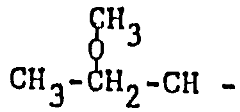 | $CH_3$ - | 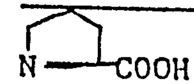 |
| | $CH_3-(CH_2)_4$ - | 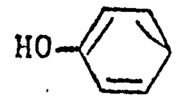 | H - | 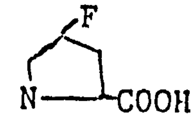 |
| | 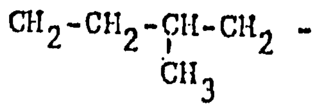 | 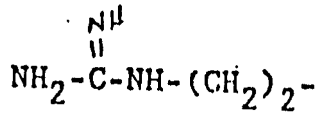 | H - | 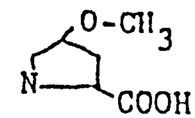 |
| | 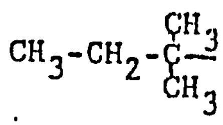 | 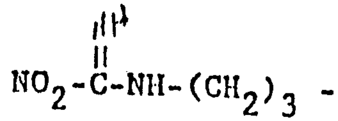 | H - | 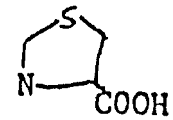 |
| | 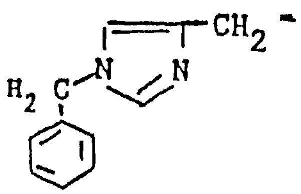 | 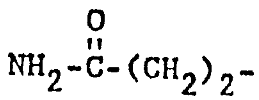 | H - | 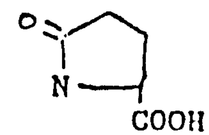 |

## Table (b)

| Compound No. | $R_1$ | $R_3$ | $R_7$ | $-\overset{R_4}{\overset{\vert}{N}}--\overset{R_5}{\overset{\vert}{CH}}-COR_6$ |
|---|---|---|---|---|
| 6 | 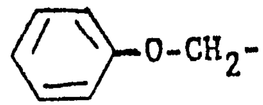 | $CH_3-$ | H- | 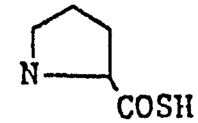 |
| 7 | 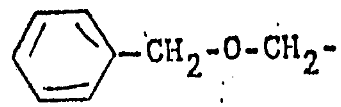 | $CH_3-$ | $CH_3-$ | 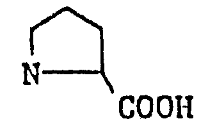 |
| 8 | 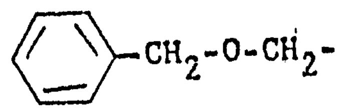 | $CH_3-\overset{O}{\overset{\Vert}{C}}-\underset{CH_3}{CH}-$ | H - | 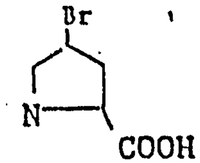 |
| 9 | 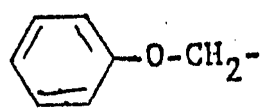 | $NH_2-C-NH-(CH_2)_3-$ | $CH_3-$ | 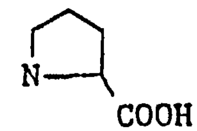 |
| 10 | 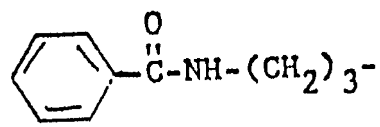 | $HS-CH_2-CH_2-CH_2-CH_2-$ | $CH_3-$ | 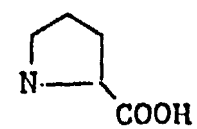 |

Table (c)

| Compound No. | $R_1$ | $R_3$ | $R_7$ | $-N(R_4)-CH(R_5)-COR_6$ |
| --- | --- | --- | --- | --- |
| 11 | 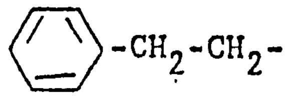 | 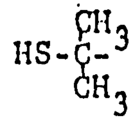 | H - | 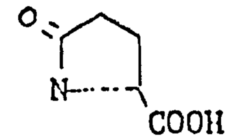 |
| 12 | 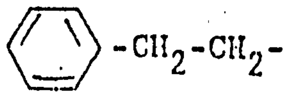 | $CH_3-$ | $CH_3-$ | 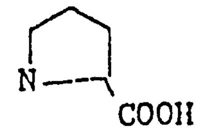 |
| 13 | 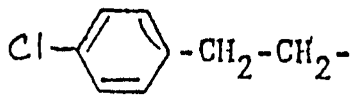 | $CH_3-$ | $CH_3-CH_2-$ | 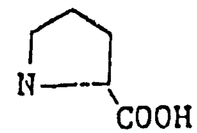 |
| 14 | 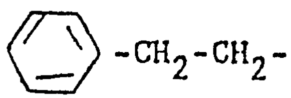 | $CH_3-$ | H | 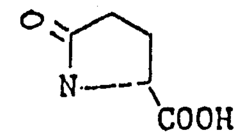 |
| 15 | 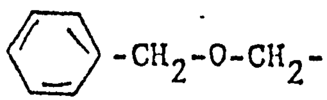 | $CH_3-$ | H. | 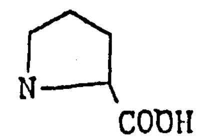 |

Table
(d)

| mpound No. | $R_1$ | $R_3$ | $R_7$ | $R_4$ $R_5$<br>$-N—CH-COR_6$ |
|---|---|---|---|---|
| | $CH_3$<br>$CH-CH_2-$<br>$CH_3$ | $CH_3$<br>$CH_3-C-$<br>$CH_3$ | H- | 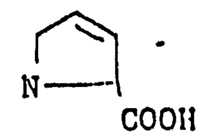 |
| | $(CH_3)_3CO\overset{O}{\overset{\parallel}{C}}-NH-(CH_2)_3-$ | $HO-CH_2-\overset{O}{\overset{\parallel}{C}}-CH_2$ | H- | 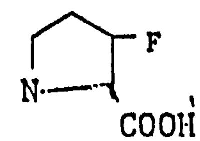 |
| | $CH_3-$ 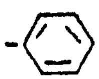 $-SO_2-NH-(CH_2)_3-$ | $Et{\cdot}OOC-CH_2-\overset{OH}{CH}-CH_2-$ | $CH_3-$ | 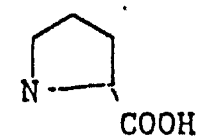 |
| | 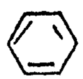 $-O-CH_2-$ | 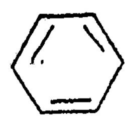 $CH_2-S-CH_2-\overset{O}{\overset{\parallel}{C}}-CH_2-CH_2-$ | $CH_3-$ | 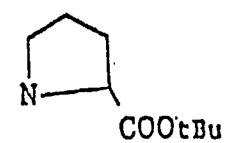 |
| | 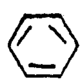 $-CH_2-CH_2-$ | 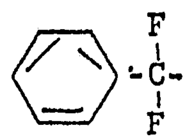 | H | 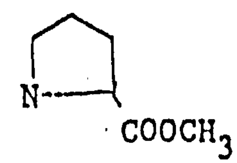 |

Table (e)

| Compound No. | $R_1$ | $R_3$ | $R_7$ | $\overset{R_4}{N}-\overset{R_5}{CH}-COR_6$ |
|---|---|---|---|---|
| 21 | 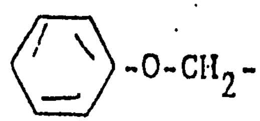 | $(CH_3)_3C\text{-}O\text{-}\overset{O}{\overset{\|}{C}}\text{-}NH\text{-}CH_2\text{-}\overset{OH}{\overset{\|}{CH}}\text{-}$ | H | 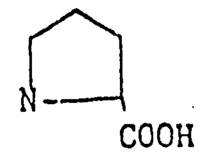 |
| 22 | 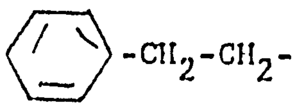 | $CH_3$ | $(CH_3)\text{-}CH\text{-}$ | 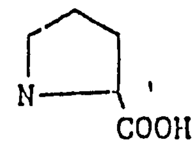 |
| 23 | 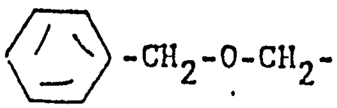 | 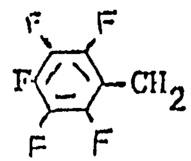 | H | 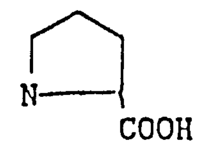 |
| 24 | $(CH_3)_3CO\overset{O}{\overset{\|}{C}}\text{-}NH\text{-}(CH_2)_4\text{-}$ | $F_2CH\text{-}$ | H | 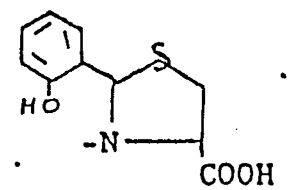 |
| 25 | 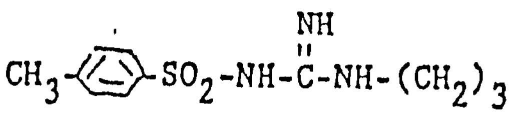 | 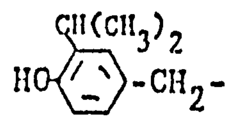 | $CH_3\text{-}$ | 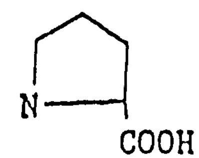 |

Table
(f)

| Compound No. | $R_1$ | $R_3$ | $R_7$ | $R_4$ $R_5$ -N . CH-$COR_6$ |
|---|---|---|---|---|
| 26 | 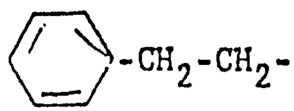 | 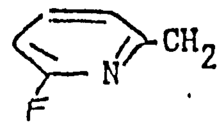 | H- | 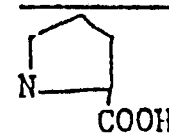 |
| 27 | $(CH_3)COC-NH-(CH_2)_4-$ | 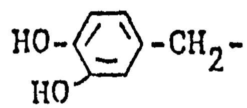 | $CH_3$- | 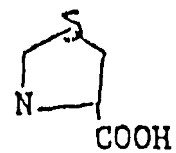 |
| 28 | 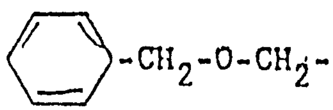 | 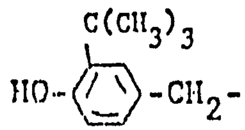 | $CH_3$- | 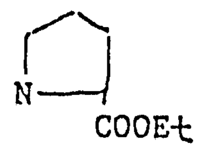 |
| 29 | 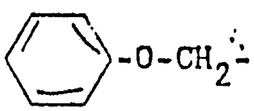 | 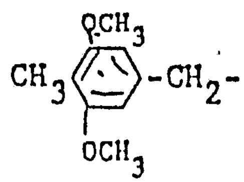 | H- | 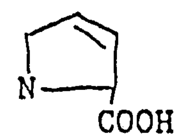 |
| 30 | 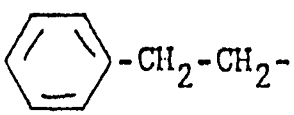 | 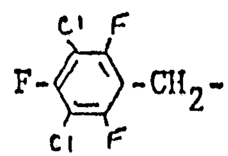 | H | 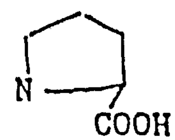 |

Table (g)

| Compound No. | $R_1$ | $R_3$ | $R_7$ | $R_4$ $R_5$ N—CH-$COR_6$ |
|---|---|---|---|---|
| 31 | 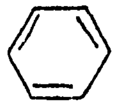 $-CH_2-CH_2-$ | H- | $CH_3-$ | 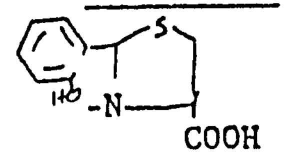 |
| 32 | 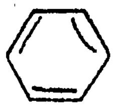 $-O-CH_2-$ | H- | $CH_3-$ | 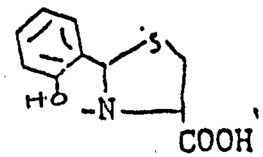 |
| 33 | 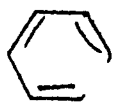 $-CH_2-O-CH_2-$ | H- | $CH_3-$ | 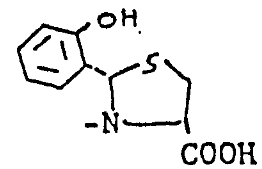 |
| 34 | Cl 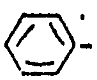 $-CH_2-CH_2-$ | H- | $CH_3-$ | 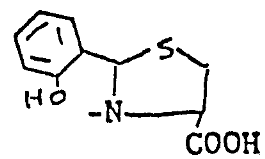 |
| 35 | $(CH_3)CO\overset{O}{\overset{\parallel}{C}}-NH_2-(CH_2)_4-$ | H- | $CH_3-$ | 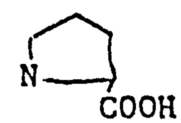 |

Table (h)

| ompound No. | $R_1$ | $R_3$ | $R_7$ | $R_4$ $R_5$ $-N—CH-COR_6$ |
|---|---|---|---|---|
| 5 | 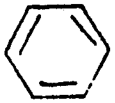 $-CH_2-CH_2-$ | $CH_3-$ | 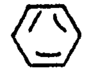 - | 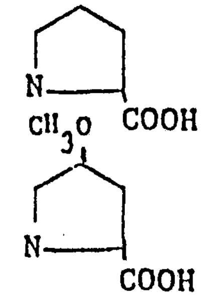 |
| 7 | 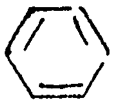 $-O-CH_2-$ | $CH_3-CH_2-O-CH_2-$ | $CH_3-$ | |
| 3 | 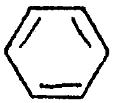 $-CH_2-O-CH_2-$ | $CH_3-$ | 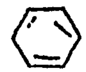 $-CH_2-$ | 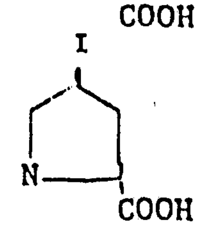 |
| 1 | 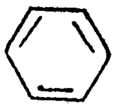 $-CH_2-CH_2-$ | $CH_3-$ | HO- 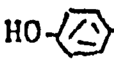 $-CH_2-$ | 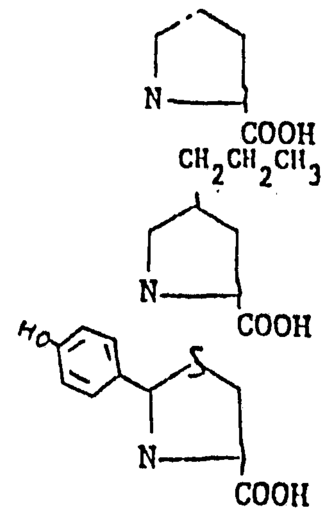 |
| | 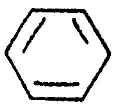 $-CH_2-CH_2-$ | H- | $(CH_3)_2CH-$ | |
| | 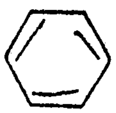 $-CH_2-CH_2-$ | H- | $CH_3CH_2-$ | |

Table (i)

| Compound No. | $R_1$ | $R_3$ | $R_7$ | $R_4$ $R_5$ $-N--CH-COR_6$ |
|---|---|---|---|---|
| 42 | 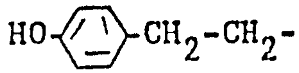 | $NH_2-CH_2-O-CH_2-CH_2-$ | H- | 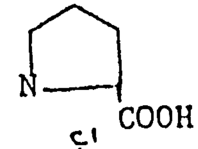 |
| 43 | 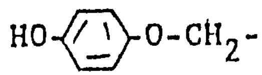 | 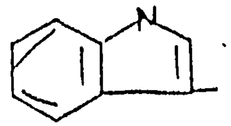 | H- | 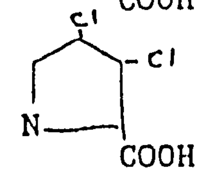 |
| 44 | 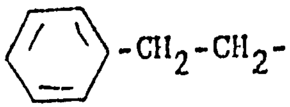 | $CH_3-$ | $CH_2F-$ | 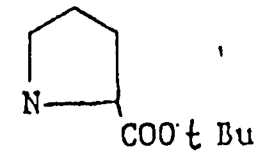 |
| 45 | 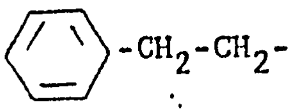 | $(CH_3)_2CH-$ | $(CH_3)_3C-O-C(=O)-NH-CH_2-$ | 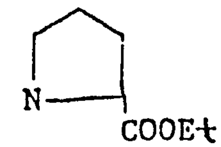 |
| 46 | 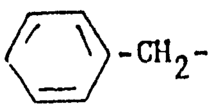 | 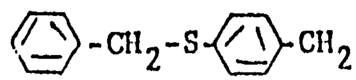 | H- | 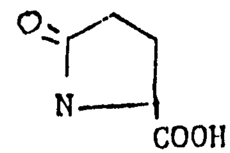 |
| 47 | 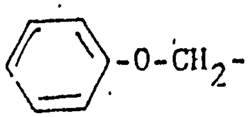 | 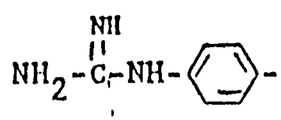 | H- | 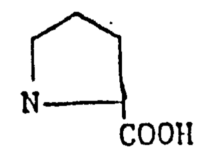 |

Table (j)

| Compound No. | $R_1$ | $R_3$ | $R_7$ | $-N(R_4)-CH(R_5)-COR_6$ |
|---|---|---|---|---|
| 48 | 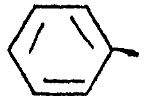 $-CH_2-CH_2-$ | $HONH-(CH_2)_4-$ | H- | 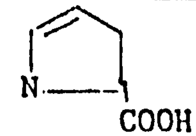 |
| 49 | 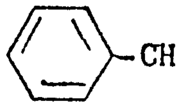 $-CH_2-CH_2-$ | NH<br>‖<br>$N_2H-C-N-(CH_2)_3-$<br>$CH_3$ | H- | 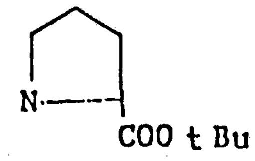 |
| 50 | 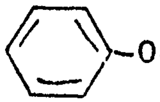 $-O-CH_2-$ | NH<br>‖<br>$H_2N-C-N-(CH_2)_3-$<br>OH | H- | 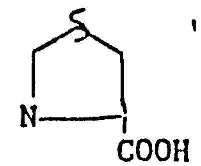 |
| 51 | 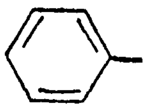 $-CH_2-O-CH_2-$ | OH<br>$CH_2$<br>\|<br>$HOCH_2-CH-CH_2-CH_2-$ | H- | 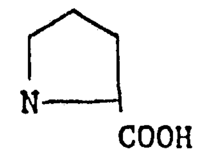 |
| 52 | 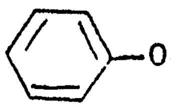 $-O-CH_2-$ | $HOOC-CH-$<br>F | H | 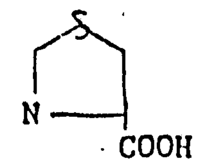 |

Table (k)

| Compound No. | $R_1$ | $R_3$ | $R_7$ | $-N(R_4)-CH(R_5)-COR_6$ |
|---|---|---|---|---|
| 53 | 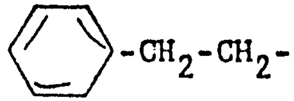 | $(CH_3)_2-CH-CH_2-$ | $CH_3-$ | 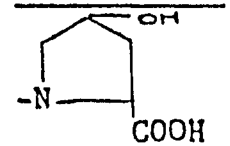 |
| 54 | 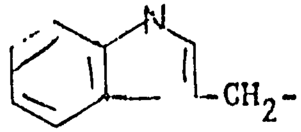 | $HS-CH_2-CH(OH)-CH_2-$ | $CH_3-$ | 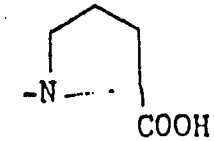 |
| 55 | 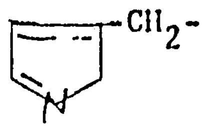 | $CH_3-CH_2-C(=O)-CH_2-$ | $CH_3-$ | 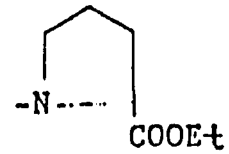 |
| 56 | 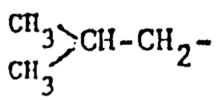 | $CH_3-$ | $CH_3-$ | 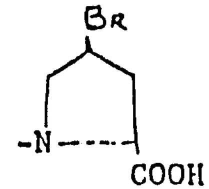 |
| 57 | 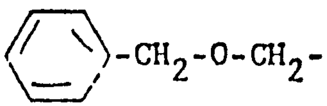 | $CH_3-C(=O)-CH_2-$ | H- | 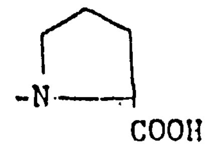 |
| 58 | 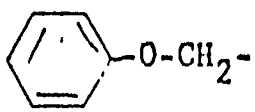 | 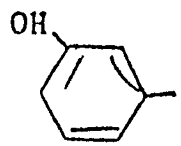 | H- | 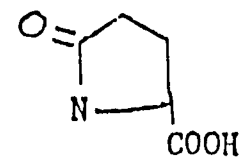 |

Table II

(For the compounds that follow, $R_7$=H, and $R_2$ can be COOH, COSH, COOEt, COOMe, COOtBu)

| Compound No. | $R_8$ | $R_1$ | $R_3$ | $-\overset{R_4}{N}-\overset{R_5}{CH}-COR_6$ |
|---|---|---|---|---|
| 59 | H | $(CH_3)_3CO\overset{O}{C}-NH-CH_2-\overset{OH}{CH}-(CH_2)_2-$ | 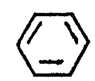 $-CH_2-$ | 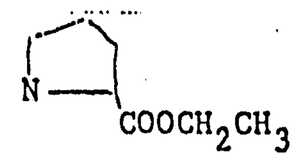 |
| 60 | H | $NH_2-CH_2-\overset{OH}{CH}-(CH_2)-$ | 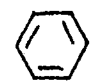 $-CH_2-$ | 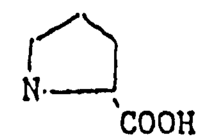 |
| 61 | H | $HONH-(CH_2)_4-$ | $(CH_3)_2CH(CH_2)_3-$ | 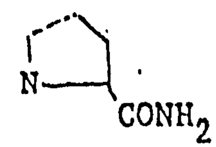 |
| 62 | H | $H_2N-\overset{NH}{C}-\overset{CH_3}{N}-(CH_2)_3-$ | $(CH_3)_2CH-$ | 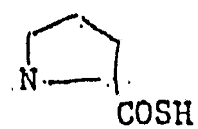 |
| 63 | H | $H_2N-\overset{NH}{C}-\overset{OH}{N}-(CH_2)_3-$ | $CH_3-CH_2-$ | 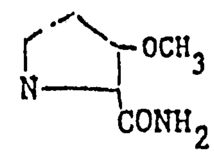 |

Table II (cont)

| pound No. | $R_8$ | $R_1$ | $R_3$ | 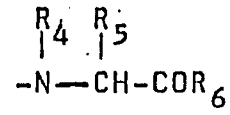 |
|---|---|---|---|---|
| 64 | H | 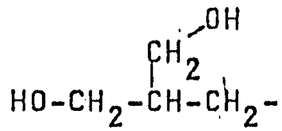 | $(CH_3)_2CH_2-$ | 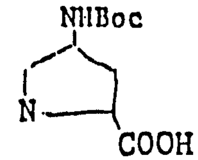 |
| 65 | H | 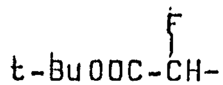 | $CH_3-$ | 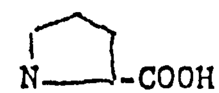 |
| 66 | H | 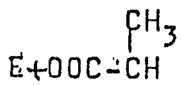 | 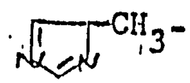 | 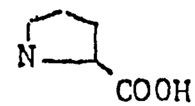 |

Table
-2-

| Compound No. | $R_8$ | $R_1$ | $R_3$ | $-\overset{R_4}{N}\ \overset{R_5}{CH}-COR_6$ |
|---|---|---|---|---|
| 7 | H | $MeOOC-\overset{CH_2}{\overset{\|\|}{C}}-$ | 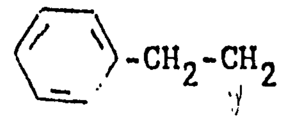 | 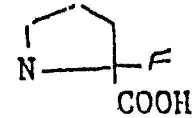 |
| 8 | H | 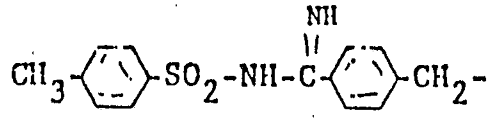 | $(CH_3)_2CH_2$ | 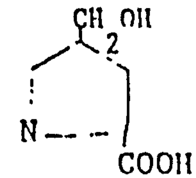 |
| 9 | H | 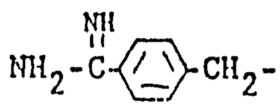 | 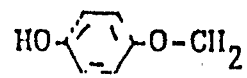 | 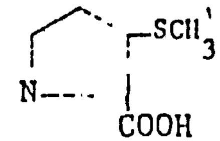 |
| 10 | H | 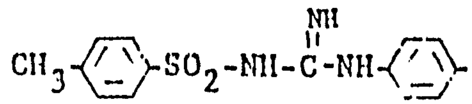 | 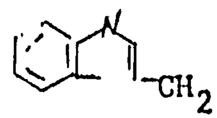 | 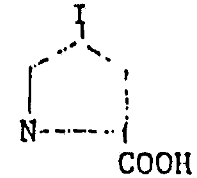 |
| 11 | H | 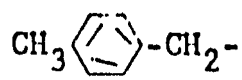 | 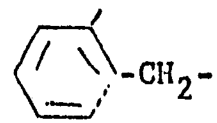 | 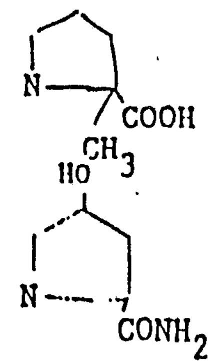 |
| 12 | H | 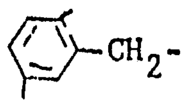 | $HOOC-CH_2-CH_2-$ | |

| Compound No. | $R_8$ | $R_1$ | $R_3$ | $\overset{R_4}{-N}-\overset{R_5}{CH}-COR_6$ |
|---|---|---|---|---|
| 73 | H | 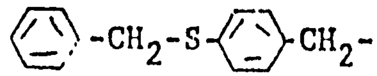 | $CH_3-\overset{OH}{CH}-CH_2-$ | 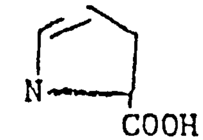 |
| 74 | H | 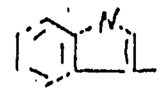 | $H_3C-S-CH_2-CH_2-$ | 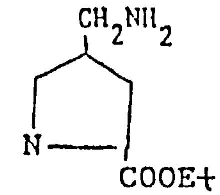 |
| 75 | $CH_3$ | $(CH_3)_3COOC-CH_2-CH_2-$ | $CH_3-$ | 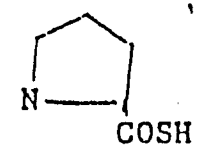 |
| 76 | $CH_3$ | $HOOC-CH_2-CH_2-$ | $CH_3-CH_2-$ | 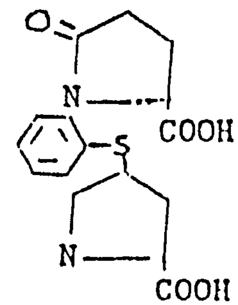 |
| 77 | $CH_3$ | $H_2N-\overset{NH}{\overset{\Vert}{C}}-NH-(CH_2)_3-$ | 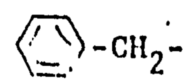 | |
| 78 | H | 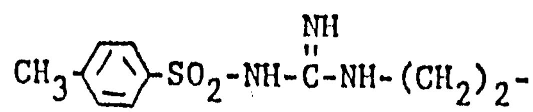 | $(CH_3)_2CH-$ | 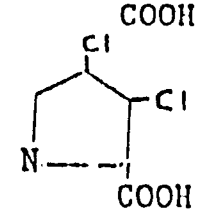 |
| 79 | H | $NH_2-CH_2-O-(CH_2)_2-$ | $CH_3-$ | 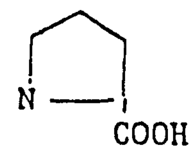 |

Table -4-

| Compound No. | $R_8$ | $R_1$ | $R_3$ | $R_4$ $R_5$ $-N—CH-COR_6$; |
|---|---|---|---|---|
| 80 | H | $H_2NCONH-CH_2-$ | $CH_3$ | 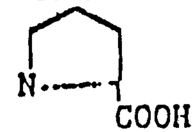 |
| 81 | - | 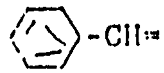 | $(CH_3)_2CH_2-$ | 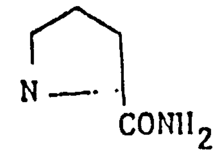 |
| 82 | - | $(CH_3)_2C=$ | 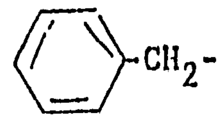 | 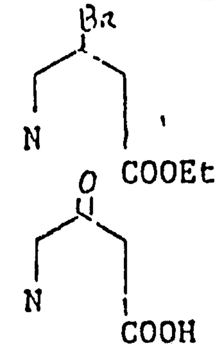 |
| 83 | - | $(CH_3CH_2)_2C=$ | $CH_3$ | |
| 84 | - | 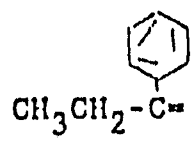 | $CH_3$ | 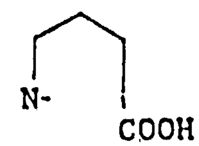 |
| 85 | - | 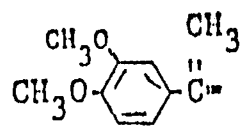 | $CH_3$ | 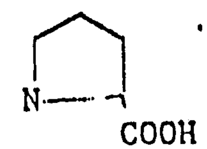 |
| 86 | H | 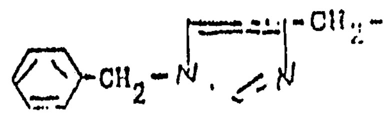 | 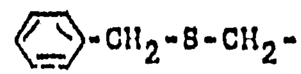 | 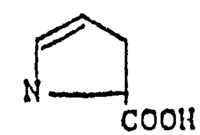 |

Table -5-

| Compound No. | $R_8$ | $R_1$ | $R_3$ | $-\overset{R_4}{\overset{\mid}{N}}-\overset{R_5}{\overset{\mid}{C}H}-COR_6$ |
|---|---|---|---|---|
| 87 | H | 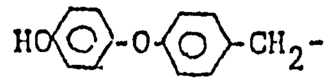 | 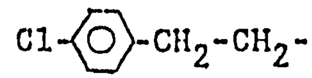 | 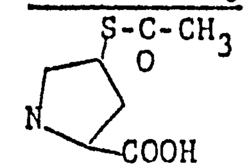 |
| 88 | H | $EtOOC-CH_2-\overset{O}{\overset{\|}{C}}-CH_2-$ | $CH_3$ | 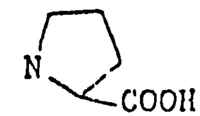 |
| 89 | H | $EtOOC-CH_2-\overset{O}{\overset{\|}{C}}-CH_2-$ | H | 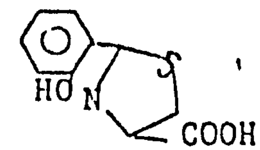 |
| 90 | H | $(CH_3)_3C-O-\overset{O}{\overset{\|}{C}}-CH_2-\overset{O}{\overset{\|}{C}}-CH_2-$ | $CH_3$ | 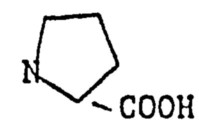 |
| 91 | H | $(CH_3)_3C-O-\overset{O}{\overset{\|}{C}}-NH-(CH_2)_2-\overset{O}{\overset{\|}{C}}-$ | $CH_3$ | 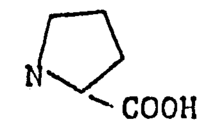 |
| 92 | H | $(CH_3)_3C-O-\overset{O}{\overset{\|}{C}}-NH-CH_2-\overset{O}{\overset{\|}{C}}-$ | $CH_3$ | 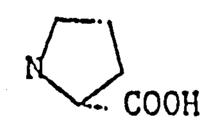 |
| 93 | H | $CH_3-\overset{O}{\overset{\|}{C}}-CH_2-$ | $CH_3$ | 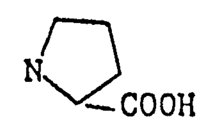 |
| 94 | H | $HO-CH_2-\overset{O}{\overset{\|}{C}}-CH_2-$ | $CH_3$ | 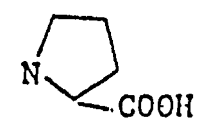 |

Table
-6-

| Compound No. | $R_8$ | $R_1$ | $R_3$ | $\overset{R_4}{-N}—\overset{R_5}{CH}-COR_6$ |
|---|---|---|---|---|
| 95 | H | $(CH_3)_3C-O-\overset{O}{C}-CH_2-\overset{O}{C}-CH_2-$ | H | 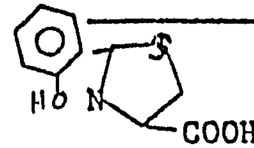 |
| 96 | H | 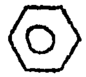 $O-CH_2-S-CH_2-\overset{O}{C}-CH_2-$ | $CH_3$ | 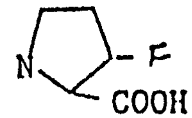 |
| 97 | H | $CH_3-\overset{O}{C}-\underset{CH_3}{CH}-$ | $CH_3$ | 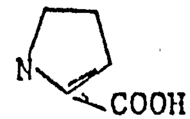 |
| 98 | $CH_3$ | $CH_3-\overset{O}{C}-CH_2-$ | $CH_3$ | 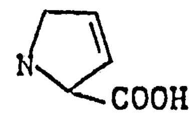 |
| 99 | $CH_3$ | 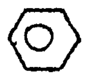 $—CH_2-S-CH_2-\overset{O}{C}-CH_2-CH_2-$ | $CH_3$ | 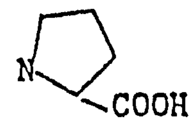 |
| 100 | $CH_3$ | $CH_3-\overset{OH}{CH}-CH_2-$ | $CH_3$ | 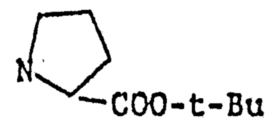 |
| 101 | H | $(CH_3)_3C-O-\overset{O}{C}-NH-CH_2-CH_2-\overset{OH}{CH}-$ | $CH_3$ | 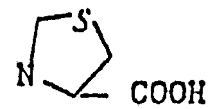 |

| Compound No. | $R_8$ | $R_1$ | $R_3$ | $-N(R_4)-CH(R_5)-COR_6$ |
|---|---|---|---|---|
| 102 | $CH_3$- | $EtOOC-CH_2-CH(OH)-CH_2-$ | 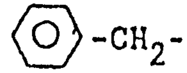 | 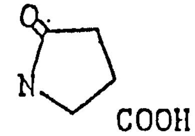 |
| 103 | $CH_3$- | $CH_3-CH_2-CH(OCH_3)-$ | 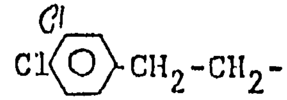 | 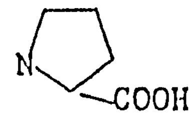 |
| 104 | H- | 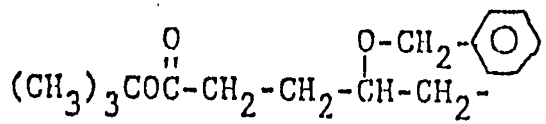 | $CH_3$ | 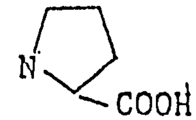 |
| 105 | H- | 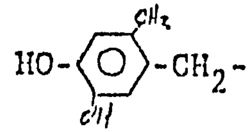 | $CH_3$ | 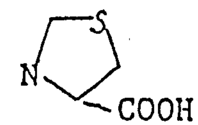 |
| 106 | H- | 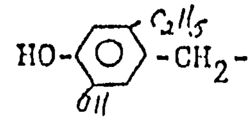 | $CH_3$ | 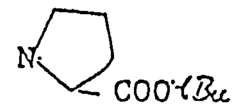 |
| 107 | H- | 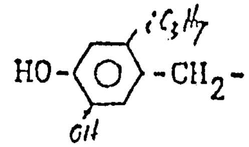 | 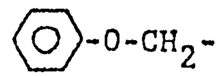 | 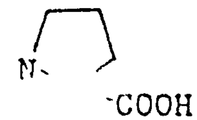 |
| 108 | H- | 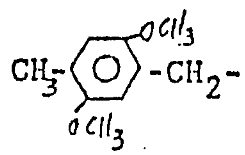 | $CH_3$ | 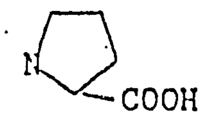 |

Table
-8-

| Compound No. | $R_8$ | $R_1$ | $R_3$ | $\overset{R_4}{\underset{\vert}{-N}}-\overset{R_5}{\underset{\vert}{CH}}-COR_6$ |
| --- | --- | --- | --- | --- |
| 109 | $CH_3-$ | 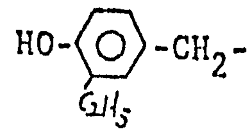 | $CH_3-$ | 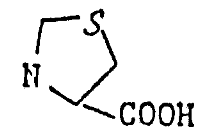 |
| 110 | $CH_3-$ | 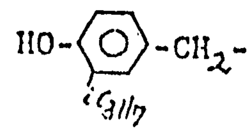 | $CH_3-$ | 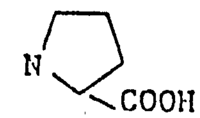 |
| 11 | $CH_3-$ | 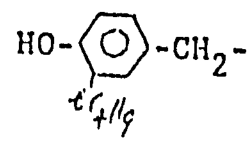 | $CH_3-$ | 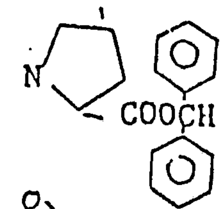 |
| 12 | H- | 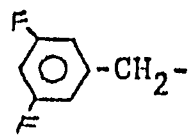 | $H_2N-(CH_2)_3CH_2-$ | 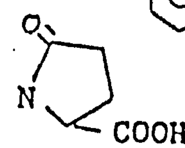 |
| 3 | H- | 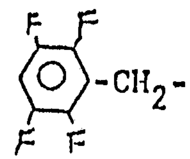 | $CH_3-$ | 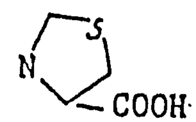 |

| Compound No. | $R_8$ | $R_1$ | $R_3$ | $-N(R_4)-CH(R_5)-COR_6$ |
|---|---|---|---|---|
| 114 | H | 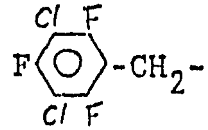 | $CH_3$- | 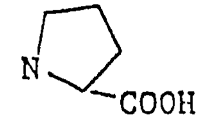 |
| 115 | H | 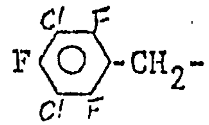 | $CH_3$- | 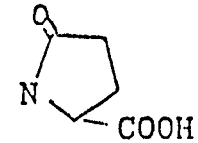 |
| 116 | H | 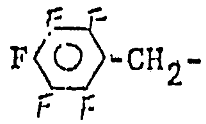 | $CH_3$- | 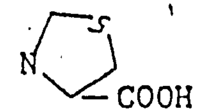 |
| 117 | H | 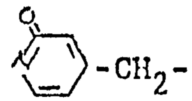 | 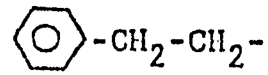 | 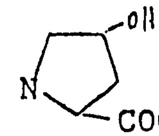 |
| 118 | H | 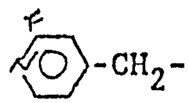 | 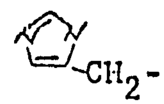 | 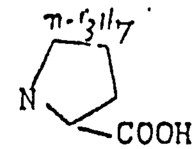 |
| 119 | H | 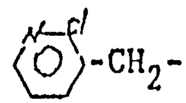 | 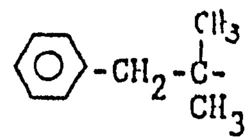 | 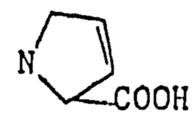 |



0048159

Table
-10-

| Compound No. | $R_8$ | $R_1$ | $R_3$ | $-N(R_4)--CH(R_5)-COR_6$ |
|---|---|---|---|---|
| 120 | H | 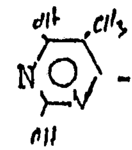 $-CH_2-$ | $HO-CH_2-$ | 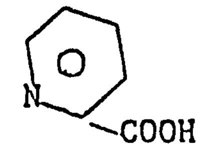 |
| 121 | H | 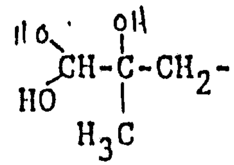 | $(CH_3)_2CH$ | 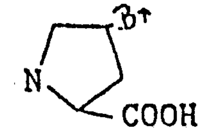 |
| 122 | $CH_3$ | $CH_3CH_2-O-CH_2-CH_2-$ | $H_2-N-C(=NH)-N(H)-(CH_2)_3-$ | 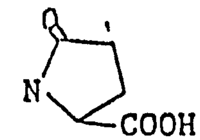 |
| 123 | H | 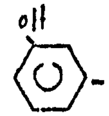 - | $H_2N-(CH_2)_4-$ | 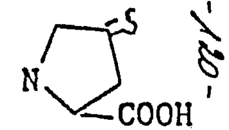 |
| 124 | H | 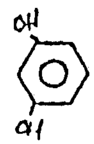 | $(CH_3)_2-NH-(CH_2)_4-$ | 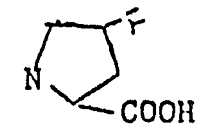 |
| 125 | H | 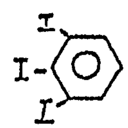 $-CH_2-$ | $(CH_3)_2-CH-$ | 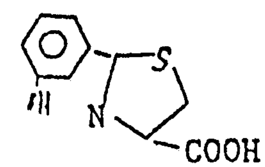 |
| 126 | $CH_3$ | $CH_3O-$ 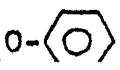 $-CH_2-$ | $CH_3-$ | 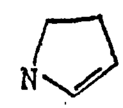 |

Example 707

A. Synthesis of N-(1-ethoxycarbonyl-1-hydroxymethyl ethyl)-D,L-Ala-Pro-t-butyl ester.

Using the method of Example 439, the named compound is prepared from -hydroxymethyl-Ala-ethyl ester and pyruvoyl-L-Pro-t-Bu-ester.

B. Synthesis of N-(1-ethoxycarbonyl-2 mercaptomethylethyl)-D,L-Ala-Pro

The desired compound is obtained by treating the product of Step A with $P_2S_5$ and then removing the t-butyl ester group with HCl.

Example 708

Synthesis of L-2-[L-1-carboxy-4-(N -methyl, N -N-methyl,N-nitroguanidino) butyl-thiopropanoyl]-L-4-butyloxy-L-Pro-t-butyl ester

A. A solution of D-(+)-2-bromopropanoic acid(0.04 mols) in 15 ml. of methylene dichloride was cooled to -40°C. A solution of DCC (0.04 mmols) in 10 ml. of methylene dichloride was added in dropwise fashion and the solution was stirred at -35 to -40°C. for 10 minutes after which there was added L-Pro-t-butyl ester (0.04 mols) in 5 ml. of methylene dichloride at -45°C. The reaction mixture was stirred at -40°C. for 1-1/2 hours and then at 4°C. overnight. Working up the product in the conventional manner yielded 10.6 g. of the named product.

B. About 20 ml. of anhydrous liquid ammonia is added to L-2-mercapto-5-N -methyl, N -nitroguanidino-pentanoic acid (0.003 mols) while the reaction flask is immersed in a dry ice-acetone bath. Sodium is added with stirring until a permanent blue color is sustained. There is then added with stirring N-(+)-D-bromo-propanoyl)-L-4-t-butyloxy-L-Pro t-butyl ester (0.03 moles) from Step A hereof. The reaction vessel is stoppered and stirred at room temperature for 1-1/2 hours. Ammonia is removed while the flask is protected with a tube containing $CaCl_2$. The residue is dissolved in water and then extracted with ethyl acetate. The aqueous phase is saturated and then acidified with solid potassium bisulfate in the presence of ethyl acetate. The solvent is removed with a rotary evaporator to yield the named compound.

0048159

Example 709

Synthesis of L-2-[L-1-thiolcarbonyl-4-(N'-methyl,N'-nitroguanidino)- butyl thiopropanoyl]-L-4-t-butyloxy-Lproline-t-butyl ester

A solution of product from Example 708 (0.0015 mols) and triethylamine (0.0015 mols) in dry tetrahydrofuran (50 ml.) is cooled to -15°C. under argon and treated with ethyl chloroformate (0.015 mols). After stirring at -15°C. for 1-1/2 hours, sodium hydrosulfide (0.025 mols) is added and the solution is stirred at 4°C. overnight. The solvent is removed with a rotary evaporator and the solid is dissolved in water and cooled in an ice bath. This solution is acidified to about pH3 with solid potassium bisulfate in the presence of ethyl acetate and the organic phase is washed with a solution of saturated sodium chloride, dried over anhydrous sodium sulfide and filtered. The solvent is removed with a rotary evaporator to yield the named compound.

Example 710

Synthesis of L-2-[L-1-thiolcarbonyl-4-(N'-methyl-guanidino-butyl-thio-propanoyl]-L-4-hydroxy-L-Pro

The product of Example 709 is subjected to HF treatment at 0 C. in the presence of anisole for 1 hour to yield the named product.

Example 711

Synthesis of N-(1,2-dicarboxy-2-fluoroethylthio)-2-phenylacetyl-L-thiazolidine-4-carboxylic acid

A. To a solution of 0.01 mol of thiazolidine-4-carboxylic acid in 10 ml.of 1N NaOH, cooled in an ice bath, is added 5 ml. of 2 N NaOH and 0.01 mol of D-2-bromo-2phenylacetyl chloride, in that order, with vigorous stirring. The reaction mixture is then stirred at 0°C. for 30 minutes and then at room temperature for 1 hour. It is then acidified with concentrated HCl in the presence of ethyl acetate to about pH 2, with cooling. The organic phase is washed with a solution of saturated NaCl, dried over anhydrous sodium sulfate and filtered. The solvent is removed with a rotary evaporator to yield the named compound.

B. Using the synthesis method of Example 708, Step B, 1-mercapto-2-fluorosuccinic acid is reacted with the product of

Example 712

Synthesis of N-[2-(1-carboxy-2-p-nitrophenyl)ethylthio-5-Boc-aminopentanoyl]-L-Pro-t-butyl ester

A. This compound is prepared in the manner described in Example 708, Step A, using 2-bromo-5-Boc-aminopentanoic acid as the starting compound.

B. This compound is then prepared in the manner described in Step B of Example 708, using mercapto-3-p-nitrophenyl propionic acid and the product of Step A hereof as the reactants.

Example 713

Synthesis of N-[2-(1-carboxy-2-p-nitrophenyl ethylthio)-5-guanidinopentanoyl]-L-Pro-t-butyl ester

The product of Example 712 is deprotected with anhydrous trifluoroacetic acid in the presence of anisole and then treated with anhydrous ethyl ether. The residue is dried in a vacuum desiccator over phosphorus pentoxide and potassium hydroxide for several hours and the guanidino group is introduced with o-methyl isourea.

Example 714

Synthesis of N-[2-(1,3-dicarboxy-1-methyl) propylthiopropanoyl)-2-O-hydroxphenyl-L-thiazolidine-4-carboxylic acid

A. 2-(O-hydroxphenyl)-L-thiazolidine-4-carboxylic acid is prepared in the manner described in step A of Example 711, using 2(O-hydroxphenyl)-L-thiazolidine-4-carboxylic acid and 2-bromo-propionyl chloride as the reactants.

B. The desired compound is prepared in the manner described in Example 708, Step B, using 2-mercapto-2-methyl glutaric acid and the product of Step A hereof as the reactants.

---

Example 715

Synthesis of N[2-(4-amino-1-carboxy-2-hydroxybutylthio)butanoyl]-L-4-methoxy-proline

A. N-(2-bromobutanoyl)-L-4-methoxyproline-t-butyl ester is prepared as described in Step A of Example 708, substituting 2-bromobutyric acid and L-4-methoxy-proline-t-butyl ester for the reactants of that step.

B. The desired compound is prepared as described in Step B of Example 708, substituting 5-Boc-amino-3-t-butyloxy-2-mercapto-pentanoic acid-t-butyl ester and the product of Step A hereof for the reactants there utilized, followed by treatment with trifluoroacetic acid in the presence of anisole to yield the named product.

Example 716

Synthesis of N-[L-2-(2-benzyloxy-1-carboxy-ethylthio)-2-p-t butyloxy-phenylacetyl]-L-proline

A. N-[2-bromo-(p-butyloxy)phenylacetyl]-L-proline t-butyl ester is prepared as described in step A of Example 708, using 2-bromo-p-t-butyloxyphenyl acetic acid as the starting material.

B. The sodium salt of 3-benzyloxy-2-mercaptopropionic acid-t-butyl ester (0.01 mol) and the product of Step A hereof (0.01 mol) are reacted according to Step B, Example 708, to yield the Pro t-butyl ester of the named compound. The ester is deprotected with trifluoroacetic acid in the presence of anisole to yield the named compound *per se*.

Example 717

Synthesis of N-[2-(5-amino-1-carboxypentylthio)-3-(2-ethoxy-5-nitrophenyl)propanoyl]-L-Pro

Following the procedure described in Step B of Example 715, 6-Boc-amino-2-mercaptohexanoic acid and N-[2-bromo-3-(2-ethoxy-5-nitrophenyl)propanoyl]-L-Pro-t-butyl ester are reacted to give the proline-t-butyl ester of the desired compound. That ester is then treated with trifluoroacetic acid in the presence of anisole to give the named compound.

Example 718

Synthesis of N-[2-(1-carboxy-3-phenylpropyl)-4-oxo-pentanoyl]-L-3,4-dehydroproline

Using the procedure described for Step B of Example 715, 4-phenyl-2-mercapto-butanoic acid is reacted with N-(2-bromo-4-oxopentanoyl)-3,4-dehydro-proline-t-butyl ester, and the product is deprotected with trifluoroacetic acid in the presence of anisole to yield the named compound.

Example 719

Synthesis of N-[2-1-ethoxycarbonyl-2-(p-Tos-amidino)phenylethoxy propanoyl]-L-proline

Sodium (0.005 gram-atoms) was added to a solution of the ethyl ester of 2-hydroxy-3-(p-Tos-amidino)-phenylpropanoic acid (0.005 mols) in 5 ml. of dry toluene, fitted with a reflux condenser and the flask protected with a calcium chloride tube. The mixture is stirred at room temperature for 1 hour. To this is added a solution of 0.005 ml. of N-(2-bromo-propanoyl)-2-proline t-butyl ester and the mixture is stirred at room temperature for 5 hours and then refluxed for another 20 hours. The mixture is then cooled to room temperature and the precipitated

sodium bromide is removed by filtration. Ethyl acetate is then added to the organic phase, which is then washed with water. It is then dried over anhydrous sodium sulfate, filtered and the solvents are removed with a rotary evaporator. The protecting group, t-butyl ester, is then removed by treatment with trifluoroacetic acid in the presence of anisole to yield the desired product.

Example 720

Synthesis of N-[2-(1-carboxy-3-phenylpropyloxy)-3-pentafluorophenyl]-propanoyl]-L-proline

Using the procedure just described in Example 719, the named compound is prepared from the reactants 2-hydroxy-4-phenyl-butanoic-t-butyl ester and 2-bromo-3-pentafluorophenylpropanoyl-L-proline-t-butylester.

Example 721

Synthesis of N-[3-(1-carboxyethylthio)-2-D-methylpropanoyl]-L-proline.

0.25 mmoles of 3-mercapto-2-D-methylpropanoyl-L-proline, 0.28 mmoles of 2-bromopropanoic acid and 0.16 mmole of $K_2CO_3$ were added to 0.6 ml. of a 50:50 mixture of absolute ethanol and water. The suspension was stirred overnight at room temperature. 0.25 mmoles of $K_2CO_3$ in 0.15 ml of water was then added and the reaction mixture was stirred for an additional 24 hours. This mixture was then acidified to pH 2.0 with HCl and the product was extracted with ethyl acetate. The organic phase was washed with saturated NaCl. The product appearedto be pure and behaved as a single substance on thin layer chromatography in two separate solvent systems. The ethyl acetate phase was dried over anhydrous $MgSO_4$ and the solvent was removed with a rotary evaporator to yield the named compound as a colorless oil.

Example 722

Synthesis of N-(1-thiocarbonyl-3-phenylpropyl)-L-alanyl-L-proline

A solution of 0.15 mmoles of 2-keto-4-phenylbutyric acid plus 0.15 mmoles of thiophenol in redistilled chloroform is cooled to -50°C in an acetone-dry ice bath. To this solution is added 0.15 mmoles of a precooled solution of dicyclohexylcarbodiimide (DCC) in chloroform and the mixture is stirred at -50°C for 1 hour. The reaction mixture is slowly warmed to room temperature and stirred for an additional 2 hours and then stirred at 4°C overnight. The mixture is

filtered to remove dicyclohexylurea, then cooled in an ice bath. The organic phase is washed with cold water, cold 1N $NaHCO_3$ and finally with cold saturated NaCl. The organic phase is dried over anhydrous $MgSO_4$ and filtered. The solvent is removed with a rotary evaporator yielding the thiophenyl ester of 2-keto-4-phenylbutyric acid.

A solution of 40 mmoles of L-Ala-L-Pro and 200 mmoles of the product above in absolute ethanol is stirred with powdered molecular sieves at room temperature for 1/2 hour. A solution of 40 mmoles of sodium cyanoborohydride in ethanol is then slowly added over the course of six hours. The reaction mixture is filtered and the solvent removed with a rotary evaporator to yield the named product. The thio acid may be formed by removing the thiophenyl group of the thiophenyl ester with NaSH.

Example 723

Synthesis of N-[3-(1-carboxyl-3-methylpentylthio)-2-methylpropanoyl]-L-proline

2-mercapto-4-methylhexanoic acid ethyl ester is prepared from the corresponding 2-hydroxy compound according to Erlenmeyer (Ber. 36) 2720 (1903).

20 mmoles of methacryloyl chloride is added with vigorous stirring to a solution of 20 mmoles of L-proline in a mixture of water and sodium bicarbonate chilled in an ice water bath. The mixture is then stirred at room temperature for 2 hours and then extracted with ether. The aqueous phase is acidified with 1NHCl and extracted with ethyl acetate. The organic phase is concentrated to dryness in vacuo. A suspension of 10 mmoles of the residue and 10 mmoles of 2-mercapto-4-methylhexanoic acid ethyl ester in toluene is refluxed for several hours until the reaction is essentially completed as indicated by thin layer chromatography. The mixture is stored overnight at room

temperature. Solvent is removed with a rotary evaporator yielding the ethyl ester of the named product. If the free acid is desired, the ester product is saponified with NaOH to form the sodium salt of the named product. The named product is obtained by acidifying the sodium salt with one equivalent of HCl.

Example 724

Synthesis of N-[2-(1-carboxyl-2-phenylethylthio)-3-phenylpropanoyl]-L-proline

Phenylalanine (8g.) in 110 ml. of 48% HBr at -5°C is reacted with a cold solution of 10 g. $NaNO_2$ in 20 ml. of $H_2O$ at 5°C for 30 minutes substantially as described by Yankeelov and Jolley (Biochemistry 11, 159 (1972)) to yield the α-Br derivative. That is, Br replaces the amino group. The product (15 mmoles) is reacted with 15 mmoles of the ethyl ester of 2-mercapto-3-phenylpropanoic acid in a mixture of ethanol and water containing $K_2CO_3$. The mixture is stirred until the reaction is complete as judged by thin layer chromatography. The solvent is removed with a rotary evaporator. 15 mmoles of this product and 15 mmoles of N-hydroxysuccinimide is dissolved in dimethylformamide (DMF) and cooled to 0°C in an ice-acetone bath. 15 mmoles of di-cyclocarbodiimide (DCC) in DMF is added dropwise and the mixture stirred at 0°C. for 30 minutes and at 4°C. overnight. Crystalline dicyclohexylurea is removed by filtration. The solvent from the filtrate is removed under reduced pressure. The resulting product is dissolved in cold tetrahydrofuran (THF) and then this solution is added to a cold solution of 15 mmoles of L-Pro and 15 mmoles of $NaHCO_3$ in THF/water. The reaction mixture is stirred overnight at room temperature and then the THF is removed with a rotary evaporator. The ethyl ester is removed by saponification with NaOH to form the sodium salt and the named free acid is formed by acidification with HCl.

Using an alternative method, the L,3,4-dehydroproline analog of this product is made. In this method, the ethyl ester of α-Br-3-phenylpropanoic acid is reacted with 2-mercapto-3-phenylpropanoic acid. The resulting compound is converted into its N-hydroxysuccinimide active ester as described above and then coupled to L-3,4-dehydroproline, or the resulting compound is coupled with DCC to L-3,4-dehydroproline ethyl ester. In either case, the ethyl ester protection is removed by saponification.

Example 725

Synthesis of 2-[(1-carboxy)-propyloxy]-5-aminohexanoyl-L-thioproline

5 mmoles of the compound $\text{Pht-NH-}(CH_2)_4\text{-CH(OH)-}\overset{\text{O}}{\overset{\|}{\text{C}}}\text{-OH}$ is dissolved in ethyl acetate and cooled to 0°C in an ice-acetone bath. 5 mmoles of DCC in EtOAc is added followed by the addition of 5 mmoles of L-thioproline ethyl ester in EtOAc. The reaction mixture is stirred overnight at 4°C. The reaction mixture is worked-up by the addition of ethyl acetate and filtration. The filtrate is washed with saturated NaCl, 0.1N $NaHCO_3$ and finally saturated NaCl. The organic phase is dried over anhydrous $MgSO_4$ and the solvent removed by a rotary evaporator to yield

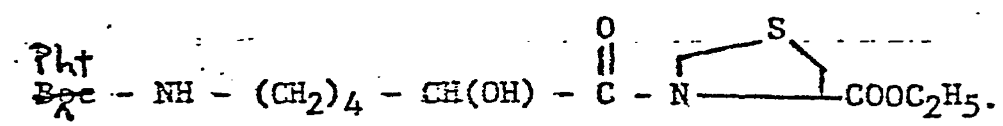

5 mmoles of the ethyl ester of 2-Br-butyric acid is added, plus 1 equivalent of sodium to produce the ether compound. The Pht group is removed by hydrazinolysis. The ethyl esters are removed by saponification with NaOH and free acid formed by acidification with HCl. The named product is obtained by evaporation of solvent or crystallization followed by filtration or decantation.

Example 726

Synthesis of 3-N-(1-carboxy-2-phenylethyl)-aminopropanoyl-L-5-keto-proline

60 mmoles of 3-N-(benzyloxycarbonyl)-aminopropanoic acid is dissolved in $CH_2Cl_2$ at 0°C. 20 mmoles of N-hydroxysuccinimide is added and then 20 mmoles of DCC is added dropwise to this mixture. The reaction mixture is stirred for 30 minutes at 0°C and then overnight at 4°C. Crystalline dicyclohexylurea is removed by filtration. The solvent from the filtrate is removed under reduced pressure. The resulting product is dissolved in cold THF and then the solution is added to a cold solution of 20 mmoles of L-glutamic acid and 40 mmoles of $NaHCO_3$ in THF/water. The reaction mixture is stirred overnight at room temperature and then the THF is removed with a rotary evaporator. The glutamyl residue is cyclized to give the L-5-keto-proline residue according to Gibian H. and Klieger E., Justus Liebig's Ann. Chemie 640, 145 (1961). The benzyloxycarbonyl group is removed by treatment with hydrogenolysis.

A solution of 40 mmoles of this product and 200 mmoles of 2-keto-3-phenylpropanoic acid t-butyl ester in ethanol is stirred with powdered molecular sieves at room temperature for 1/2 hour. A solution of 40 mmoles of sodium cyanoborohydride in ethanol is slowly added over the course of six hours. The reaction mixture is filtered. The t-butyl ester is removed by treatment with TFA in anisole. The named product is obtained after removal of the solvent with a rotary evaporator.

Example 727

Synthesis of N-[2-(1-carboxyethylthio)-propanoyl]-L-3-hydroxyproline

Into 200 mmoles of 2-bromopropionic acid dissolved in 350 ml. of dry dichloromethane plus 2 ml. concd. $H_2SO_4$ is bubbled a stream of 2-methylpropene at room temperature for 1½ hours, and the sealed vessel is stored overnight, to form the t-butyl ester. The volume of the solvent

is reduced by two-thirds under reduced pressure. The residual solution is diluted in ethyl ether and the organic phase is then washed successively with 1N sodium bicarbonate, and saturated sodium chloride. The organic phase is dried over magnesium sulfate and the solution is filtered and the solvent is removed at reduced pressure. The t-butyl ester is then purified by vacuum distillation. 10 mmoles of the product is reacted with 10 mmoles of thiolactic acid in a mixture of ethanol and water containing $K_2CO_3$. The mixture is stirred until the reaction is complete as judged by thin layer chromatography. The solvent is removed with a rotary evaporator. The residue is dissolved in water and washed with ethyl ether. To the aqueous solution is added 25 ml. of ethyl ether and 5 ml. of ethyl acetate. The mixture is cooled in an ice bath and then acidified with concd. HCl. The organic phase is separated and washed twice with saturated sodium chloride and then dried over anhydrous $MgSO_4$ and filtered. Solvent is thereupon removed with a rotary evaporator. 5 mmoles each of the product and N-hydroxysuccinimide are dissolved in $CH_2Cl_2$/DMF (7:2 by volume) and cooled to -4°C in an ice-acetone bath. 5 mmoles of DCC in dichloromethane are added dropwise and the mixture stirred at -4°C for 30 minutes and at +4°C overnight. Crystalline dicyclohexylurea is removed by filtration, and the solvent is removed from the filtrate under reduced pressure. The residue is dissolved in EtOAc, washed until neutral and recovered from the solvent. The resulting product is dissolved in THF and water (1:1) at room temperature and then this solution is added dropwise to 5 mmoles each of L-3-hydroxyproline and $NaHCO_3$ in THF/water (1:1) which has been prepared by dissolving L-3-hydroxyproline in THF, added dropwise, followed by addition of $H_2O$ and $NaHCO_3$. The reaction mixture is stirred overnight at room temperture and then the THF is removed

with a rotary evaporator. The t-butyl ester is removed by treatment with TFA in anisole to yield the named product.

Example 728

Synthesis of N-(2-mercaptoisopropyl)-L-alanyl-L-proline

60 mmoles of pyruvic acid is coupled to 60 mmoles of L-proline t-butyl ester using 60 mmoles of DCC as described in Example 722 to yield N-pyruvoyl-L-proline ethyl ester. 40 mmoles of this product is reacted with 200 mmoles of 2-amino-1-propanol in ethanol with stirring in the presence of molecular sieves at room temperature. A solution of 40 mmoles of sodium cyanoborohydride in ethanol is then slowly added over the course of 6 hours. The reaction mixture is filtered and the solvent removed by a rotary evaporator. The product is purified by partition chromatography (Sephadex G-25 developed with butanol/acetic acid/$H_2O$(4:1:5). 20 mmoles of this product is converted to the corresponding mercapto compound as described in Examples 723. The ethyl ester is removed by saponification to yield the named product.

Example 729

Synthesis of 2-[1-carboxy-2-phenylethoxy] propanoyl-L-proline

5 mmoles of 2-hydroxyl-3-phenylpropanoic acid ethyl ester and 5 mmoles of 2-bromopropanoic acid t-butyl ester are reacted in the presence of 1 equivalent of sodium to form the product 2-[(1-ethoxycarbonyl-2-phenyl)-ethoxy]propanoic acid t-butyl ester. The t-butyl ester is removed by treatment with TFA in anisole. The resulting product (5 mmoles) is dissolved in DMF and reacted with 5 mmoles of L-proline in the presence of 5 mmoles of DCC as described in Example 725. The ethyl ester is removed by saponification with ethanolic KOH and the acid formed by acidification with HCl yielding the named product.

Example 730

N-[2-(tetrahydro-5-methyl-2-furanone-3-thio-propanoyl]-L-proline

A solution of 1.52 ml. (0.011 mole) of anhydrous $K_2CO_3$ in 3 ml. of water was added dropwise to a solution of 1.88 grams (0.0105 mole) of α-bromo-γ-valerolactone and 1.06 gm (0.01 moles) of thiolactic acid in 3 ml. of absolute alcohol with stirring and cooling in an ice-acetone bath. The cooling bath was removed 5 minutes after complete addition of the bicarbonate solution. The mixture was stirred at room temperature overnight and 20 ml. of water was added. It was then washed 3 times with diethyl ether, cooled in an ice bath, acidified with 1.0 ml. of concentrated HCl and extracted three times with 10 ml. of diethyl ether. The combined organic phase was washed 3 times with aqueous saturated NaCl, dried over anhydrous $MgSO_4$ and filtered. Solvent was removed with a rotary evaporator. The product was a light yellow oily residue in a yield of 1.77 gm.

It showed, upon infrared examination in chloroform, a strong, broad carbonyl band of the COOH group at 1720 $cm^{-1}$, a very strong, sharp carbonyl band characteristic of lactone at 1768 $cm^{-1}$, a strong, sharp, lactone ester band at 1178 $cm^{-1}$ and there was also absorption between 2400 and 2800 $cm^{-1}$.

The crude product was dissolved in 4 ml. of dichloroethane with cooling in an ice-acetone bath. There was added 0.997 grams (.00087 moles) of hydroxy succinimide in 2 ml. of dimethylformamide with continued cooling, followed by addition of 1.969 grams (.0087 moles and 10% excess) of dicyclohexylcarbodiimide dissolved in 4 ml. of dichloroethane. The mixture was stirred in the ice-acetone bath for 30 minutes and then stirred at 4°C overnight. Dicyclohexylurea (1.78g.) was removed by filtration and washed with ethyl acetate (20 ml.) and diethyl ether (10 ml.). The remaining organic phase was washed twice with 1N sodium bicarbonate and twice with saturated NaCl and dried

over anhydrous $MgSO_4$ and filtered. The solvent was then removed under high vacuum to give 2.62 g. of a light, oily residue.

450 mg. (1.5 mmoles) of the crude oily residue in 1 ml. of dioxane was added to a solution of 1.72 mg. (0.55 mmoles) of L-proline and 146 mg. (1.5 mmoles) of $NaHCO_3$ in 1 ml. of water and the mixture was stirred at room temperature for 30 minutes. 0.5 ml. of dioxane was then added and the mixture was stirred at room temperature overnight. The reaction being then incomplete, 2 ml. of dioxane and 1 ml. of water were added and the mixture was stirred for an additional 24 hours. 10 ml. of water and 1 ml. of 1N sodium bicarbonate were then added and the mixture was extracted four times with 2 ml. of diethyl ether. 10 ml. of ethyl acetate was then added and the mixture cooled in an ice bath and acidified with concentrated HCl to pH2. The aqueous phase was extracted three times with 5 ml. of water and the combined organic phase was washed 3 times with 1 ml. water and twice with saturated NaCl solution. The organic phase was dried over anhydrous $MgSO_4$ and filtered and the solvent removed under high vacuum yielding 313 mg. of a thick oily residue. After purification by Sephadex LH-20 column chromatography (1.2 x 97.5 cm.) and repurification by the same system the product in chloroform solution was examined by infrared. It retained the strong, sharp ester band of lactone at 1181 $cm^{-1}$, showed a new strong, sharp amide carbonyl band at 1640 $cm^{-1}$, the carbonyl band of the carboxyl group appeared as a shoulder at 1722 $cm^{-1}$, a very strong, sharp carbonyl band of lactone appeared at 1760 $cm^{-1}$ and COOH absorption appeared between 2400 and 2800 $cm^{-1}$. The product was subjected also to paper electrophoresis at pH2 and 5 and to thin layer chromatography in 4 systems

Example 731

N-2-(1-carboxyl-3-hydroxybutylthio)-propanoyl-L-proline

The product of Example 730 (0.5 mmole) is contacted with a molar excess (0.8 mmole) ethanolic KOH for 3 hours. Ethanol is removed with a rotary evaporator, 5 ml. of ethyl acetate and 1 ml. of water is added and the mixture is cooled. It is then acidified with concentrated HCl to pH2, washed twice with water and 3 times with saturated salt solution, dried over anhydrous $MgSO_4$, and filtered. The solvent is then removed with a rotary evaporator and the product is then subjected to thin layer chromatography.

Example 732

N-2-(1-carboxyl-3-acetyloxybutylthio)-propanoyl-L-proline

The product of Example 731 (0.5 m. mole) is dissolved in 1 ml. of pyridine and reacted with acetic anhydride (0.75) moles. Completion of the reaction is determined by thin layer chromatography.

Example 733

Synthesis of N-[(1-carboxy-2-benzyloxy)ethyl]-alanyl-L-proline

N-pyruvoyl-L-proline, 50 mmoles, (prepared as described in Example 728 is reacted with 10 mmoles of the ethyl ester of O-benzyl-serine in ethanol with stirring in the presence of molecular sieves at room temperature. A solution of 10 mmoles of sodium cyanoborohydride in ethanol is added slowly over 5 hours. The reaction mixture is filtered and the solvent of the filtrate removed by a rotary evaporator. The product is purified by conventional column chromatography techniques. The ethyl ester group is removed by saponification to yield the named compound.

Examples 734-751

By substituting the ethyl ester of O-benzyl-serine in Example 733 with the ethyl ester of each reactant compound listed in the Table, a series of analogs are formed of the general formula:

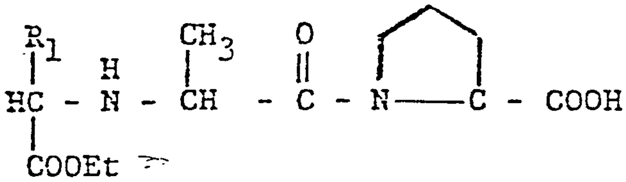

wherein $R_1$ is as shown in the table. The ethyl ester is converted to free acid, if desired, by saponification in each instance.

Table

| Example No. | Reactant | $R_1$ |
|---|---|---|
| 735 | Ethyl ester of O-methyl-serine | $CH_3OCH_2$- |
| 736 | Ethyl ester of O-ethyl-serine | $CH_3CH_2OCH_2$- |
| 737 | Ethyl ester of O-tertbutyl-serine | 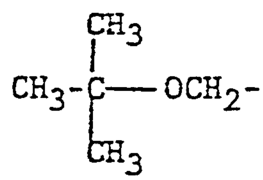 |
| 738 | Ethyl ester of N-δ-Boc-ornithine | H<br>Boc-N-$(CH_2)_3$- |
| 739 | Ethyl ester of N-ε-Boc-lysine | H<br>Boc-N-$(CH_2)_4$- |
| 740 | Ethyl ester of N-Cbo-lysine | H<br>Cbo-N-$(CH_2)_4$- |
| 741 | Ethyl ester of O-benzyl threonine | 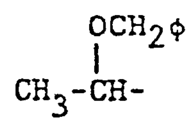 |
| 742 | Ethyl ester of O-ethyl-threonine | 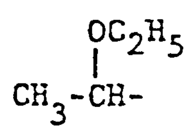 |
| 743 | Ethyl ester of O-t-butyl-threonine | 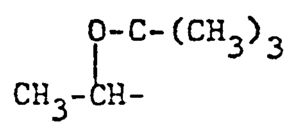 |

Table (cont.)

| Example No. | Reactant | $R_1$ |
|---|---|---|
| 744 | Ethyl ester of O-benzyl tyrosine | 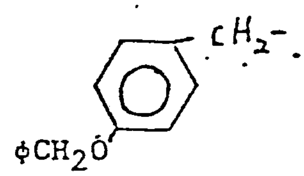 |
| 745 | Ethyl ester of O-methyl tyrosine | 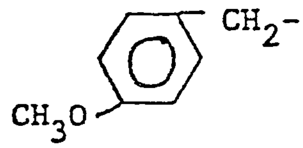 |
| 746 | Ethyl ester of O-ethyl tyrosine | $CH_3CH_2O\phi CH_2-$ |
| 747 | Ethyl ester of O-t.butyl tyrosine | $(CH_3)_3-C-O-\phi CH_2-$ |
| 748 | Ethyl ester of S-benzyl cysteine | $\phi CH_2S-CH_2-$ |
| 749 | Ethyl ester of S-methyl cysteine | $CH_3S-CH_2-$ |
| 750 | Ethyl ester of S-ethyl cysteine | $CH_3CH_2-S-CH_2-$ |
| 751 | Ethyl ester of S-acetyl penicillamine | $(CH_3)_2 - C -$ <br> \| <br> $S-C-CH_3$ <br> ‖ <br> O |

Example 752

The products of Examples733 and737 are each converted by treatment with anhydrous HF in the presence of anisole so that $R_1$ of the formula of Example734 becomes $HOCH_2$.

By the same treatment the product of Example738 is converted so that $R_1$ thereof is $NH_2(CH_2)_3-$, the product of Examples742 and743 are converted to a product in which $R_1$ becomes

$$CH_3 - \overset{\overset{OH}{|}}{CH} -$$

, and the products of Examples744 and745 are each converted to a product in which $R_1$ is 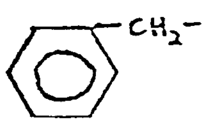 .

Example 753

Synthesis of N-[(1,3-dicarboxy)propyl]-Alanyl-Proline

A solution of 50 mmoles of the diethyl ester of α-keto-glutaric acid and 10 mmoles of L-Ala-L-Pro in ethanol is stirred with powdered molecular sieves at room temperature for 30 minutes. A solution of sodium cyanoborohydride, 10 mmoles, in ethanol is added slowly over the next 5 hours. The mixture is filtered, and the solvent of the filtrate is removed with a rotary evaporator. The residue is dissolved in 60% acetic acid, and the solution is extracted twice with ethyl acetate. The solvent of the aqueous phase is removed by freeze-drying. The product is obtained by partition column chromatography [butanol/acetic acid/$H_2O$ (4:1:5 by vol.)]. The named compound is obtained by removing the ethyl ester protecting groups by saponification.

Example 754

Synthesis of N-[2-(1,2-dicarboxyethylthio)-butyl]-4-keto-L-proline

The acid chloride of 2-bromo-butyric acid, 10 mmoles, and 2M NaOH, are separately added dropwise over 30 minutes to a solution of L-4-keto-proline, 10 mmoles, in 1 M NaOH: all at 0°C. The reaction is then stirred at room temperature for 3 1/2 hours. The mixture is cooled to 0°C and then acidified with cold concentrated $H_2SO_4$ to pH 2. The mixture is extracted 4 times with ethyl acetate. The ethyl acetate extracts are pooled, and the resulting solution is extracted with 10 mmol of $NaHCO_3$ in $H_2O$. The aqueous phase is extracted several times with ethyl acetate and then is acidified with concentrated HCl in the presence of fresh ethyl acetate. The organic solution is washed with saturated NaCl and then dried over anhydrous $MgSO_4$. After filtration, solvent is removed under high vacuum. The residue is dissolved in ethanol and is

sodium ethoxide as base; all at 0°C. The mixture is stirred under $N_2$ for 10 minutes at 0°C and then at room temperature overnight. Solvent is removed with a rotary evaporator. The residue is dissolved in $H_2O$, and the solution is extracted with diethyl ether. The aqueous solution is acidified to pH 2 with concentrated HCl and the product is extracted into ethyl acetate. The ethyl acetate phase is washed with saturated NaCl and then dried over anhydrous $MgSO_4$. After filtration, solvent is removed under reduced pressure. The named compound is obtained by partition column chromatography (Sephadex G-25 equilibrated and developed with butanol/acetic acid/$H_2O$ [4:1:5]).

Example 755

Synthesis of N-[(1-carboxy-2-phenyl)ethyl]-alanyl-L-proline thio acid

Proline thiophenyl ester, 40 mmoles, is coupled to pyruvic acid, 40 mmoles; using the DCC coupling procedure of Examples 722and724to yield N-pyruvoyl-L-proline thiophenyl ester. L-Phenylalanine, 5 mmoles, and N-pyruvoyl-L-proline thiophenyl ester, 20 mmoles, are stirred in ethanol with molecular sieves at room temperature for 30 minutes. Sodium cyanoborohydride, 5 mmoles, is added slowly over 6 hours. The molecular sieves are removed by filtration, and the solvent of the filtrate is removed under reduced pressure. The benzoyl group of the thiophenyl ester is removed with NaSH in ethanol, under $N_2$, to yield the named compound.

Example 756

Synthesis of N-(1-amido-isopropyl)-alanyl-L-proline

N-pyruvoyl-L-proline is prepared by the DCC coupling method of Examples722and724. Leucine amide, 5 mmoles, and N-pyruvoyl-L-proline, 25 mmoles, are stirred in ethanol with

After filtration, the solvent of the filtrate is removed under reduced pressure. The named compound is obtained by chromatography on LH-20 equilibrated and developed with 6% butanol.

Example 757

Synthesis of N-[(1-carboxy-2-phenyl)ethyl]-alanyl-L-proline amide

N-pyruvoyl-L-proline amide is prepared by coupling 20 mmoles of pyruvic acid and 20 mmoles of L-proline amide essentially as described in Example722. L-Phenylalanine, 5 mmoles, and N-pyruvoyl-L-proline amide, 20 mmoles, in tetrahydrofuran are stirred with molecular sieves for 30 minutes and then sodium cyanoborohydride, 5 mmoles, is added slowly over 6 hours. After filtration, solvent is removed from the filtrate. The ethyl ester is removed by saponification to yield the named compound.

Example 758

Synthesis of N-[2-(1-carboxy-2-phenylethylthio)-3-benzyloxypropanoyl]-L-proline

O-Benzyl-serine is converted to 2 bromo-3-benzyloxy-propanoic acid by the bromination technique described by Testa et al. (Helv. Chim. Acta 46, 766, 1963).

This compound is converted into its N-hydroxysuccinimide ester according to the method of Example 724and is coupled to L-proline-t-butyl-ester as is also described in Example 724. This method will not work for dehydroproline.

The resulting product is then reacted with 2-mercapto-3-phenylpropanoic acid essentially as described in Example 724. The t-butyl ester is removed by treatment with trifluoroacetic acid in the presence of anisole to yield the named compound.

## Examples 759-763

By substituting one of the reactants of the following Table for O-benzyl-serine in Example 758, a series of analogs is obtained in which $R_3$ is as shown:

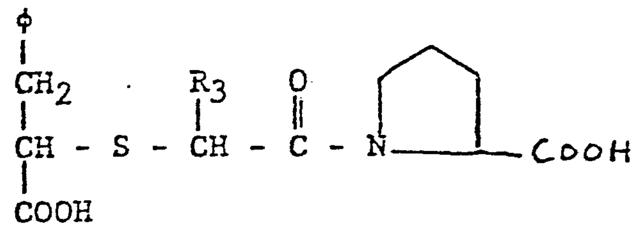

### Table

| Example | Reactant | $R_3$ |
|---|---|---|
| 759 | O-methyl-serine | $CH_3O-CH_2$ |
| 760 | O-ethyl-serine | $C_2H_5O-CH_2$ |
| 761 | O-benzyl-threonine | $\phi CH_2OCH-$<br>$CH_3$ |
| 762 | O-methyl-threonine | $CH_3O-CH-$<br>$CH_3$ |
| 763 | O-ethyl-threonine | $C_2H_5-O-CH-$<br>$CH_3$ |

## Example 764

The compound synthesized in each of Example 758 and Example 761 are, by treatment with anhydrous HF in the presence of anisole, converted to compounds in which $R_3$ is respectively either $HOCH_2-$ or $HOCH-$.
$CH_3$

## Example 765

By the methods described in United States Patent No. 4,204,991, the serine based compounds of Examples 759-760 are converted into the corresponding dehydro-alanine analogs, and the threonine based compounds of Examples 761-763 are converted

2,3- or the

Example 766

By reacting the 2-bromo compounds produced in the second step of each of Examples 758-763 inclusive with 2-hydroxy-3-phenylpropanoic acid ethyl ester in the presence of 1 equivalent of sodium, analogs of the compounds of each of Examples 758-763 are formed in which the thioether linkage is replaced with an ether oxygen.

Example 767

Synthesis of N-(1-ethoxycarbonylethyl)-L-alanyl-L-proline

N-pyruvoyl-L-proline, 50 mmoles (prepared as per Example is reacted with 10 mmoles of the ethyl ester of alanine and then with 10 mmoles of sodium cyanoborohydride according to the method of Example 733. The product is recovered and purified as described in Example 733.

Example 768

The inhibitory potency of several compounds *in vitro* was measured in the following assay. The $I_{50}$ values are reported in Table 1. Human plasma ACE was assayed in 0.05 M Hepes buffer, pH 8.0 containing 0.1 M NaCl and 0.75 M $Na_2SO_4$. The substrate employed was hippuryl-His-Leu at a final concentration of $1 \times 10^{-4}$ M, (Km = $2 \times 10^{-4}$ M), together with about 130,000 cpm/50 µl of [$^3$H]hippuryl-His-Leu (25 Ci/mmole). Enzyme was diluted in the above buffer such that 40 ul of buffered enzyme was capable of hydrolyzing 13% of substrate in a 15 minute incubation at 37°C. To initiate the assay, 40 µl of enzyme and 10 µl of buffer or inhibitor dissolved in buffer were preincubated for two minutes at 37°C. Substrate, 50 µl, was then added to initiate reaction and the solution was incubated for 15 minutes at 37°C. To terminate the reaction, 1 ml of 0.1 M HCl was added, following which 1 ml of ethyl acetate was added. The mixture was

An aliquot, 500 ul. of the ethyl acetate layer was transferred to a liquid scintillation vial containing 10 ml of Riafluor, trademark New England Nuclear Corporation, Boston, Massachusetts. For determination of $I_{50}$ values, enzyme activity in the presence of inhibitor at a series of different concentrations was compared to activity in the absence of inhibitor. A plot of log inhibitor concentration versus percent inhibition yielded the $I_{50}$ value; the concentration of inhibitor required to reduce enzymic activity by 50%.

Table 1

| Compound | $I_{50}$ |
|---|---|
| N-[3-(1-carboxyethylthio)-2-D-methylpropanoyl]-L-proline | $1.5 \times 10^{-6}M$ |
| N-[2-(1-carboxy-3-hydroxy-butylthio)-propanoyl]-L-proline | $3 \times 10^{-8}M$ |
| N-[2-(tetrahydro-5-methyl-2-furanone-3-thio)-propanoyl]-L-proline | $1.3 \times 10^{-7}M$ |
| N-(1-carboxyethyl)-L-alanyl-L-proline | $8 \times 10^{-9}M$ (Known Compound) |
| N-(1-ethoxycarbonylethyl)-L-alanyl-L-proline | $6 \times 10^{-8}M$ |
| N-[2-(1-carboxyethylthio)-propanoyl]-L-proline | $4 \times 10^{-7}M$ |

Example 769

Intravenous Effectiveness of N-[3-(1-carboxyethyl-thio)-2-D-methylpropanoyl]-L-proline

Rats (190-290 g body weight) were fasted overnight and then anesthetized with intraperitoneal pentobarbital, 50-60 mg/kg. Tracheostomy was performed and the animals were ventilated mechanically. A cannula was inserted into a femoral vein for injection of angiotensin I, and a second cannula was inserted into a common carotid artery for direct measurement of arterial blood pressure. Heparin, 1,000 units, was injected via the femoral vein to prevent coagulation. Blood pressure was measured with a pressure transducer connected to a polygraph. The rats were injected with 400 ng/ml of angiotensin I in 20 µl ________________

of 0.9 g % NaCl; an amount of angiotensin I sufficient to raise mean arterial blood pressure by approximately 40 mm Hg. After the responsiveness of a given rat to angiotensin I was established, the named compound at 20 μmole/kg (drug dissolved in 0.15 ml of $H_2O$ plus 10 μl of 1 N $NaHCO_3$), was given intravenously. At timed intervals, the effects of 400 ng/kg of angiotensin I on mean arterial blood pressure were tested. Results are shown below:

| Time After IV Administration (minutes) | Blood Pressure Reponse to 400 ng/kg of Angiotensin I (% of Control) |
|---|---|
| -5 | 100% (40 mmHg) |
| +2 | 67.5 |
| 8 | 62.5 |
| 15 | 37.5 |
| 21 | 37.5 |

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as fall within the scope of the appended claims.

CLAIMS



0048159

1. Novel compounds having the general formula

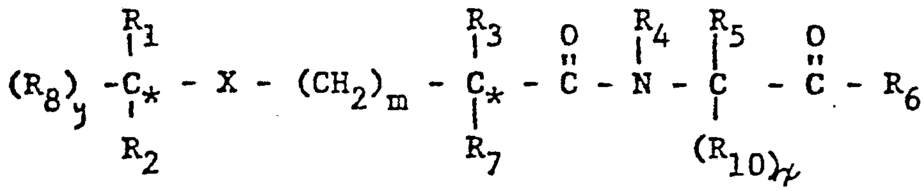

wherein $x$ and $y$ are 0 or 1, X may be S, O or N-$R_9$ and $R_9$ may be -H or -$CH_3$, m is 0 or 1, and $R_{10}$ is H, $CH_3$, F, Cl or Br;

$R_2$ is COOH, $CH_2COOH$, COSH, $CH_2COSH$, $CH_2SH$, $CH_2CH_2SH$, a physiologically acceptable non-toxic salt of any of them; COOY; $CH_2COOY$, COSY, $CH_2COSY$, $CH_2SY$ or $CH_2CH_2SY$, wherein Y is phenyl, benzyl or a 1-5 carbon alkyl group, $C{=}O{-}N{<}^{A_1}_{A_2}$ wherein either of $A_1$ and $A_2$ may be H, phenyl, benzyl or a 1-5 alkyl group;

$R_4$ and $R_5$ together form a ring with the nitrogen and carbon atoms to which they are respectively attached, which ring is one of the structures:

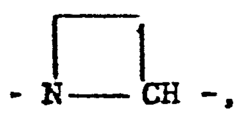 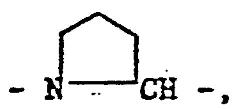 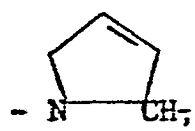 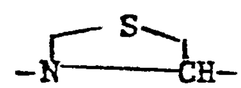

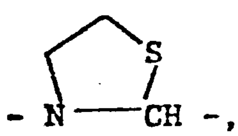 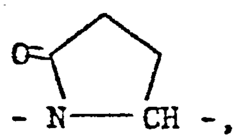 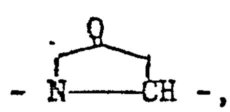 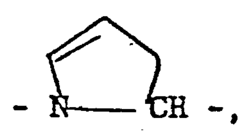 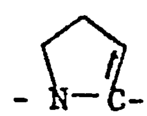

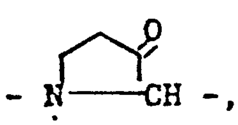 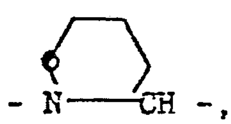 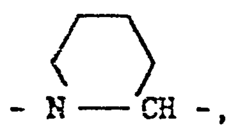 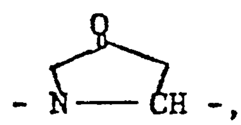 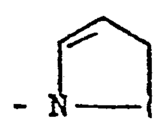

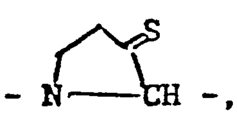 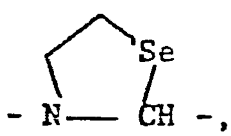 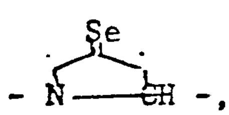 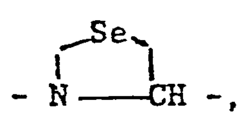 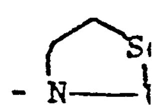

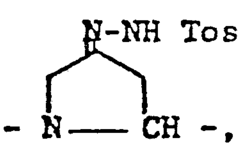 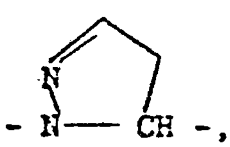 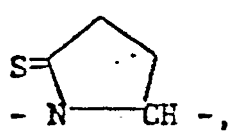 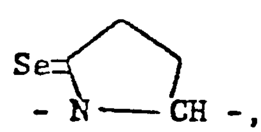

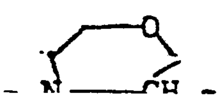 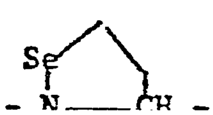 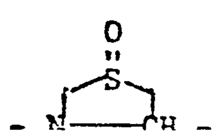 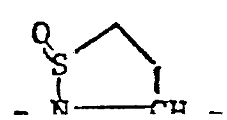

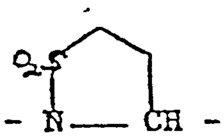, 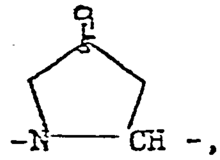, 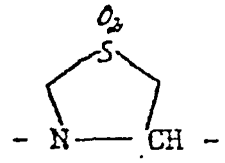, 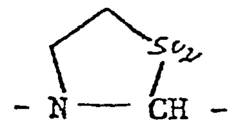,

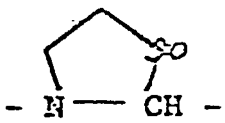, 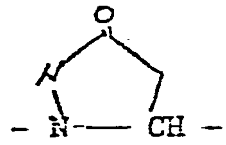, 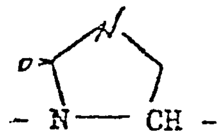, 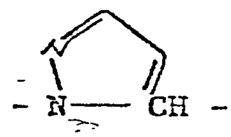,

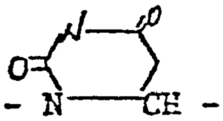, 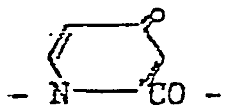, 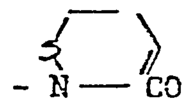 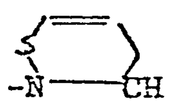, 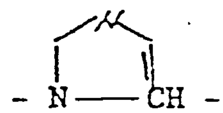,

it being understood that any of these structures may be monosubstituted with -OH, $-OCH_3$, F, -O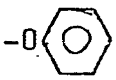, $-OCH_2$ 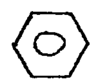, $-\overset{O}{\overset{\|}{C}}-OY$, Cl, Br, I, phenyl, hydroxyphenyl, -SH, $-SCH_3$, - S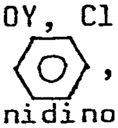, $-SCH_2$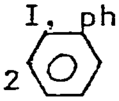, $-NHCH_3$, $-CH_2NH_2$, $-CH_3$, $-CH_2OH$, propyl, guanidino, nitroguanidino or thioguanidino and that the five or six-membered rings may be disubstituted with -OH, F, Cl, Br, I, $OCH_3$, or any combination of two of these substituents;

$R_6$ is $NH_2$, -OM or -SM, wherein M may be H, an alkyl group of 1-3 carbon atoms or any other ester moiety hydrolyzable under mammalian <u>in vitro</u> conditions to -OH, or an ionically bonded anion of a physiologically acceptable non-toxic salt;

$R_7$ is $H_1$-$CH_3$, halomethyl, hydroxymethyl, aminomethyl or mercaptomethyl;

$R_8$ is H-, $CH_3$, amino, halomethyl, hydroxymethyl, aminomethyl, dihalomethyl, trihalomethyl, mercaptomethyl, methoxymethyl, methylthiomethyl, methoxycarbonylmethyl, cyanomethyl, benzyl, acetoxymethyl, $CH_2 = CH - CH_2$, isobutyl, mercaptoalkyl of 2-3 carbon atoms, hydroxyalkyl of 2-3 carbon atoms, ethyl, acetylthioethyl, benzamido, acetamido, phthaloylaminoalkylene wherein the alkylene group has 1-4 carbon atoms, alkoxycarbonyl isoalkylene wherein the alkyl group contains 1-6 carbons and the isoalkylene group contains 3-5 carbons, benzoylamine, alkanoylamine of 1-6 carbons, alkylamide of 1-6 carbons, phenylamine or alkylamine of 1-6 carbons.

(A) $R_1$ and $R_3$ are the same or different and are each any of

(i) mono-N substituted alkylene of 2-4 carbons wherein the N substituent is benzoyl, Boc, Cbo, Tos, formyl or acetyl;

(ii) hydroxyphenyl or hydroxyphenyl-(1-6C)-alkylene hydroxyalkylene having 1-4 carbons, or thiol analogs thereof,

(iii) mercaptoalkylene of 1-6 carbons;

(iv) phenylalkylene wherein the alkylene group has 1-6 carbons, $C_1$-$C_5$ straight or branched chain alkyl or phenyl,

(v) phenylthioalkylene, phenoxyalkylene, benzyloxyalkylene or benzylthioalkylene wherein the alkylene group has 1-6 carbons;

(vi) alkylthioalkylene wherein the alkyl and alkylene groups have 1-6 carbons;

(vii) alkoxyphenyl or alkoxybenzyl in which the alkoxy group has 1-6 carbons, phenoxyphenyl, phenoxybenzyl, benzyloxybenzyl or benzyloxyphenyl or a thioether analog of any of them;

(viii) $-(CH_2)_n-\underset{\displaystyle OB}{\underset{|}{CH}}-CH_3$ wherein n = 0 - 4 and B = H or a 1-6 carbon alkyl group; or an -SB analog thereof;

(ix) $(CH_2)_pCOOZ$ or $(CH_2)_pCOSZ$ wherein p = 0 - 3 and Z is H, phenyl, benzyl, a 1-6 carbon alkyl group, or an anion of a physiologically acceptable salt;

(x) $-(CH_2)_n-\underset{\displaystyle O-\underset{\displaystyle \overset{\|}{O}}{C}-Z}{\underset{|}{CH}}-CH_3$ or $-(CH_2)_n-\underset{\displaystyle \underset{\displaystyle \overset{\|}{O}}{C}-OZ}{\underset{|}{CH}}-CH_3$

or $-(CH_2)_n-\underset{\displaystyle S-\underset{\displaystyle \overset{\|}{O}}{C}-Z}{\underset{|}{CH}}-CH_3$ or $-(CH_2)_n-\underset{\displaystyle \underset{\displaystyle \overset{\|}{O}}{C}-SZ}{\underset{|}{CH}}-CH_3$

wherein n and Z each have the same significance as above;

(xi) $HO-(CH_2)_n-\overset{\displaystyle D}{\overset{|}{CH}}-$ or $HS-(CH_2)_n-\overset{\displaystyle D}{\overset{|}{CH}}-$ wherein n = 0 - 4, D is phenyl, thienyl or a 1-3 carbon alkyl group;

(xii) $HO - (CH_2)_n - X\ (CH_3)_2$ -, $HS - (CH_2)_n - C\ (CH_3)_2$ -, phydroxyphenyl - $(CH_2)_n - C\ (CH_3)_2$ - or p-mercaptophenyl- $(CH_2)_n - C\ (CH_3)_2$ - wherein n has the same significance as above.

(xiii) p - mercaptophenyl - $(CH_2)_n - CH_2$ - or p-hydroxyphenyl- $(CH_2)_n - CH_2$ - wherein the phenyl ring has one or two nitro or amino substituents and n has the same significance as above;

(xiv) $CH_3\ (CH_2)_n - \overset{OH}{CH}$ - or $CH_3\ (CH_2)_n - \overset{SH}{CH}$ - wherein n has the same significant as above.

(xv) $NH_2$ - alkylene or $NO_2$ - alkylene containing one hydroxy or mercapto substituent and having 1-6 carbon atoms;

(xvi) hydroxy- or mercapto-phenoxybenzyl;

(xvii) $ZO\text{-}(CH_2)_q - \overset{O}{\overset{\|}{C}}\ (CH_2)_n$, $ZS\text{-}(CH_2)_q - \overset{O}{\overset{\|}{C}} - (CH_2)_n$ -,

$NH_2\text{-}(CH_2)_q - \overset{O}{\overset{\|}{C}} - (CH_2)_n$ -, $NO_2(CH_2)_q - \overset{O}{\overset{\|}{C}} - (CH_2)_n$ -,

$HONH - (CH_2)_q - \overset{O}{\overset{\|}{C}} - (CH_2)_n$ -; $NH_2NH - (CH_2)_q - \overset{O}{\overset{\|}{C}} - (CH_2)_n$ -;

$ZO - \overset{O}{\overset{\|}{C}}\ (CH_2)_q - \overset{O}{\overset{\|}{C}} - (CH_2)_n$,- $ZS - \overset{O}{\overset{\|}{C}} - (CH_2)_q - \overset{O}{\overset{\|}{C}} - (CH_2)_n$ - or

$NH_2\overset{O}{\overset{\|}{C}}N - (CH_2)_q - \overset{O}{\overset{\|}{C}} - (CH_2)_n$ - wherein q = 1 - 5 and z and n have the same significance as above.

(xviii) $ZO\text{-}(CH_2)_q - \overset{OH}{CH} - (CH_2)_n$ -, $ZS\text{-}(CH_2)_q - \overset{OH}{CH} - (CH_2)_n$ -,

$NH_2 - (CH_2)_q - \overset{OH}{CH} - (CH_2)_n$ -, $NO_2 - (CH_2)_q - \overset{OH}{CH} - (CH_2)_n$ -,

$ZO - (CH_2)_q - \overset{OH}{CH} - (CH_2)_n$ -, $ZS - - (CH_2)_q - \overset{OH}{CH} - (CH_2)_n$ -,

$HONH - (CH_2)_q - \overset{OH}{CH} - (CH_2)_n$ -, $NH_2NH\text{-}(CH_2)_q - \overset{OH}{CH} - (CH_2)_n$ -

or $NH_2\overset{O}{\overset{\|}{C}}NH\text{-}(CH_2)_q\ \text{-}\overset{OH}{CH}\text{-}(CH_2)_m$-wherein Z, q and n all have the same

$(CH_2)_n$ -, $G - (CH_2)_q - \overset{O}{\overset{\|}{C}} - (CH_2)_n$ -, $G - (CH_2)_q - \overset{OH}{\overset{|}{C}H} - (CH_2)_n$ -, $NH_2 - \underset{O}{\underset{\|}{C}} - (CH_2)_q - \underset{O}{\underset{\|}{C}} - (CH_2)_n$ -, $NH_2 - \overset{O}{\overset{\|}{C}} - (CH_2)_q - \overset{OH}{\overset{|}{C}H} - (CH_2)_n$ -

wherein G is an alkacyl or alkacyloxy group of 1-6 carbons, a benzoyl or benzoyloxy group, or a phenylalkacyl or phenylalkacyloxy group wherein the alkacyl or alkacyloxy group contains 2-6 carbons and q and n have the same significance as set forth above;

(xx) $K - (CH_2)_n - \overset{O}{\overset{\|}{C}} - (CH_2)_n -$ or $K\ (CH_2)_n - \overset{OH}{\overset{|}{C}H} - (CH_2)_n -$

wherein n has the significance stated above and K is selected from carboxyphenyl, aminophenyl, nitrophenyl, halophenyl, hydroxyphenyl, alkylthiophenyl, alkylphenyl, mercaptophenyl, cyanophenyl, mercapto-carbonylphenyl, alkylcarbonylphenyl, alkylcarbonyloxphenyl, hydrazinophenyl, ureidophenyl, alkylcarbonylaminophenyl, alkylcarbonylthiophenyl, alkaloxyphenyl and hydroxy minophenyl, wherein all alkyl groups contain 1-6 carbon atoms;

(xxi) $L - (CH_2)_n - \overset{O}{\overset{\|}{C}} - (CH_2)_n -$ or $L-(CH_2)_n - \overset{OH}{\overset{|}{C}H} - (CH_2)_n -$

wherein n has the significance stated above and L is selected from cycloalkyl groups of 3-7 carbons which may be unsubstituted with up to two groups selected from among carboxy, amino, nitro, halo, hydroxy, mercapto, mercaptocarbonyl, hydroxyamino, alkyl, alkylcarbonyl, alkylcarbonyloxy, alkylthio, alkylcarbonylamino, alkylcarbonylthio, cyano, hydrazino, ureido and alkyloxy, wherein all alkyl groups contain 1-6 carbon atoms;

(xxii) guanidino alkylene, thioguanidinoalkylene, or nitroguanidino alkylene in which the alkylene groups contain 1-6 carbon atoms;

(xxiii) ring substituted aryl groups in which the ring substituents may be the same or different and may comprise up to ______

five per ring of any of the following: $-NH_2$, $-OZ$, $-SZ$, halogen, $-CN$, $-NO_2$, $-COOZ$, COSZ, $CONH_2$, $-NHNH_2$, alkyl, alkylcarbonyl, alkylcarbonyloxy, alkylcarbonylamino, haloalkyl, dihaloalkyl, trihalomethyl, hydroxyamino, alkylcarbonylthio, phenoxy, and benzyloxy wherein the alkyl groups contain 1 - 6 carbon atoms and Z has the same significance as above;

(xxiv) amidoalkylene or alkylcarbonyl-aminoalkylene wherein the alkyl and alkylene groups contain 1 - 6 carbon atoms;

(xxv) hydroxyaminoalkylene of 1 - 6 carbons;

(xxvi) vinyl and substituted vinyl groups in which the substituents may be alkyl, aryl, cycloalkyl or heterocyclic groups;

(xxvii) unsubstituted heterocyclic groups selected from among phenothiazinyl, pyrrolidinyl, pyrrolyl, quinolinyl, imidazolyl, pyridyl, thyminyl, benzothiazinyl, indolyl, thienyl, purinyl, piperidinyl, morpholinyl, azaindolyl, pyrazinyl, pyrimidyl, piperonyl, piperazinyl, furanyl, thiazolyl, and thiazolidinyl, cytosinyl;

(xxviii) alkylene or alkenyl groups of 1 - 6 carbons substituted with one of the heterocyclic rings from (xxvii) above;

(xxix) groups from (xxvii) or (xxviii) above, containing up to four ring substituents on the heterocyclic ring selected from among $-OZ$, $-SZ$, $-COOZ$, $-NO_2$, $-NH_2$, $-COSZ$, halogen, haloalkyl, dihaloalkyl, trihalomethyl, cyano, $-CONH_2$, alkyl, alkylcarbonyl, alkylcarbonyloxy, alkylcarbonyl-amino, alkylcarbonylthio, phenoxy, benzyloxy, $-NH\overset{O}{C}-NH_2$, $-NHNH_2$ and HONH - wherein Z has the same significance as above;

(xxx) groups from (xxvii), (xxviii) or (xxix) attached to

(xxxi) mono-, di- or tri-alkyl-, alkenyl- or phenyl-silyl or selenyl wherein the alkyl or alkenyl groups contain 1 - 6 carbons.

B. When $R_1$ is a group from among (xi) - (xxxi) above, $R_3$ may also be any of H, 1 - 5 carbon straight or branched chain alkyl, phenyl, -OH, alkoxy of 1 - 5 carbons, benzyloxy, benzyloxyalkylene or phenoxyalkylene wherein the alkylene has 1 - 5 carbons, alkoxyalkylene having 1 - 5 carbons in the alkoxy and alkylene groups, aminoalkylene of 1 - 6 carbons, alkenyl of 1 - 6 carbons, benzyl, hydroxyalkyl of 1 - 6 carbons, mercaptoalkyl of 1 - 6 carbons, histidinyl, haloalkyl of 1 - 6 carbons, 4 -aminoethyl-benzyl, acetamidoalkyl of 1 - 5 carbons, benzylthiomethylene, or dimethylaminoalkyl of 1 - 5 carbons.

C. When $R_3$ is a group from among (k) - (xxxi) above, $R_1$ may also be any of H, $C_1$ - $C_8$ straight or branched chain alkyl, phenyl, benzyl, unsubstituted aminoalkylene of 2 - 6 carbons, hydroxyalkylene of 1 - 6 carbons, hydroxyphenyl, phenoxyalkylene or benzyloxyalkylene wherein the alkylene group has 1 - 6 carbons, cycloalkyl of 3 - 6 carbons, cycloalkyl methyl, 3 indolyl-phenylethyl, methylthioethyl, 3-indolyl alkyl wherein the alkyl group contains 1 - 5 carbons, imidazolyl, imidazolylalkyl wherein the alkyl group contains 1 - 5 carbons, phenoxymethyl, phenylthiomethyl, 4-aminomethyl benzyl, 2-aminophenethyl, naphthylethyl, 4-halophenethyl, 3,4-dihalophenethyl or phenoxyphenethyl, or

D. $R_1$ and $R_2$ together may form with -CH a lactone ring of the formula:

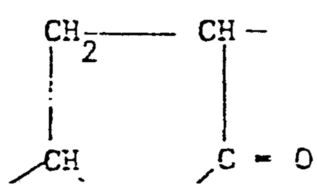

or

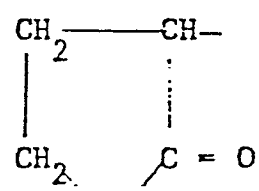

induce D-penicillamine-like adverse effects, applicants prepared and disclosed in copending application Ser. No. 187992 filed September 17, 1980, a series of compounds having analogous side chain structure to an effective substrate for the enzyme, benzoyl-Phe-Ala-pro. The analogous compounds possess an ether or thioether linkage and a substituted carboxymethyl proximate to the ether or thioether. In some compounds, the substituted carboxymethyl group is replaced by corresponding substituted thiocarboxymethyl or mercaptomethyl group.

Such compounds are stable and have durations of action much longer than that of captopril. Thus, the inhibitors of Application Ser. No. 187992 may be used for treating hypertension with less frequent dosage schedules than required for captopril and may be capable of administration under less rigorously controlled conditions.

Patchett et al., in European application 79105015.6 publication number 0012401, published on or about June 25, 1980, discloses carboxyalkyl dipeptide derivatives which closely related to the compounds of U.S. Ser. No. 187992 and exhibit generally similar durations of action in *in vitro* tests.

Applicants have now discovered additional heretofore undisclosed compounds which are somewhat related to those earlier disclosed. In at least some cases these compounds are more efficacious and/or longer acting as ACE inhibitors than any of the previously disclosed compounds.

0048159

## BRIEF DESCRIPTION OF THE INVENTION

Novel inhibitors of ACE are disclosed which have the general formula:

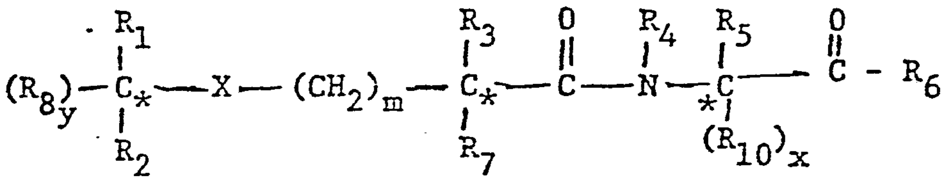

wherein x and y are 0 or 1, X may be S, O or N-$R_9$ and $R_9$ may be -H or -$CH_3$ m is 0 or 1, and $R_{10}$ is H, $CH_3$, F, Cl or Br;

$R_2$ is COOH, $CH_2COOH$, COSH, $CH_2COSH$, $CH_2SH$, $CH_2CH_2SH$, a physiologically acceptable non-toxic salt of any of them; COOY; $CH_2COOY$, COSY, $CH_2COSY$, $CH_2SY$ or $CH_2CH_2SY$, wherein Y is phenyl, benzyl or a 1 - 5 carbon alkyl group, 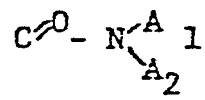 wherein either of $A_1$ and $A_2$ may be H, phenyl, benzyl or a 1 - 5 alkyl group;

$R_4$ and $R_5$ together form a ring with the nitrogen and carbon atoms to which they are respectively attached, which ring is one of the structures: